# EUROPEAN PATENT SPECIFICATION

(11) **EP 4 267 763 B1**
(45) Date of publication and mention of the grant of the patent: **23.07.2025**
(21) Application number: 21847666.1
(22) Date of filing: 23.12.2021
(51) Int. Cl.: C12Q 1/6858, C12Q 1/6855

(54) **COMPOSITIONS AND METHODS FOR HIGHLY SENSITIVE DETECTION OF TARGET SEQUENCES IN MULTIPLEX REACTIONS**
ZUSAMMENSETZUNGEN UND VERFAHREN ZUM HOCHEMPFINDLICHEN NACHWEIS VON ZIELSEQUENZEN IN MULTIPLEX-REAKTIONEN
COMPOSITIONS ET PROCÉDÉS DE DÉTECTION HAUTEMENT SENSIBLE DE SÉQUENCES CIBLES DANS DES RÉACTIONS MULTIPLEX

(30) Priority: 24.12.2020 US 202063199421 P; 23.04.2021 US 202163201338 P; 28.05.2021 US 202163202179 P
(43) Date of publication of application: 01.11.2023
(73) Proprietor: Life Technologies Corporation, Carlsbad, CA 92008 (US)
(72) Inventor: ALLEN, Michael, Carlsbad, California 92008 (US); ANDERSEN, Mark, Carlsbad, California 92008 (US)
(74) Representative: Thermo Fisher Scientific- Life Sciences Solutions Group
(86) International application number: PCT/US2021/073095
(87) International publication number: WO 2022/140793

(56) References cited:
- WO-A1-2012/149438
- WO-A1-2013/081864
- WO-A1-2013/158215
- WO-A1-2015/120177
- WO-A1-2017/214561
- WO-A1-2019/006392
- WO-A1-2019/046817
- WO-A1-2019/062614
- WO-A1-2020/227100
- WO-A1-2022/053610
- VARLEY KATHERINE ELENA ET AL: "Nested Patch PCR enables highly multiplexed mutation discovery in candidate genes", vol. 18, no. 11, 1 November 2008 (2008-11-01), pages 1844 - 1850, XP002678933, ISSN: 1088-9051, Retrieved from the Internet <URL:http://genome.cshlp.org/content/18/11/1844> [retrieved on 20081010], DOI: 10.1101/GR.078204.108
- N. E. BROUDE ET AL: "Multiplex allele-specific target amplification based on PCR suppression", PROCEEDINGS OF THE NATIONAL ACADEMY OF SCIENCES, vol. 98, no. 1, 2 January 2001 (2001-01-02), pages 206 - 211, XP055767314, ISSN: 0027-8424, DOI: 10.1073/pnas.98.1.206

## Description

### RELATED APPLICATIONS

This application claims priority to and the benefit under each of U.S. Provisional Application no. 63/199,421, filed December 24, 2020, and U.S. Provisional Application no. 63/201,338, filed April 23, 2021, and U.S. Provisional Application no. 63/202,179, filed May 28, 2021.

### SEQUENCE LISTING

The material in the electronic Sequence Listing is submitted as a text (.txt) file entitled "LT01594_ST25.txt" created on December 23, 2021 which has a file size of 114 KB.

### FIELD OF THE INVENTION

The present invention relates to methods of preparing a library of target nucleic acid sequences and compositions and uses for highly sensitive detection of target sequences.

### BRIEF SUMMARY OF THE INVENTION

The invention is set out in the appended set of claims. The invention relates to a method for preparing a library of one or more target nucleic acid sequences present in a sample at low frequency comprising: amplifying within a single amplification reaction mixture a multiplex of different target sequences from a sample including a plurality of different target sequences, wherein the amplifying includes contacting at least a portion of the sample with a plurality of target-specific primers, and a polymerase under amplification conditions, thereby producing a multiplex of different amplified target sequences, wherein at least two of the plurality of target-specific primers include cleavable groups and at least one of the produced multiplex of different amplified target sequences include cleavable groups at both ends of resulting amplicon, and wherein at least one of the plurality of target specific primers is a blocking primer that does not include the cleavable groups and at least one of the produced multiplex of different amplified target sequences includes a cleavable group at only one end of resulting amplicon; cleaving the cleavable groups of the multiplex of different amplified target sequences and forming cleaved ends; and ligating at least two adapters to cleaved ends of at least one of the multiplex of different amplified target sequences, thereby producing one or more adapter-ligated amplified target sequences; thereby preparing a library of different adapter-ligated target sequences, wherein amplicons resulting from blocking target specific primers are not capable of ligating adapter, and wherein none of the adapters in the ligation reaction hybridizes under high stringency conditions to any one of the multiplex of different amplified target sequences. The invention further relates to a method for preparing a library of one or more nucleic acid target sequences present in a sample at low frequency comprising amplifying within a single amplification reaction mixture a multiplex of different target sequences from a sample including a plurality of different target sequences, wherein the amplifying includes contacting at least a portion of the sample with a plurality of adapters capable of amplification of one or more nucleic acid target sequences in the sample under conditions wherein the nucleic acid target sequences undergo a first amplification, wherein at least two of the plurality of adapters comprise a universal handle sequence and a target nucleic acid sequence and a cleavable moiety and optionally one or more tag sequences wherein the target nucleic acid sequence of the adapter includes at least one cleavable moiety and the universal handle sequence does not include the cleavable moiety and at least one of the produced multiplex of different amplified target sequences include cleavable groups at both ends of resulting amplicon, and wherein at least one of the plurality of adapters is a blocking primer that does not include the cleavable moiety or universal handle sequence and at least one of the produced multiplex of different amplified target sequences includes a cleavable group and universal handle sequence at no more than one end of resulting amplicon; digesting resulting first amplification products to reduce or eliminate resulting primer dimers and prepare partially digested target amplicons, producing gapped, double stranded amplicons, then repairing the partially digested target amplicons; and amplifying the repaired target amplicons in a second amplification using universal primers, thereby producing one or more adapter-amplified target sequences, thereby preparing a library of different adapter-amplified target sequences, wherein amplicons resulting from blocking primer are not compatible with second universal primer amplification and wherein none of the adapters in the amplification reaction hybridizes under high stringency conditions to any one of the multiplex of different amplified target sequences. Provided are methods for preparing a library of one or more target nucleic acid sequences present in a sample for detection of target nucleic acids present at low frequency, as well as compositions and uses therefor. Methods comprise contacting at least a portion of a sample with a plurality of target specific primers or adapters capable of amplification of one or more target nucleic acid sequences under conditions wherein the target nucleic acid(s) undergo a first amplification, and including one or more target specific blocking primers in the reaction; digesting the resulting first amplification products and either ligating the resulting digested amplicons to universal adapter sequences or repairing the resulting digested amplicons; and amplifying the ligated or repaired products in a second amplification, thereby producing a library of target nucleic acid sequences. In particular aspects provided methods comprise use of target specific primers comprising cleavable groups and target specific blocking primers lacking cleavable groups. Provided methods may be carried out in a single, addition only workflow reaction, allowing for rapid production of highly multiplexed target libraries. Resulting library compositions provide optimized features for analytical detection of target sequences in a sample, including sequencing applications.

One aspect of the invention comprises methods for preparing a library of one or more target sequences present in a sample at low frequency. In certain aspects the methods comprise amplifying within a single amplification reaction mixture a multiplex of different target sequences from a sample including a plurality of different target sequences, wherein the amplifying includes contacting at least a portion of the sample with a plurality of target-specific primers, and a polymerase under amplification conditions, thereby producing a multiplex of different amplified target sequences. Provided methods include at least two of the plurality of target-specific primers having cleavable groups and at least one of the plurality of target specific primers does not include the cleavable groups. The methods further comprise cleaving the cleavable groups of the multiplex of different amplified target sequences and forming cleaved ends, and ligating at least two adapters to cleaved ends of at least one of the multiplex of different amplified target sequences, thereby producing one or more adapter-ligated amplified target sequences, thereby preparing a library of different adapter-ligated target sequences. In some aspects at least one of the produced multiplex of different amplified target sequences from the first amplification include cleavable groups at both ends of resulting amplicon and at least one of the produced multiplex of different amplified target sequences includes a cleavable group at only one end of resulting amplicon. In some examples amplicons resulting from blocking target specific primer lacking cleavable groups are not capable of ligating adapter. In certain aspects none of the adapters in the ligation reaction hybridizes under high stringency conditions to any one of the multiplex of different amplified target sequences.

Another aspect of the invention comprises methods for preparing a library of one or more target sequences present in a sample at low frequency comprising amplifying within a single amplification reaction mixture a multiplex of different target sequences from a sample including a plurality of different target sequences, wherein the amplifying includes contacting a nucleic acid sample with a plurality of adapters capable of amplification of one or more target nucleic acid sequences in the sample under conditions wherein the target nucleic acid(s) undergo a first amplification. Provided methods include at least two of the plurality of adapters comprising a universal handle sequence and optionally one or more tag sequences and a target nucleic acid sequence and a cleavable moiety, wherein the target nucleic acid sequence of the adapter includes at least one cleavable moiety and the universal handle sequence does not include the cleavable moiety; and at least one of the plurality of target specific primers does not include the cleavable groups, optional tag sequence or a universal handle sequence. The methods further comprise digesting resulting first amplification products to reduce or eliminate resulting primer dimers and prepare partially digested target amplicons, producing gapped, double stranded amplicons, then repairing the partially digested target amplicons, followed by amplifying the repaired target amplicons in a second amplification using universal primers, thereby producing one or more adapter-ligated amplified target sequences. In some aspects at least one of the produced multiplex of different first amplified target sequences include cleavable groups at both ends of resulting amplicon and at least one of the produced multiplex of different amplified target sequences includes a cleavable group at only one end of resulting amplicon. In some aspects amplicons resulting from incorporation of blocking target specific primer lacking universal handle are not capable of second amplification round processing and will not generate amplicons for sequencing analysis.

In a further aspect, compositions are provided. In some aspects provided are compositions comprising nucleic acid libraries generated by the methods described herein. In additional aspects, compositions comprising a plurality of target specific primers are provided, wherein at least two of the plurality of target-specific primers include cleavable groups, and wherein at least one of the plurality of target specific primers does not include the cleavable groups. In certain aspects, compositions include at least two and up to one hundred thousand target specific primer pairs and at least one and up to one hundred thousand target specific blocker primers. In other aspects, compositions comprising a plurality of nucleic acid adapters are provided, wherein at least two of the plurality of adapters comprise a 5' universal handle sequence, one or more unique tag sequences, and a 3' target nucleic acid sequence wherein each adapter comprises a cleavable moiety; and at least one of the plurality of nucleic acid adapters does not comprise cleavable groups, universal handle sequence or unique tag sequence In certain aspects the target nucleic acid sequence of the adapter includes at least one cleavable moiety, cleavable moieties are included flanking either end of the tag sequence and the universal handle sequence does not include the cleavable moiety. In additional certain aspects, compositions include at least two and up to one hundred thousand target specific adapter pairs and at least one and up to one hundred thousand target specific blocker primers.

Still further, uses of provided compositions and kits comprising provided compositions for analysis of sequences of the nucleic acid libraries are additional aspects of the invention. In particular aspects, analysis of the sequences of the resulting libraries enables detection of low frequency alleles in a sample of interest.

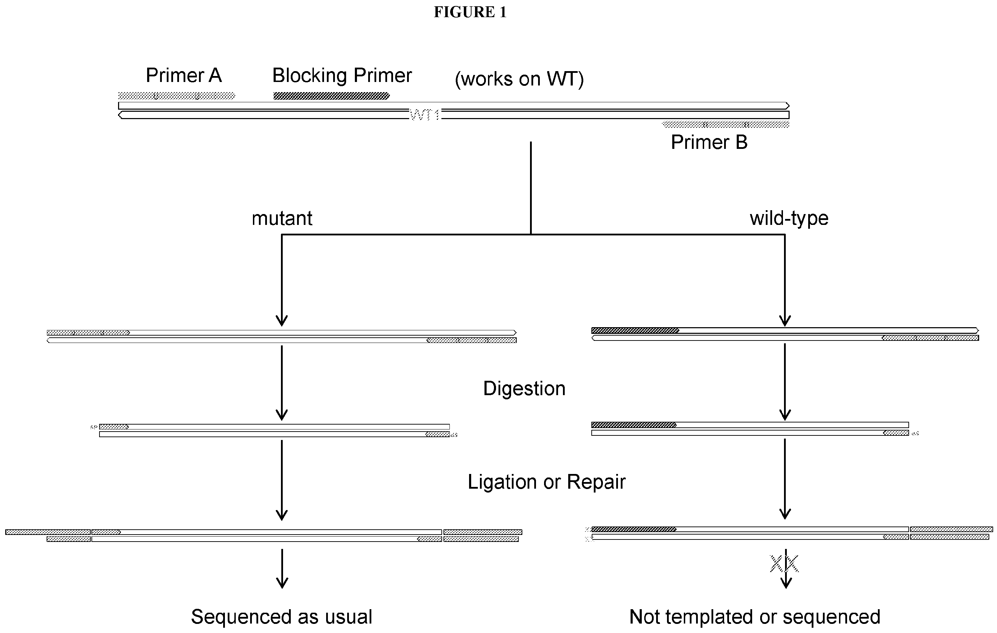

WO2013/081864 discloses e.g. in Fig. 1 target specific multiplex amplification using primers which are removed by digestion followed by a separate adapter ligation step and universal amplification. Varley et al. disclose in GENOME RESEARCH, COLD SPRING HARBOR LABORATORY PRESS, US vol. 18, no. 11, pages 1844-1850, a "Nested Patch PCR enables highly multiplexed mutation discovery in candidate genes". It involves target specific primers which are cleaved from the amplicons by the addition of heat-labile uracil DNA glycosylase, endonuclease VIII, and single-strand-specific exonuclease I. Then nested patch oligonucleotides are annealed to the target amplicons and serve as a patch between the correct amplicons and universal primers. WO2019/006392 provides library preparation methods using plurality of adaptors, through a series of amplification, digestion, and repair steps using adaptors. WO2012/149438 provides methods for multiplex PCR, various target-specific primers are provided that allow for the selective amplification of target sequences. WO2013158215 discloses methods for preparation of RNA libraries for sequencing, expression profiling, microarray and other uses and for simplification of the library preparation process.

### BRIEF DESCRIPTION OF THE DRAWINGS

The features of the invention are set forth with particularity in the appended claims. A better understanding of the features and advantages of the present invention will be obtained by reference to the detailed description that sets forth illustrative embodiments, in which the principles of the invention are utilized, and the accompanying drawings of which:
FIG. 1 depicts a schematic of an illustrative embodiment of the invention.

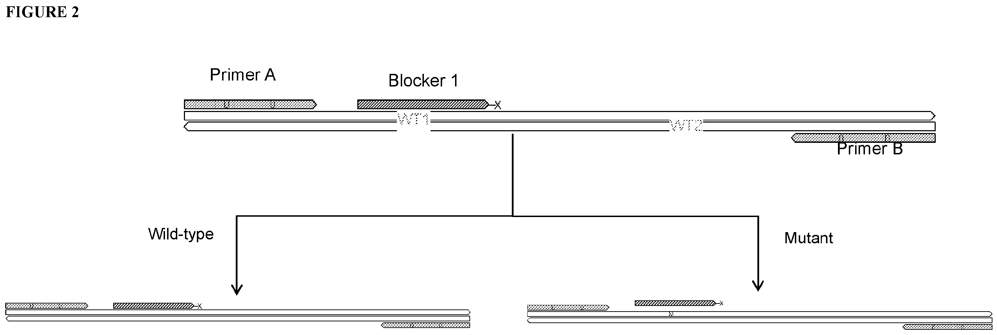

FIG. 2 depicts a schematic of alternative blocking mechanisms known in the art, e.g., DNA blockers, RNA blockers and/or LNA blockers. Blocker oligos prevent primer extension and formation of amplicon at wild type loci.

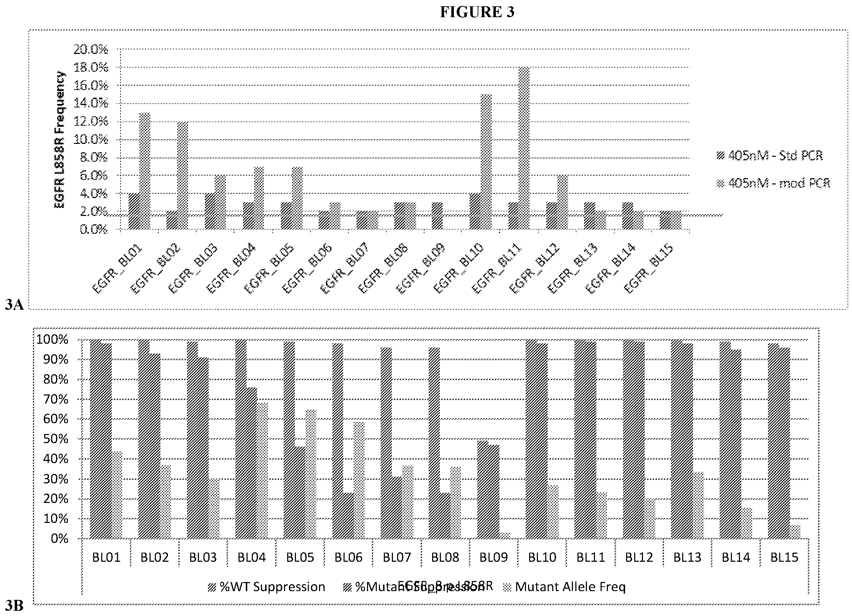

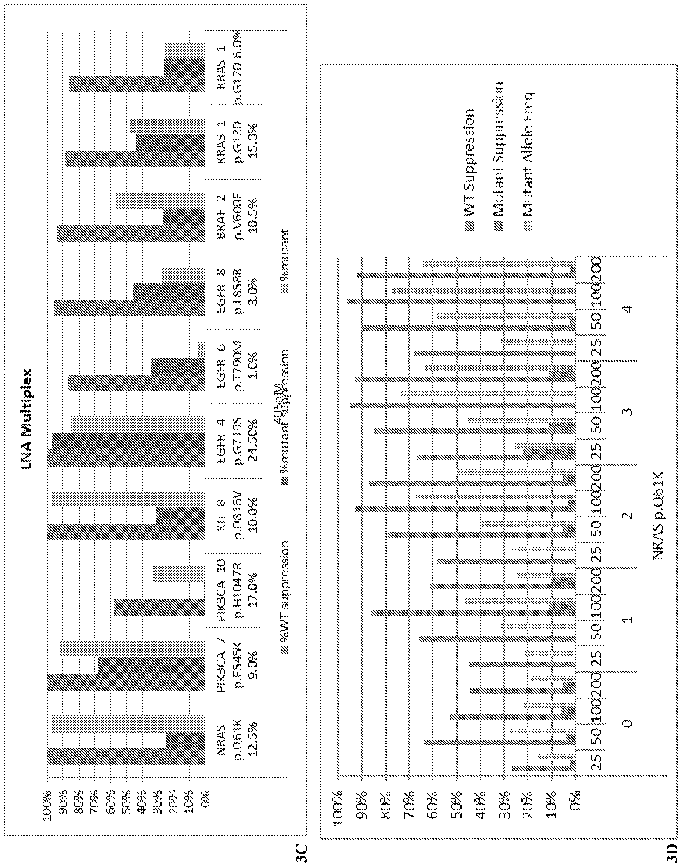

FIGS 3A-3D depict exemplary results described in Example 1. FIG 3A depicts results using DNA blocking oligos; FIG 3B depicts results using RNA blocking oligos; FIG 3C depicts results using LNA blocking oligos and FIG 3D depicts results of experiments using blocking oligos according to an embodiment of the invention.

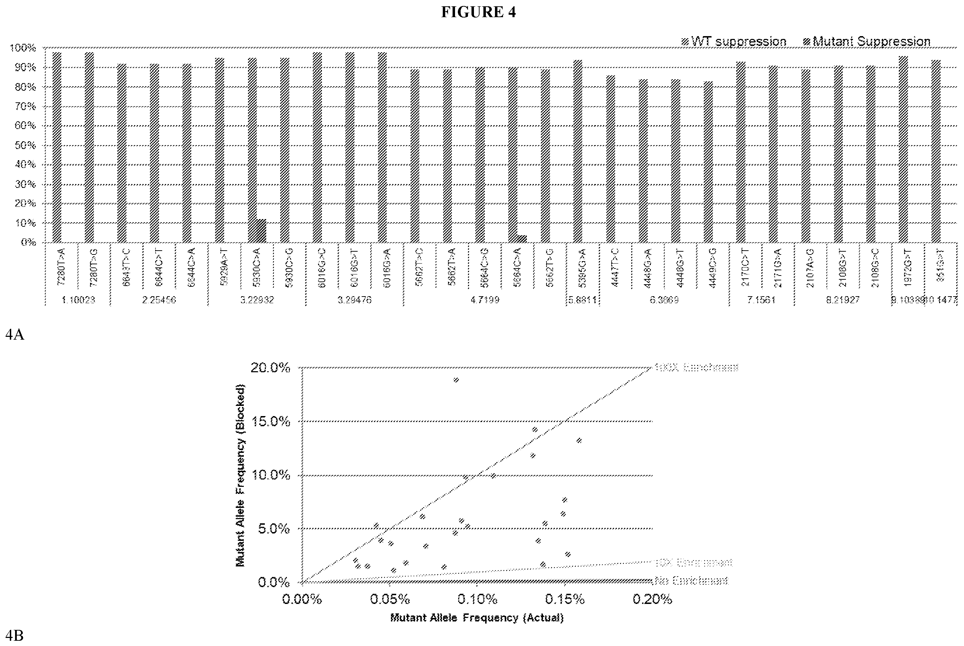

FIGS 4A-4B depict exemplary results described in Example 2. A 122-plex custom panel generated included blocking primers according to the instant invention. FIG 4A demonstrates representative results of amplicons efficiently suppressed using the described methods. FIG 4B depicts results comparing actual mutant frequency in samples to those frequencies detected in samples comprising blocking oligos.

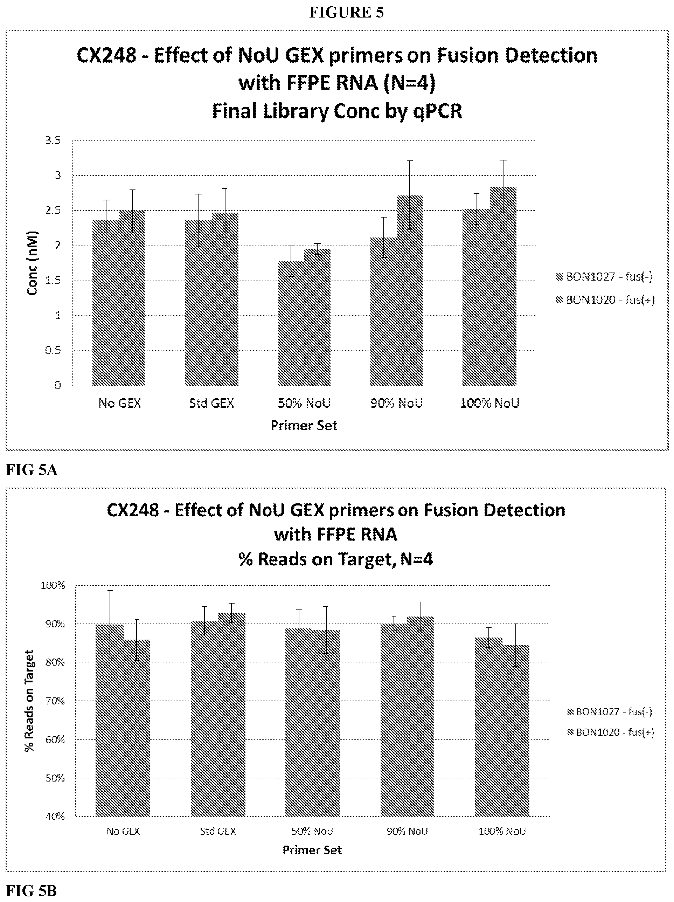

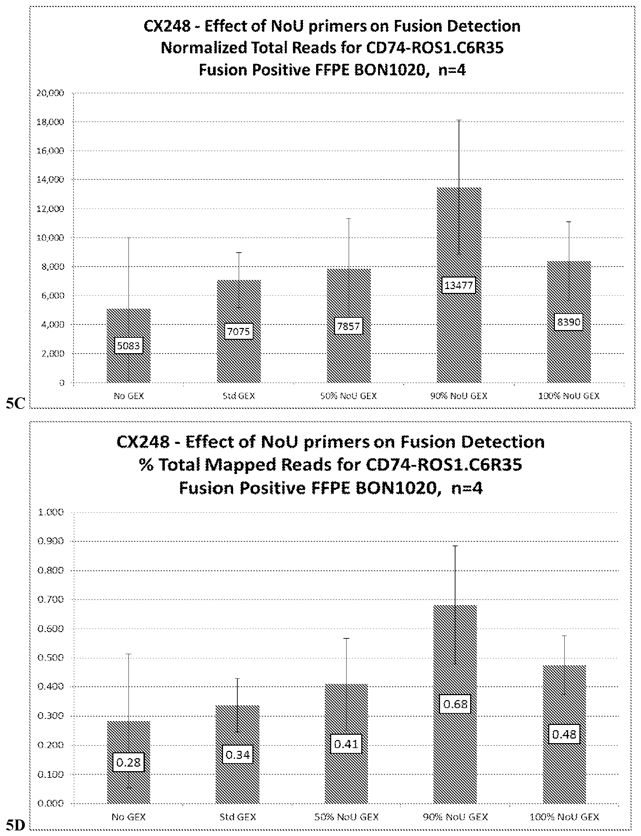

FIGS 5A-5D depict exemplary results described in Example 3. FIG 5A and 5B depict results demonstrating library preparation does not change when GEX and/or blocking primers are included in library reactions; FIG 5C depicts increase in low frequency fusion detection when blocking primers are present, trending in a concentration dependent manner; and FIG 5D depicts how percentage of total mapped reads for the detected fusion similarly increase when blocking primers are present, trending in a concentration dependent manner.

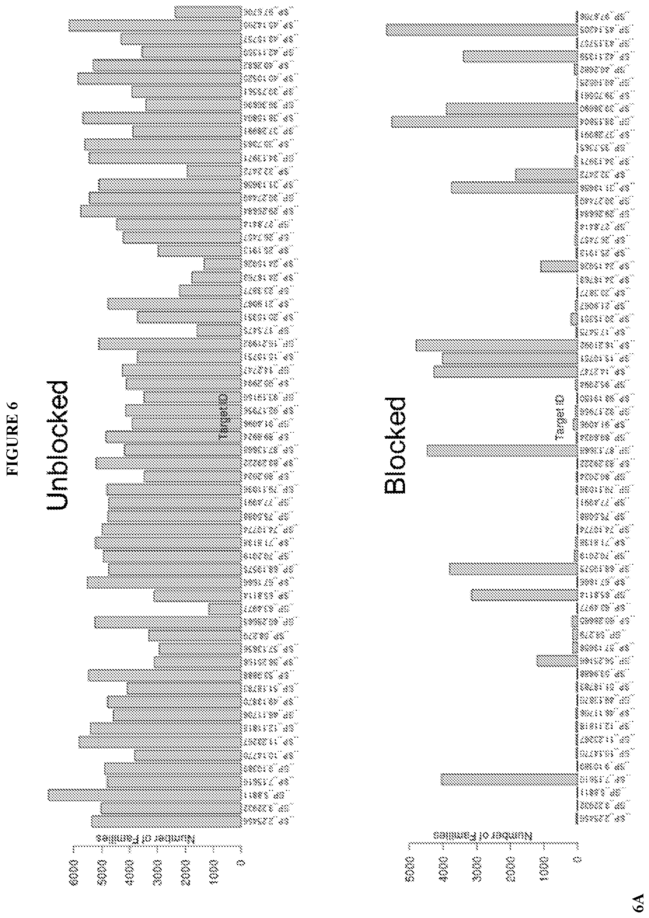

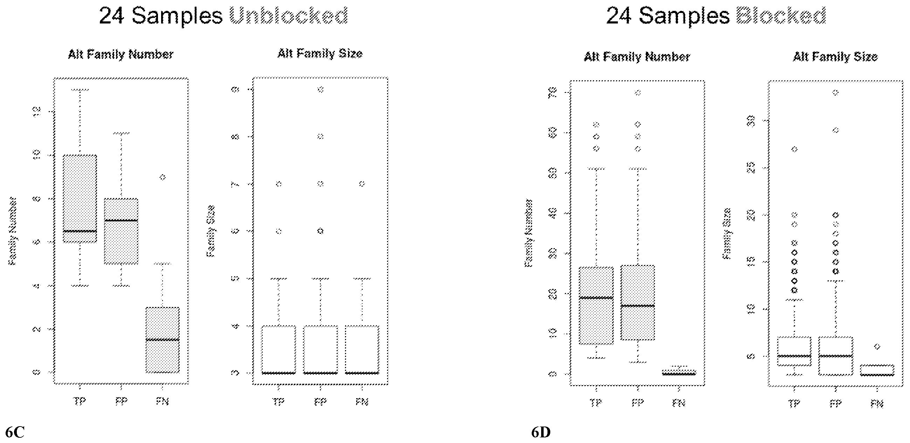

FIGS 6A-6D depict exemplary results described in Example 4. FIG 5A and FIG 5B depict results demonstrating resulting families identified for targets from Ion AmpliSeq HD experiments in unblocked and blocked experiments. FIGS 6C-6D depict in more detail increases in numbers of families, as well as sizes of families in blocked experiments for true positive as well as false positive results, as well as a decrease in both number and size of false negatives detected.

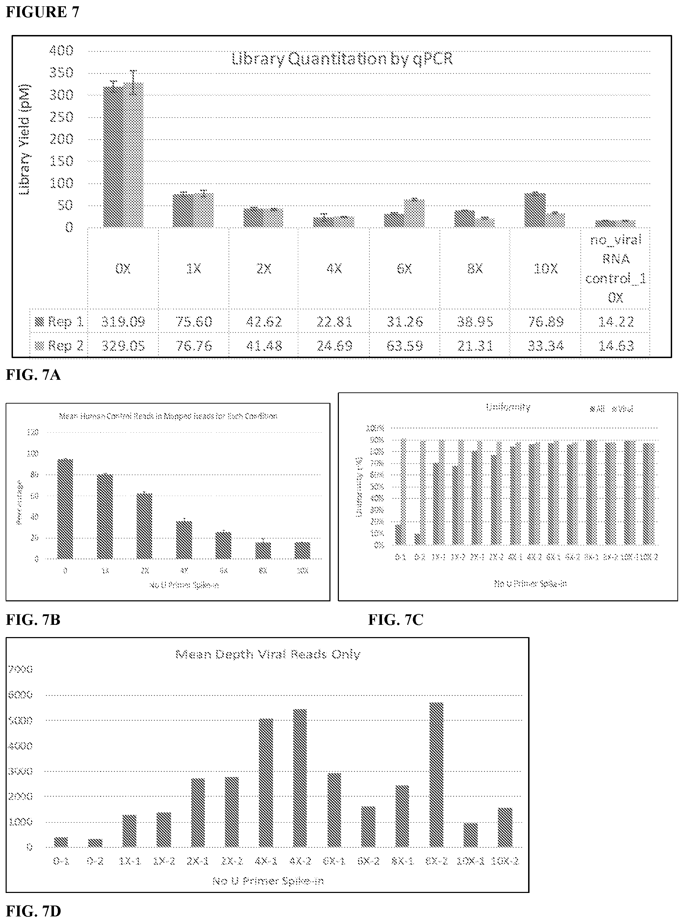

FIGS 7A-7D depict exemplary results described in Example 5. FIG 7A depicts reduction in library yield when blocking primer sets targeting human control genes are present; FIG 7B depicts reduction of human control read sequences in conjunction with increased blocking primer pairs; FIG 7C depicts results of library uniformity as well as increases in viral reads present in library with increased blocking primer pairs; and FIG 7D depicts increases in mean depth of viral reads with increases in blocking primer pair, with an apparent optimal increase at 4x blocking primer pair.

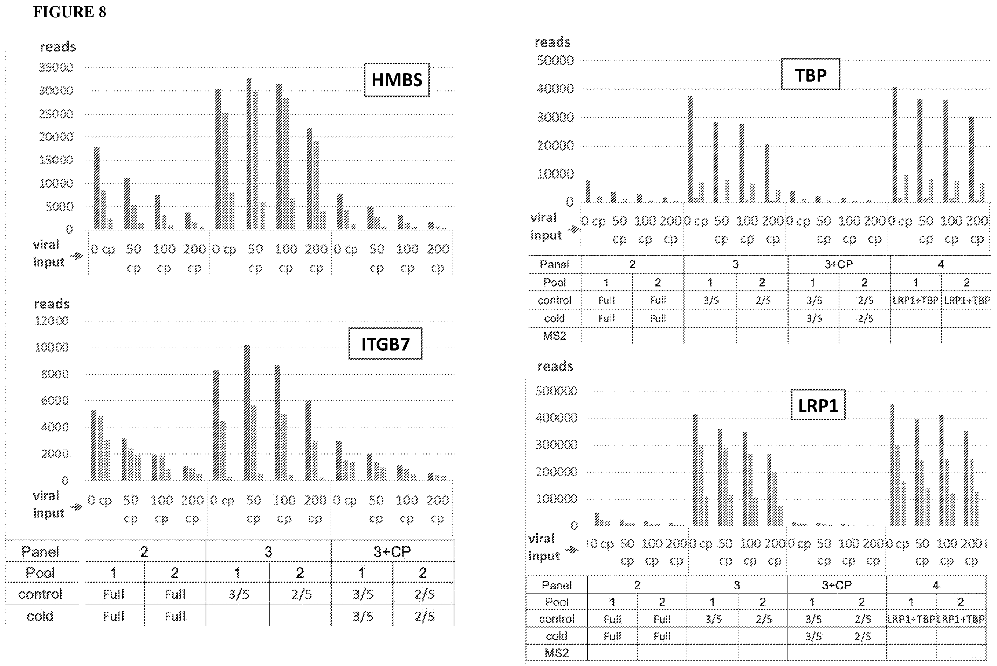

FIGS 8A-8D depict exemplary results described in Example 6. Each of FIG 8A-8D depict reduction in library yield when blocking primer sets targeting human control genes, HMBS (8A), ITGB7 (8B), TBP (8C), and LRP1 (8D), are present for each of control genes detected.

### DESCRIPTION OF THE INVENTION

Section headings used herein are for organizational purposes only and are not to be construed as limiting the described subject matter in any way. When definitions of terms in cited references appear to differ from the definitions provided in the present teachings, the definition provided in the present teachings shall control. It will be appreciated that there is an implied "about" prior to the temperatures, concentrations, times, etc discussed in the present teachings, such that slight and insubstantial deviations are within the scope of the present teachings herein. In this application, the use of the singular includes the plural unless specifically stated otherwise. It is noted that, as used in this specification, singular forms "a," "an," and "the," and any singular use of a word, include plural referents unless expressly and unequivocally limited to one referent. Also, the use of "comprise", "comprises", "comprising", "contain", "contains", "containing", "include", "includes", and "including" are not intended to be limiting. It is to be understood that both the general description is exemplary and explanatory only and not restrictive of the disclosure.

Unless otherwise defined, scientific and technical terms used in connection with the disclosure described herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization used herein are those well-known and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. Techniques and procedures described herein are generally performed according to conventional methods well known in the art and as described in various general and more specific references that are cited and discussed throughout the instant specification. *See, e.g.,* Sambrook et al., Molecular Cloning: A Laboratory Manual (Third ed., Cold Spring Harbor Laboratory Press, Cold Spring Harbor, N.Y. 2000). Unless specifically provided, any nomenclature utilized in connection with, and laboratory procedures and techniques described herein are those well-known and commonly used in the art. As utilized in accordance with embodiments provided herein, the following terms, unless otherwise indicated, shall be understood to have the following meanings:

As used herein, "amplify", "amplifying" or "amplification reaction" and their derivatives, refer generally to an action or process whereby at least a portion of a nucleic acid molecule (referred to as a template nucleic acid molecule) is replicated or copied into at least one additional nucleic acid molecule. The additional nucleic acid molecule optionally includes sequence that is substantially identical or substantially complementary to at least some portion of the template nucleic acid molecule. A template target nucleic acid molecule may be single-stranded or double-stranded. The additional resulting replicated nucleic acid molecule may independently be single-stranded or double-stranded. In some examples, amplification includes a template-dependent in vitro enzyme-catalyzed reaction for the production of at least one copy of at least some portion of a target nucleic acid molecule or the production of at least one copy of a target nucleic acid sequence that is complementary to at least some portion of a target nucleic acid molecule. Amplification optionally includes linear or exponential replication of a nucleic acid molecule. In some aspects, such amplification is performed using isothermal conditions; in other aspects, such amplification can include thermocycling. In some aspects, the amplification is a multiplex amplification that includes simultaneous amplification of a plurality of target sequences in a single amplification reaction. At least some target sequences can be situated on the same nucleic acid molecule or on different target nucleic acid molecules included in a single amplification reaction. In some examples, "amplification" includes amplification of at least some portion of DNA- and/or RNA-based nucleic acids, whether alone, or in combination. An amplification reaction can include single or double-stranded nucleic acid substrates and can further include any amplification processes known to one of ordinary skill in the art. In some examples, an amplification reaction includes polymerase chain reaction (PCR). In some examples, an amplification reaction includes isothermal amplification.

As used herein, "amplification conditions" and derivatives (e.g., conditions for amplification, etc.) generally refers to conditions suitable for amplifying one or more nucleic acid sequences. Amplification can be linear or exponential. In some aspects, amplification conditions include isothermal conditions or alternatively include thermocycling conditions, or a combination of isothermal and thermocycling conditions. In some aspects, conditions suitable for amplifying one or more target nucleic acid sequences includes polymerase chain reaction (PCR) conditions. Typically, amplification conditions refer to a reaction mixture that is sufficient to amplify nucleic acids such as one or more target sequences, or to amplify an amplified target sequence ligated or attached to one or more adapters, e.g., an adapter-attached amplified target sequence. Generally, amplification conditions include a catalyst for amplification or for nucleic acid synthesis, for example a polymerase; a primer that possesses some degree of complementarity to the nucleic acid to be amplified; and nucleotides, such as deoxyribonucleoside triphosphates (dNTPs) to promote extension of a primer once hybridized to a nucleic acid. Amplification conditions can require hybridization or annealing of a primer to a nucleic acid, extension of the primer and a denaturing step in which the extended primer is separated from the nucleic acid sequence undergoing amplification. Typically, though not necessarily, amplification conditions can include thermocycling. In some aspects, amplification conditions include a plurality of cycles wherein steps of annealing, extending and separating are repeated. Typically, amplification conditions include cations such as Mg⁺⁺ or Mn⁺⁺ (e.g., MgCl₂, etc) and can also optionally include various modifiers of ionic strength.

As used herein, "target sequence" "target nucleic acid sequence" or "target sequence of interest" and derivatives, refers generally to any single or double-stranded nucleic acid sequence that can be amplified or synthesized according to the disclosure, including any nucleic acid sequence suspected or expected to be present in a sample. In some examples, the target sequence is present in double-stranded form and includes at least a portion of the particular nucleotide sequence to be amplified or synthesized, or its complement, prior to the addition of target-specific primers or appended adapters. Target sequences can include the nucleic acids to which primers useful in the amplification or synthesis reaction can hybridize prior to extension by a polymerase. In some aspects, the term refers to a nucleic acid sequence whose sequence identity, ordering or location of nucleotides is determined by one or more of the methods of the disclosure.

The term "portion" and its variants, as used herein, when used in reference to a given nucleic acid molecule, for example a primer or a template nucleic acid molecule, comprises any number of contiguous nucleotides within the length of the nucleic acid molecule, including the partial or entire length of the nucleic acid molecule.

As used herein, "contacting" and its derivatives, when used in reference to two or more components, refers generally to any process whereby the approach, proximity, mixture or commingling of the referenced components is promoted or achieved without necessarily requiring physical contact of such components, and includes mixing of solutions containing any one or more of the referenced components with each other. The referenced components may be contacted in any particular order or combination and the particular order of recitation of components is not limiting. For example, "contacting A with B and C" encompasses embodiments where A is first contacted with B then C, as well as embodiments where C is contacted with A then B, as well as embodiments where a mixture of A and C is contacted with B, and the like. Furthermore, such contacting does not necessarily require that the end result of the contacting process be a mixture including all of the referenced components, as long as at some point during the contacting process all of the referenced components are simultaneously present or simultaneously included in the same mixture or solution. For example, "contacting A with B and C" can include embodiments wherein C is first contacted with A to form a first mixture, which first mixture is then contacted with B to form a second mixture, following which C is removed from the second mixture; optionally A can then also be removed, leaving only B. Where one or more of the referenced components to be contacted includes a plurality (e.g., "contacting a target sequence with a plurality of target-specific primers and a polymerase"), then each member of the plurality can be viewed as an individual component of the contacting process, such that the contacting can include contacting of any one or more members of the plurality with any other member of the plurality and/or with any other referenced component (e.g., some but not all of the plurality of target specific primers can be contacted with a target sequence, then a polymerase, and then with other members of the plurality of target-specific primers) in any order or combination.

As used herein, the term "primer" and its derivatives refer generally to any polynucleotide that can hybridize to a target sequence of interest. In some aspects, the primer can also serve to prime nucleic acid synthesis. Typically, a primer functions as a substrate onto which nucleotides can be polymerized by a polymerase; in some aspects, however, a primer can become incorporated into a synthesized nucleic acid strand and provide a site to which another primer can hybridize to prime synthesis of a new strand that is complementary to the synthesized nucleic acid molecule. A primer may be comprised of any combination of nucleotides or analogs thereof, which may be optionally linked to form a linear polymer of any suitable length. In some aspects, a primer is a single-stranded oligonucleotide or polynucleotide. (For purposes of this disclosure, the terms 'polynucleotide" and "oligonucleotide" are used interchangeably herein and do not necessarily indicate any difference in length between the two). In some aspects, a primer is double-stranded. If double stranded, a primer is first treated to separate its strands before being used to prepare extension products. Preferably, the primer is an oligodeoxyribonucleotide. A primer must be sufficiently long to prime the synthesis of extension products. Lengths of the primers will depend on many factors, including temperature, source of primer and the use of the method. In some examples, a primer acts as a point of initiation for amplification or synthesis when exposed to amplification or synthesis conditions; such amplification or synthesis can occur in a template-dependent fashion and optionally results in formation of a primer extension product that is complementary to at least a portion of the target sequence. Exemplary amplification or synthesis conditions can include contacting the primer with a polynucleotide template (e.g., a template including a target sequence), nucleotides and an inducing agent such as a polymerase at a suitable temperature and pH to induce polymerization of nucleotides onto an end of the target-specific primer. If double-stranded, the primer can optionally be treated to separate its strands before being used to prepare primer extension products. In some examples, the primer is an oligodeoxyribonucleotide or an oligoribonucleotide. In some examples, the primer can include one or more nucleotide analogs. The exact length and/or composition, including sequence, of the target-specific primer can influence many properties, including melting temperature (Tm), GC content, formation of secondary structures, repeat nucleotide motifs, length of predicted primer extension products, extent of coverage across a nucleic acid molecule of interest, number of primers present in a single amplification or synthesis reaction, presence of nucleotide analogs or modified nucleotides within the primers, and the like. In some examples, a primer can be paired with a compatible primer within an amplification or synthesis reaction to form a primer pair consisting or a forward primer and a reverse primer. In some examples, the forward primer of the primer pair includes a sequence that is substantially complementary to at least a portion of a strand of a nucleic acid molecule, and the reverse primer of the primer of the primer pair includes a sequence that is substantially identical to at least of portion of the strand. In some examples, the forward primer and the reverse primer are capable of hybridizing to opposite strands of a nucleic acid duplex. Optionally, the forward primer primes synthesis of a first nucleic acid strand, and the reverse primer primes synthesis of a second nucleic acid strand, wherein the first and second strands are substantially complementary to each other, or can hybridize to form a double-stranded nucleic acid molecule. In some examples, one end of an amplification or synthesis product is defined by the forward primer and the other end of the amplification or synthesis product is defined by the reverse primer. In some examples, where the amplification or synthesis of lengthy primer extension products is required, such as amplifying an exon, coding region, or gene, several primer pairs can be created than span the desired length to enable sufficient amplification of the region. In some examples, a primer can include one or more cleavable groups. In some examples, primer lengths are in the range of about 10 to about 60 nucleotides, about 12 to about 50 nucleotides and about 15 to about 40 nucleotides in length. Typically, a primer is capable of hybridizing to a corresponding target sequence and undergoing primer extension when exposed to amplification conditions in the presence of dNTPS and a polymerase. In some instances, the particular nucleotide sequence or a portion of the primer is known at the outset of the amplification reaction or can be determined by one or more of the methods disclosed herein. In some examples, the primer includes one or more cleavable groups at one or more locations within the primer.

As used herein, "target-specific primer" and its derivatives, refers generally to a single stranded or double-stranded polynucleotide, typically an oligonucleotide, that includes at least one sequence that is at least 50% complementary, typically at least 75% complementary or at least 85% complementary, more typically at least 90% complementary, more typically at least 95% complementary, more typically at least 98% or at least 99% complementary, or identical, to at least a portion of a nucleic acid molecule that includes a target sequence. In such instances, the target-specific primer and target sequence are described as "corresponding" to each other. In some aspects, the target-specific primer is capable of hybridizing to at least a portion of its corresponding target sequence (or to a complement of the target sequence); such hybridization can optionally be performed under standard hybridization conditions or under stringent hybridization conditions. In some examples, the target-specific primer is not capable of hybridizing to the target sequence, or to its complement, but is capable of hybridizing to a portion of a nucleic acid strand including the target sequence, or to its complement. In some examples, the target-specific primer includes at least one sequence that is at least 75% complementary, typically at least 85% complementary, more typically at least 90% complementary, more typically at least 95% complementary, more typically at least 98% complementary, or more typically at least 99% complementary, to at least a portion of the target sequence itself; in other examples, the target-specific primer includes at least one sequence that is at least 75% complementary, typically at least 85% complementary, more typically at least 90% complementary, more typically at least 95% complementary, more typically at least 98% complementary, or more typically at least 99% complementary, to at least a portion of the nucleic acid molecule other than the target sequence. In some aspects, the target-specific primer is substantially non-complementary to other target sequences present in the sample; optionally, the target-specific primer is substantially non-complementary to other nucleic acid molecules present in the sample. In some examples, nucleic acid molecules present in the sample that do not include or correspond to a target sequence (or to a complement of the target sequence) are referred to as "non-specific" sequences or "non-specific nucleic acids". In some examples, the target-specific primer is designed to include a nucleotide sequence that is substantially complementary to at least a portion of its corresponding target sequence. In some examples, a target-specific primer is at least 95% complementary, or at least 99% complementary, or identical, across its entire length to at least a portion of a nucleic acid molecule that includes its corresponding target sequence. In some examples, a target-specific primer can be at least 90%, at least 95% complementary, at least 98% complementary or at least 99% complementary, or identical, across its entire length to at least a portion of its corresponding target sequence. In some examples, a forward target-specific primer and a reverse target-specific primer define a target-specific primer pair that can be used to amplify the target sequence via template-dependent primer extension. Typically, each primer of a target-specific primer pair includes at least one sequence that is substantially complementary to at least a portion of a nucleic acid molecule including a corresponding target sequence but that is less than 50% complementary to at least one other target sequence in the sample. In some examples, amplification can be performed using multiple target-specific primer pairs in a single amplification reaction, wherein each primer pair includes a forward target-specific primer and a reverse target-specific primer, each including at least one sequence that substantially complementary or substantially identical to a corresponding target sequence in the sample, and each primer pair having a different corresponding target sequence. In some examples, the target-specific primer can be substantially non-complementary at its 3' end or its 5' end to any other target-specific primer present in an amplification reaction. In some examples, the target-specific primer can include minimal cross hybridization to other target-specific primers in the amplification reaction. In some examples, target-specific primers include minimal cross-hybridization to non-specific sequences in the amplification reaction mixture. In some examples, the target-specific primers include minimal self-complementarity. In some examples, the target-specific primers can include one or more cleavable groups located at the 3' end. In some examples, the target-specific primers can include one or more cleavable groups located near or about a central nucleotide of the target-specific primer. In some examples, one of more targets-specific primers includes only non-cleavable nucleotides at the 5' end of the target-specific primer. In some examples, a target specific primer includes minimal nucleotide sequence overlap at the 3'end or the 5' end of the primer as compared to one or more different target-specific primers, optionally in the same amplification reaction. In some examples 1, 2, 3, 4, 5, 6, 7, 8, 9, 10 or more, target-specific primers in a single reaction mixture include one or more of the above examples. In some examples, substantially all of the plurality of target-specific primers in a single reaction mixture includes one or more of the above embodiments.

As used herein, the term "adapter" denotes a nucleic acid molecule that can be used for manipulation of a polynucleotide of interest. In some examples, adapters are used for amplification of one or more target nucleic acids. In some examples, the adapters are used in reactions for sequencing. In some examples, an adapter has one or more ends that lack a 5' phosphate residue. In some examples, an adapter comprises, consists of, or consist essentially of at least one priming site. Such priming site containing adapters can be referred to as "primer" adapters. In some examples, the adapter priming site can be useful in PCR processes. In some examples an adapter includes a nucleic acid sequence that is substantially complementary to the 3' end or the 5' end of at least one target sequences within the sample, referred to herein as a gene specific target sequence, a target specific sequence, or target specific primer. In some examples, the adapter includes nucleic acid sequence that is substantially non-complementary to the 3' end or the 5' end of any target sequence present in the sample. In some examples, the adapter includes single stranded or double-stranded linear oligonucleotide that is not substantially complementary to an target nucleic acid sequence. In some examples, the adapter includes nucleic acid sequence that is substantially non-complementary to at least one, and preferably some or all of the nucleic acid molecules of the sample. In some examples, suitable adapter lengths are in the range of about 10-75 nucleotides, about 12-50 nucleotides and about 15-40 nucleotides in length. Generally, an adapter can include any combination of nucleotides and/or nucleic acids. In some aspects, adapters include one or more cleavable groups at one or more locations. In some aspects, the adapter includes sequence that is substantially identical, or substantially complementary, to at least a portion of a primer, for example a universal primer. In some examples, adapters include a tag sequence to assist with cataloguing, identification or sequencing. In some aspects, an adapter acts as a substrate for amplification of a target sequence, particularly in the presence of a polymerase and dNTPs under suitable temperature and pH.

As used herein, "polymerase" and its derivatives, generally refers to any enzyme that can catalyze the polymerization of nucleotides (including analogs thereof) into a nucleic acid strand. Typically, but not necessarily, such nucleotide polymerization can occur in a template-dependent fashion. Such polymerases can include without limitation naturally occurring polymerases and any subunits and truncations thereof, mutant polymerases, variant polymerases, recombinant, fusion or otherwise engineered polymerases, chemically modified polymerases, synthetic molecules or assemblies, and any analogs, derivatives or fragments thereof that retain the ability to catalyze such polymerization. Optionally, the polymerase can be a mutant polymerase comprising one or more mutations involving the replacement of one or more amino acids with other amino acids, the insertion or deletion of one or more amino acids from the polymerase, or the linkage of parts of two or more polymerases. Typically, the polymerase comprises one or more active sites at which nucleotide binding and/or catalysis of nucleotide polymerization can occur. Some exemplary polymerases include without limitation DNA polymerases and RNA polymerases. The term "polymerase" and its variants, as used herein, also refers to fusion proteins comprising at least two portions linked to each other, where the first portion comprises a peptide that can catalyze the polymerization of nucleotides into a nucleic acid strand and is linked to a second portion that comprises a second polypeptide. In some examples, the second polypeptide can include a reporter enzyme or a processivity-enhancing domain. Optionally, the polymerase can possess 5' exonuclease activity or terminal transferase activity. In some examples, the polymerase can be optionally reactivated, for example through the use of heat, chemicals or re-addition of new amounts of polymerase into a reaction mixture. In some aspects, the polymerase can include a hot-start polymerase and/or an aptamer based polymerase that optionally can be reactivated.

The terms "identity" and "identical" and their variants, as used herein, when used in reference to two or more nucleic acid sequences, refer to similarity in sequence of the two or more sequences (e.g., nucleotide or polypeptide sequences). In the context of two or more homologous sequences, the percent identity or homology of the sequences or subsequences thereof indicates the percentage of all monomeric units (e.g., nucleotides or amino acids) that are the same (i.e., about 70% identity, preferably 75%, 80%, 85%, 90%, 95%, 98% or 99% identity). The percent identity can be over a specified region, when compared and aligned for maximum correspondence over a comparison window, or designated region as measured using a BLAST or BLAST 2.0 sequence comparison algorithms with default parameters described below, or by manual alignment and visual inspection. Sequences are said to be "substantially identical" when there is at least 85% identity at the amino acid level or at the nucleotide level. Preferably, the identity exists over a region that is at least about 25, 50, or 100 residues in length, or across the entire length of at least one compared sequence. A typical algorithm for determining percent sequence identity and sequence similarity are the BLAST and BLAST 2.0 algorithms, which are described in Altschul et al, Nuc. Acids Res. 25:3389-3402 (1977). Other methods include the algorithms of Smith & Waterman, Adv. Appl. Math. 2:482 (1981), and Needleman & Wunsch, J. Mol. Biol. 48:443 (1970), etc. Another indication that two nucleic acid sequences are substantially identical is that the two molecules or their complements hybridize to each other under stringent hybridization conditions.

The terms "complementary" and "complement" and their variants, as used herein, refer to any two or more nucleic acid sequences (e.g., portions or entireties of template nucleic acid molecules, target sequences and/or primers) that can undergo cumulative base pairing at two or more individual corresponding positions in antiparallel orientation, as in a hybridized duplex. Such base pairing can proceed according to any set of established rules, for example according to Watson-Crick base pairing rules or according to some other base pairing paradigm. Optionally there can be "complete" or "total" complementarity between a first and second nucleic acid sequence where each nucleotide in the first nucleic acid sequence can undergo a stabilizing base pairing interaction with a nucleotide in the corresponding antiparallel position on the second nucleic acid sequence. "Partial" complementarity describes nucleic acid sequences in which at least 20%, but less than 100%, of the residues of one nucleic acid sequence are complementary to residues in the other nucleic acid sequence. In some examples, at least 50%, but less than 100%, of the residues of one nucleic acid sequence are complementary to residues in the other nucleic acid sequence. In some examples, at least 70%, 80%, 90%, 95% or 98%, but less than 100%, of the residues of one nucleic acid sequence are complementary to residues in the other nucleic acid sequence. Sequences are said to be "substantially complementary" when at least 85% of the residues of one nucleic acid sequence are complementary to residues in the other nucleic acid sequence. In some examples, two complementary or substantially complementary sequences are capable of hybridizing to each other under standard or stringent hybridization conditions. "Non-complementary" describes nucleic acid sequences in which less than 20% of the residues of one nucleic acid sequence are complementary to residues in the other nucleic acid sequence. Sequences are said to be "substantially non-complementary" when less than 15% of the residues of one nucleic acid sequence are complementary to residues in the other nucleic acid sequence. In some examples, two non-complementary or substantially non-complementary sequences cannot hybridize to each other under standard or stringent hybridization conditions. A "mismatch" is present at any position in the two opposed nucleotides are not complementary. Complementary nucleotides include nucleotides that are efficiently incorporated by DNA polymerases opposite each other during DNA replication under physiological conditions. In a typical example, complementary nucleotides can form base pairs with each other, such as the A-T/U and G-C base pairs formed through specific Watson-Crick type hydrogen bonding, or base pairs formed through some other type of base pairing paradigm, between the nucleobases of nucleotides and/or polynucleotides in positions antiparallel to each other. The complementarity of other artificial base pairs can be based on other types of hydrogen bonding and/or hydrophobicity of bases and/or shape complementarity between bases.

As used herein, "amplified target sequences" and its derivatives, refers generally to a nucleic acid sequence produced by the amplification of/amplifying the target sequences using target-specific primers and the methods provided herein. The amplified target sequences may be either of the same sense (the positive strand produced in the second round and subsequent even-numbered rounds of amplification) or antisense (i.e., the negative strand produced during the first and subsequent odd-numbered rounds of amplification) with respect to the target sequences. For the purposes of this disclosure, amplified target sequences are typically less than 50% complementary to any portion of another amplified target sequence in the reaction.

As used herein, terms "ligating", "ligation" and derivatives refer generally to the act or process for covalently linking two or more molecules together, for example, covalently linking two or more nucleic acid molecules to each other. In some examples, ligation includes joining nicks between adjacent nucleotides of nucleic acids. In some examples, ligation includes forming a covalent bond between an end of a first and an end of a second nucleic acid molecule. In some embodiments, for example embodiments wherein the nucleic acid molecules to be ligated include conventional nucleotide residues, the ligation can include forming a covalent bond between a 5' phosphate group of one nucleic acid and a 3' hydroxyl group of a second nucleic acid thereby forming a ligated nucleic acid molecule. In some examples, any means for joining nicks or bonding a 5'phosphate to a 3' hydroxyl between adjacent nucleotides can be employed. In an exemplary aspect, an enzyme such as a ligase can be used.

As used herein, "ligase" and its derivatives, refers generally to any agent capable of catalyzing the ligation of two substrate molecules. In some examples, the ligase includes an enzyme capable of catalyzing the joining of nicks between adjacent nucleotides of a nucleic acid. In some examples, a ligase includes an enzyme capable of catalyzing the formation of a covalent bond between a 5' phosphate of one nucleic acid molecule to a 3' hydroxyl of another nucleic acid molecule thereby forming a ligated nucleic acid molecule. Suitable ligases may include, but not limited to, T4 DNA ligase; T7 DNA ligase; Taq DNA ligase, and *E*. *coli* DNA ligase.

As defined herein, a "cleavable group" generally refers to any moiety that once incorporated into a nucleic acid can be cleaved under appropriate conditions. For example, a cleavable group can be incorporated into a target-specific primer, an amplified sequence, an adapter or a nucleic acid molecule of the sample. In an exemplary embodiment, a target-specific primer can include a cleavable group that becomes incorporated into the amplified product and is subsequently cleaved after amplification, thereby removing a portion, or all, of the target-specific primer from the amplified product. The cleavable group can be cleaved or otherwise removed from a target-specific primer, an amplified sequence, an adapter or a nucleic acid molecule of the sample by any acceptable means. For example, a cleavable group can be removed from a target-specific primer, an amplified sequence, an adapter or a nucleic acid molecule of the sample by enzymatic, thermal, photo-oxidative or chemical treatment. In one aspect, a cleavable group can include a nucleobase that is not naturally occurring. For example, an oligodeoxyribonucleotide can include one or more RNA nucleobases, such as uracil that can be removed by a uracil glycosylase. In some aspects, a cleavable group can include one or more modified nucleobases (such as 7-methylguanine, 8-oxo-guanine, xanthine, hypoxanthine, 5,6-dihydrouracil or 5-methylcytosine) or one or more modified nucleosides (i.e., 7-methylguanosine, 8-oxo-deoxyguanosine, xanthosine, inosine, dihydrouridine or 5-methylcytidine). The modified nucleobases or nucleotides can be removed from the nucleic acid by enzymatic, chemical or thermal means. In one aspect, a cleavable group can include a moiety that can be removed from a primer after amplification (or synthesis) upon exposure to ultraviolet light (i.e., bromodeoxyuridine). In another aspect, a cleavable group can include methylated cytosine. Typically, methylated cytosine can be cleaved from a primer for example, after induction of amplification (or synthesis), upon sodium bisulfite treatment. In some examples, a cleavable moiety can include a restriction site. For example, a primer or target sequence can include a nucleic acid sequence that is specific to one or more restriction enzymes, and following amplification (or synthesis), the primer or target sequence can be treated with the one or more restriction enzymes such that the cleavable group is removed. Typically, one or more cleavable groups can be included at one or more locations with a target-specific primer, an amplified sequence, an adapter or a nucleic acid molecule of the sample.

As used herein, "digestion", "digestion step" and its derivatives, generally refers to any process by which a cleavable group is cleaved or otherwise removed from a target-specific primer, an amplified sequence, an adapter or a nucleic acid molecule of the sample. In some examples, the digestion step involves a chemical, thermal, photo-oxidative or digestive process.

As used herein, the term "hybridization" is consistent with its use in the art, and generally refers to the process whereby two nucleic acid molecules undergo base pairing interactions. Two nucleic acid molecule molecules are said to be hybridized when any portion of one nucleic acid molecule is base paired with any portion of the other nucleic acid molecule; it is not necessarily required that the two nucleic acid molecules be hybridized across their entire respective lengths and in some aspects, at least one of the nucleic acid molecules can include portions that are not hybridized to the other nucleic acid molecule. The phrase "hybridizing under stringent conditions" and its variants refers generally to conditions under which hybridization of a target-specific primer to a target sequence occurs in the presence of high hybridization temperature and low ionic strength. As used herein, the phrase "standard hybridization conditions" and its variants refers generally to conditions under which hybridization of a primer to an oligonucleotide (i.e., a target sequence), occurs in the presence of low hybridization temperature and high ionic strength. In one exemplary embodiment, standard hybridization conditions include an aqueous environment containing about 100 mm magnesium sulfate, about 500 mM Tris-sulfate at pH 8.9, and about 200 mM ammonium sulfate at about 50-55°C., or equivalents thereof.

As used herein, the term "end" and its variants, when used in reference to a nucleic acid molecule, for example a target sequence or amplified target sequence, can include the terminal 30 nucleotides, the terminal 20 and even more typically the terminal 15 nucleotides of the nucleic acid molecule. A linear nucleic acid molecule comprised of linked series of contiguous nucleotides typically includes at least two ends. In some examples, one end of the nucleic acid molecule can include a 3' hydroxyl group or its equivalent, and can be referred to as the "3' end" and its derivatives. Optionally, the 3' end includes a 3' hydroxyl group that is not linked to a 5' phosphate group of a mononucleotide pentose ring. Typically, the 3' end includes one or more 5' linked nucleotides located adjacent to the nucleotide including the unlinked 3' hydroxyl group, typically the 30 nucleotides located adjacent to the 3' hydroxyl, typically the terminal 20 and even more typically the terminal 15 nucleotides. Generally, the one or more linked nucleotides can be represented as a percentage of the nucleotides present in the oligonucleotide or can be provided as a number of linked nucleotides adjacent to the unlinked 3' hydroxyl. For example, the 3' end can include less than 50% of the nucleotide length of the oligonucleotide. In some aspects, the 3' end does not include any unlinked 3' hydroxyl group but can include any moiety capable of serving as a site for attachment of nucleotides via primer extension and/or nucleotide polymerization. In some aspects, the term "3' end" for example when referring to a target-specific primer, can include the terminal 10 nucleotides, the terminal 5 nucleotides, the terminal 4, 3, 2 or fewer nucleotides at the 3'end. In some aspects, the term "3' end" when referring to a target-specific primer can include nucleotides located at nucleotide positions 10 or fewer from the 3' terminus. As used herein, "5' end", and its derivatives, generally refers to an end of a nucleic acid molecule, for example a target sequence or amplified target sequence, which includes a free 5' phosphate group or its equivalent. In some aspects, the 5' end includes a 5' phosphate group that is not linked to a 3' hydroxyl of a neighboring mononucleotide pentose ring. Typically, the 5' end includes to one or more linked nucleotides located adjacent to the 5' phosphate, typically the 30 nucleotides located adjacent to the nucleotide including the 5' phosphate group, typically the terminal 20 and even more typically the terminal 15 nucleotides. Generally, the one or more linked nucleotides can be represented as a percentage of the nucleotides present in the oligonucleotide or can be provided as a number of linked nucleotides adjacent to the 5' phosphate. For example, the 5' end can be less than 50% of the nucleotide length of an oligonucleotide. In another exemplary embodiment, the 5' end can include about 15 nucleotides adjacent to the nucleotide including the terminal 5' phosphate. In some examples, the 5' end does not include any unlinked 5' phosphate group but can include any moiety capable of serving as a site of attachment to a 3' hydroxyl group, or to the 3'end of another nucleic acid molecule. In some aspects, the term "5' end" for example when referring to a target-specific primer, can include the terminal 10 nucleotides, the terminal 5 nucleotides, the terminal 4, 3, 2 or fewer nucleotides at the 5'end. In some aspects, the term "5' end" when referring to a target-specific primer can include nucleotides located at positions 10 or fewer from the 5' terminus. In some aspects, the 5' end of a target-specific primer can include only non-cleavable nucleotides, for example nucleotides that do not contain one or more cleavable groups as disclosed herein, or a cleavable nucleotide as would be readily determined by one of ordinary skill in the art. A "first end" and a "second end" of a polynucleotide refer to the 5' end or the 3'end of the polynucleotide. Either the first end or second end of a polynucleotide can be the 5' end or the 3' end of the polynucleotide; the terms "first" and "second" are not meant to denote that the end is specifically the 5' end or the 3' end.

As used herein "tag," "barcode," "unique tag" or "tag sequence" and its derivatives, refers generally to a unique short (6-14 nucleotide) nucleic acid sequence within an adapter or primer that can act as a 'key' to distinguish or separate a plurality of amplified target sequences in a sample. For the purposes of this disclosure, a barcode or unique tag sequence is incorporated into the nucleotide sequence of an adapter or primer. As used herein, "barcode sequence" denotes a nucleic acid fixed sequence that is sufficient to allow for the identification of a sample or source of nucleic acid sequences of interest. A barcode sequence can be, but need not be, a small section of the original nucleic acid sequence on which the identification is to be based. In some examples a barcode is 5-20 nucleic acids long. In some examples, the barcode is comprised of analog nucleotides, such as L-DNA, LNA, PNA, etc. As used herein, "unique tag sequence" denotes a nucleic acid sequence having at least one random sequence and at least one fixed sequence. A unique tag sequence, alone or in conjunction with a second unique tag sequence, is sufficient to allow for the identification of a single target nucleic acid molecule in a sample. A unique tag sequence can, but need not, comprise a small section of the original target nucleic acid sequence. In some aspects a unique tag sequence is 2-50 nucleotides or base-pairs, or 2-25 nucleotides or base-pairs, or 2-10 nucleotides or base-pairs in length. A unique tag sequence can comprise at least one random sequence interspersed with a fixed sequence.

As used herein, "comparable maximal minimum melting temperatures" and its derivatives, refers generally to the melting temperature (Tm) of each nucleic acid fragment for a single adapter or target-specific primer after digestion of a cleavable groups. The hybridization temperature of each nucleic acid fragment generated by an adapter or target-specific primer is compared to determine the maximal minimum temperature required preventing hybridization of a nucleic acid sequence from the target-specific primer or adapter or fragment or portion thereof to a respective target sequence. Once the maximal hybridization temperature is known, it is possible to manipulate the adapter or target-specific primer, for example by moving the location of one or more cleavable group(s) along the length of the primer, to achieve a comparable maximal minimum melting temperature with respect to each nucleic acid fragment to thereby optimize digestion and repair steps of library preparation.

As used herein, "addition only" and its derivatives, refers generally to a series of steps in which reagents and components are added to a first or single reaction mixture. Typically, the series of steps excludes the removal of the reaction mixture from a first vessel to a second vessel in order to complete the series of steps. Generally, an addition only process excludes the manipulation of the reaction mixture outside the vessel containing the reaction mixture. Typically, an addition-only process is amenable to automation and high-throughput.

As used herein, "polymerizing conditions" and its derivatives, refers generally to conditions suitable for nucleotide polymerization. In typical embodiments, such nucleotide polymerization is catalyzed by a polymerase. In some examples, polymerizing conditions include conditions for primer extension, optionally in a template-dependent manner, resulting in the generation of a synthesized nucleic acid sequence. In some examples, the polymerizing conditions include polymerase chain reaction (PCR). Typically, the polymerizing conditions include use of a reaction mixture that is sufficient to synthesize nucleic acids and includes a polymerase and nucleotides. The polymerizing conditions can include conditions for annealing of a target-specific primer to a target sequence and extension of the primer in a template dependent manner in the presence of a polymerase. In some aspects, polymerizing conditions can be practiced using thermocycling. Additionally, polymerizing conditions can include a plurality of cycles where the steps of annealing, extending, and separating the two nucleic strands are repeated. Typically, the polymerizing conditions include a cation such as MgCl₂. Generally, polymerization of one or more nucleotides to form a nucleic acid strand includes that the nucleotides be linked to each other via phosphodiester bonds, however, alternative linkages may be possible in the context of particular nucleotide analogs.

As used herein, the term "nucleic acid" refers to natural nucleic acids, artificial nucleic acids, analogs thereof, or combinations thereof, including polynucleotides and oligonucleotides. As used herein, the terms "polynucleotide" and "oligonucleotide" are used interchangeably and mean single-stranded and double-stranded polymers of nucleotides including, but not limited to, 2'-deoxyribonucleotides (nucleic acid) and ribonucleotides (RNA) linked by internucleotide phosphodiester bond linkages, *e.g.* 3'-5' and 2'-5', inverted linkages, *e.g.* 3'-3' and 5'-5', branched structures, or analog nucleic acids. Polynucleotides have associated counter ions, such as H⁺, NH₄⁺, trialkylammonium, Mg²⁺, Na⁺ and the like. An oligonucleotide can be composed entirely of deoxyribonucleotides, entirely of ribonucleotides, or chimeric mixtures thereof. Oligonucleotides can be comprised of nucleobase and sugar analogs. Polynucleotides typically range in size from a few monomeric units, *e.g.* 5-40, when they are more commonly frequently referred to in the art as oligonucleotides, to several thousands of monomeric nucleotide units, when they are more commonly referred to in the art as polynucleotides; for purposes of this disclosure, however, both oligonucleotides and polynucleotides may be of any suitable length. Unless denoted otherwise, whenever a oligonucleotide sequence is represented, it will be understood that the nucleotides are in 5' to 3' order from left to right and that "A" denotes deoxyadenosine, "C" denotes deoxycytidine, "G" denotes deoxyguanosine, "T" denotes thymidine, and "U' denotes deoxyuridine. As discussed herein and known in the art, oligonucleotides and polynucleotides are said to have "5' ends" and "3' ends" because mononucleotides are typically reacted to form oligonucleotides via attachment of the 5' phosphate or equivalent group of one nucleotide to the 3' hydroxyl or equivalent group of its neighboring nucleotide, optionally via a phosphodiester or other suitable linkage.

As used herein, the term "polymerase chain reaction" ("PCR") refers to the method of K. B. Mullis U.S. Pat. Nos. 4,683,195 and 4,683,202, , which describe a method for increasing the concentration of a segment of a polynucleotide of interest in a mixture of genomic DNA without cloning or purification. This process for amplifying the polynucleotide of interest consists of introducing a large excess of two oligonucleotide primers to the DNA mixture containing the desired polynucleotide of interest, followed by a precise sequence of thermal cycling in the presence of a DNA polymerase. The two primers are complementary to their respective strands of the double stranded polynucleotide of interest. To effect amplification, the mixture is denatured and the primers then annealed to their complementary sequences within the polynucleotide of interest molecule. Following annealing, the primers are extended with a polymerase to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired polynucleotide of interest. The length of the amplified segment of the desired polynucleotide of interest (amplicon) is determined by the relative positions of the primers with respect to each other, and therefore, this length is a controllable parameter. By virtue of repeating the process, the method is referred to as the "polymerase chain reaction" (hereinafter "PCR"). Because the desired amplified segments of the polynucleotide of interest become the predominant nucleic acid sequences (in terms of concentration) in the mixture, they are said to be "PCR amplified". As defined herein, target nucleic acid molecules within a sample including a plurality of target nucleic acid molecules are amplified via PCR. In a modification to the method discussed above, the target nucleic acid molecules can be PCR amplified using a plurality of different primer pairs, in some cases, one or more primer pairs per target nucleic acid molecule of interest, thereby forming a multiplex PCR reaction. Using multiplex PCR, it is possible to simultaneously amplify multiple nucleic acid molecules of interest from a sample to form amplified target sequences. It is also possible to detect the amplified target sequences by several different methodologies (e.g., quantitation with a bioanalyzer or qPCR, hybridization with a labeled probe; incorporation of biotinylated primers followed by avidin-enzyme conjugate detection; incorporation of ³²P-labeled deoxynucleotide triphosphates, such as dCTP or dATP, into the amplified target sequence). Any oligonucleotide sequence can be amplified with the appropriate set of primers, thereby allowing for the amplification of target nucleic acid molecules from genomic DNA, cDNA, formalin-fixed paraffin-embedded DNA, fine-needle biopsies and various other sources. In particular, the amplified target sequences created by the multiplex PCR process as disclosed herein, are themselves efficient substrates for subsequent PCR amplification or various downstream assays or manipulations.

As defined herein "multiplex amplification" refers to selective and non-random amplification of two or more target sequences within a sample using at least one target-specific primer. In some aspects, multiplex amplification is performed such that some or all of the target sequences are amplified within a single reaction vessel. The "plexy" or "plex" of a given multiplex amplification refers generally to the number of different target-specific sequences that are amplified during that single multiplex amplification. In some aspects, the plexy can be about 12-plex, 24-plex, 48-plex, 96-plex, 192-plex, 384-plex, 768-plex, 1536-plex, 3072-plex, 6144-plex or higher.

As used herein, the term "sensitivity" refers to a minimum amount (e.g., number of copies, mass, percent presence) of a template nucleic acid that can be detected by a given assay. As used herein, the term "specificity" refers to the ability of an assay to distinguish between amplification from a matched template versus a mismatched template. The term "selectivity" refers to the extent to which an assay can be used to determine minor sequences in mixtures without interference from majority or abundant sequences. Selectivity is often expressed as a ratio or percentage.

The disclosure adapts and utilizes techniques for preparing targeted multiplex nucleic acid libraries. Products and techniques for targeted multiplex library preparation have been disclosed and are commercially available. See, e.g., US Patent No., US9,957, 558B2 and US Patent Application Publication No. US2019/0002872A1. See also Ion AmpliSeq technology and Ion AmpliSeq HD technology, and ONCOMINE products, Thermo Fisher Scientific, Inc. Targeted multiplex nucleic acid library preparation has been used to isolate target sequences of interest. However, the procedures can be overwhelmed by the presence of abundant target sequences which can reduce ability to effectively detect or analyze rare or low frequency sequences in the sample. Methods in the art have utilized blocking oligonucleotides in amplification reactions to reduce abundant sequences, however, such methods require specialized and expensive reagents, and often require complex workflows for effective implementation.

We have found implementation of simple, add-in blocking oligos to established workflows can be utilized to effectively reduce or eliminate abundant sequences and allow for efficient production of rare or low frequency sequences of interest in target libraries. Provided methods demonstrated improved results over use of established blocking reagents used in current amplification suppression methods. Resulting library compositions are useful for follow on analysis of rare or low frequency alleles.

Provided herein are methods for preparing nucleic acid libraries and methods of analysis of rare sequences present in a biological sample comprising highly abundant wild type, control, and/or background sequences in a multiplex reaction. Typically, high wild type, control and/or background sequences of normal components of a sample can outweigh and mask effective analysis of rare target sequences of interest, rendering analysis difficult and ineffective. We have developed methods for multiplex preparation of library for follow on analytics that provide for effective identification and analysis of rare and/or low frequency sequences in a sample. Provided methods include streamlined, simple addition only steps incorporated into established library preparation procedures and analytics for efficient incorporation into laboratory procedures.

### Methods of Preparing Nucleic Acid Libraries

In one aspect, provided are first methods for preparing a library of one or more target sequences present in a sample at low frequency comprising amplifying within a single amplification reaction mixture a multiplex of different target sequences from a sample including a plurality of different target sequences, wherein the amplifying includes contacting at least a portion of the sample with a plurality of target-specific primers, and a polymerase under amplification conditions, thereby producing a multiplex of different amplified target sequences, and wherein (i) at least two of the plurality of target-specific primers include cleavable groups and at least one of the produced multiplex of different amplified target sequences include cleavable groups at both ends of resulting amplicon, and (ii) at least one of the plurality of target specific primers does not include the cleavable groups and at least one of the produced multiplex of different amplified target sequences includes at most a cleavable group at only one end of resulting amplicon. The methods further comprise cleaving the cleavable groups of the multiplex of different amplified target sequences and forming cleaved ends; and ligating at least two adapters to cleaved ends of at least one of the multiplex of different amplified target sequences, thereby producing one or more adapter-ligated amplified target sequences, to thereby prepare a library of different adapter-ligated target sequences, wherein amplicons resulting from blocking target specific primer are not capable of ligating adapter, and wherein none of the adapters in the ligation reaction hybridizes under high stringency conditions to any one of the multiplex of different amplified target sequences. In some aspects target nucleic acid sequences used in provided methods are selected from one or more of RNA, DNA, cDNA, or TNA. In certain aspects target nucleic acid sequences are selected from cfNA, ctNA, and tissue nucleic acid from cells.

Disclosed but not claimed one or more of the target nucleic acids of interest is a sequence variation. One or more sequence variations of interest are present in low concentration in a sample. In particular examples a sequence variation is any one or more of a deletion, insertion, translocation, or inversion of one or more nucleotides. In some aspects, one or more blocking target specific primers is directed to wild type sequence. In certain aspects one or more blocking target specific primers is directed to wild type or an allele specific sequence in order to reduce the presence of wild type or allele specific amplicons in the resulting library in order to detect a variant allele target the sequence of interest.

In some examples, one or more sequences of interest are present in low concentration in a sample. In certain examples one or more of the target nucleic acids is a pathogenic nucleic acid to the host sample. In particular examples one or more of the target nucleic acids is selected from viral, bacterial and microbe nucleic acid. In some examples, one or more blocking target specific primers is directed to an allele specific sequence native to the host sample. In certain examples one or more blocking target specific primers is directed to host sequence in order to reduce the presence of abundant host target nucleic acid amplicons in the resulting library in order to detect pathogen nucleic acid target sequence of interest.

In certain examples, the methods comprise use of primers containing a cleavable group wherein the cleavable group includes methylguanine, 8-oxo-guanine, xanthine, hypoxanthine, 5,6-dihydrouracil, uracil, 5-methylcytosine, thymine-dimer, 7-methylguanosine, 8-oxo-deoxyguanosine, xanthosine, inosine, dihydrouridine, bromodeoxyuridine, uridine or 5-methylcytidine. wherein each of the plurality of target-specific primers has any one or more of the following criteria: (1) includes two or more modified nucleotides within the primer sequence, at least one of which is included near or at the termini of the primer and at least one of which is included at, or about the center nucleotide position of the primer sequence; (2) length is about 15 to about 40 bases in length; (3) Tₘ is from about 60°C to about 70°C; (4) has low cross-reactivity with non-target sequences present in the sample; (5) at least the first four nucleotides (going from 3' to 5' direction) are non-complementary to any sequence within any other primer present in the reaction; and (6) are non-complementary to any consecutive stretch of at least 5 nucleotides within any other produced amplified target sequence.

In certain examples, provided methods comprising steps of amplifying, cleaving, ligating and preparing the library are carried out in a singles reaction vessel. In some aspects provided methods further comprise combining two or more libraries and/or pools of libraries each separately prepared into a single reaction, thereby preparing a library of different adapter-ligated target sequences.

In some aspects, an adapter includes a barcode, tag and/or universal priming sequence. In particular aspects, methods comprise use of no more than two different adapters.

In some examples an adapter is a double stranded adapter ligated to the end of at least one digested different target specific amplicon using a ligase capable of blunt-ended ligation. In certain examples a ligase is an isothermal ligase.

In provided first methods for preparing a library of one or more target sequences present in a sample at low frequency, the at least one of the plurality of target specific primers that does not include cleavable groups is a blocking primer wherein the at least one of the produced multiplex of different amplified target sequences includes at most a cleavable group at only one end of resulting amplicon is not processed through the remainder of the workflow and does not produce a productive adapter ligated amplicon. In some aspects methods comprise at least one blocking primer directed to a target sequence for suppression in library production, wherein the blocking primer comprises a DNA sequence complementary to the target sequence downstream (i.e., inside) of either a productive forward target specific primer or productive reverse target specific primer in the multiplex reaction. In some examples methods comprise at least one blocking primer directed to a target sequence that overlaps in part or in full with a productive forward target specific primer or productive reverse target specific primer in the multiplex for the target sequence. In some examples methods comprise at least one blocking primer directed to a target sequence that overlaps entirely with a productive forward target specific primer or a productive reverse target specific primer in the multiplex for the target sequence. In some examples methods comprise at least one blocking primer directed to a target sequence that does not overlap in part or in full with a productive forward target specific primer or a productive reverse target specific primer in the multiplex for the target sequence. In some examples the blocking primer is complementary to a wild type sequence that includes loci corresponding to at least one variant position of interest such that the blocking primer suppresses production of wild type sequence in the library.

In some aspects methods comprise at least one pair of forward and reverse blocking primers directed to a target sequence, wherein each of the blocking primers overlap in part or in full with a productive forward target specific primer and in part or in full with a productive reverse target specific primer for the target sequence. In certain aspects methods comprise at least one pair of forward and reverse blocking primers directed to a target sequence, wherein each of the forward and reverse blocking primers overlap substantially with a productive forward target specific primer and substantially with a productive reverse target specific primer for the target sequence. In certain aspects methods comprise at least one pair of forward and reverse blocking primers directed to a target sequence, wherein each of the forward and reverse blocking primers overlap entirely with a productive forward target specific primer and entirely with a productive reverse target specific primer for the target sequence. In certain aspects methods comprise at least one pair of forward and reverse blocking primers directed to a target sequence, wherein neither of the forward or reverse blocking primers overlap with a productive forward target specific primer or with a productive reverse target specific primer for the target sequence.

In some examples, blocking primers comprise one or more of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence. In certain aspects, blocking primers comprise each of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence.

In some examples, blocking primer(s) are included in methods of the invention in concentration similar to forward and reverse productive primers in the reaction. In some examples, blocking primer(s) are included at concentration less than forward and reverse productive primers in the reaction. In some examples, blocking primer(s) are included at least 0.5 fold of forward and reverse productive primers in the reaction. In some examples, blocking primer(s) are included at concentration equal to forward and reverse productive primers in the reaction. In some examples, blocking primer(s) are included in methods of the invention in concentration higher than forward and reverse productive primers in the reaction. In some examples, blocking primer(s) are included at least 1.5 fold higher than forward and reverse productive primers in the reaction. In certain examples, blocking primer(s) are included at least 2 fold higher than forward and reverse productive primers in the reaction. In certain examples, blocking primer(s) are included at least 4 fold higher than forward and reverse productive primers in the reaction. In certain aspects, blocking primer(s) are included at least 8 fold or higher than forward and reverse productive primers in the reaction.

In another aspect, provided are second methods for preparing a library of one or more target sequences present in a sample at low frequency comprising amplifying within a single amplification reaction mixture a multiplex of different target sequences from a sample including a plurality of different target sequences, wherein the amplifying includes contacting at least a portion of the sample with a plurality of target-specific primers, and a polymerase under amplification conditions, thereby producing a multiplex of different amplified target sequences, and wherein (i) at least two of the plurality of adapters comprise a universal handle sequence and a target nucleic acid sequence and a cleavable moiety and optionally one or more tag sequences wherein the target nucleic acid sequence of the adapter includes at least one cleavable moiety and the universal handle sequence does not include the cleavable moiety and at least one of the produced multiplex of different amplified target sequences include cleavable groups at both ends of resulting amplicon, and (ii) at least one of the plurality of target specific primers does not include the cleavable moiety or universal handle sequence and at least one of the produced multiplex of different amplified target sequences includes a cleavable group and universal handle sequence at no more than one end of resulting amplicon. The methods further comprise digesting the cleavable groups of the multiplex of different amplified target sequences to reduce or eliminate primer dimers and form partially digested target amplicons; repairing the partially digested amplicons of at least one of the multiplex of different amplified target sequences; and amplifying repaired target amplicons with two universal adapters corresponding to the universal handle sequences, thereby producing one or more adapter amplified target sequences, to thereby prepare a library of different adapter amplified target sequences, wherein amplicons resulting from blocking target specific primer are not compatible with amplification using universal adapter, and wherein none of the adapters in the amplification reaction hybridizes under high stringency conditions to any one of the multiplex of different amplified target sequences. In some aspects target nucleic acid sequences used in provided methods are selected from one or more of RNA, DNA, cDNA, or TNA. In certain aspects target nucleic acid sequences are selected from cfNA, ctNA, and tissue nucleic acid from cells.

In some aspects target nucleic acid sequences used in provided methods are selected from one or more of RNA, DNA, cDNA, or TNA. In certain aspects target nucleic acid sequences are selected from cfNA, ctNA, and tissue nucleic acid from cells.

Disclosed but not claimed are one or more of the target nucleic acids of interest is a sequence variation. One or more sequence variations of interest are present in low concentration in a sample. In particular examples a sequence variation is any one or more of a deletion, insertion, translocation, or inversion of one or more nucleotides. In some examples, one or more blocking target specific primers is directed to wild type sequence. In certain examples one or more blocking target specific primers is directed to wild type or an allele specific sequence in order to reduce the presence of wild type or allele specific amplicons in the resulting library in order to detect a variant allele target the sequence of interest.

In some examples, one or more sequences of interest are present in low concentration in a sample. In certain examples one or more of the target nucleic acids is a pathogenic nucleic acid to the host sample. In particular examples one or more of the target nucleic acids is selected from viral, bacterial and microbe nucleic acid. In some examples, one or more blocking target specific primers is directed to an allele specific sequence native to the host sample. In certain examples one or more blocking target specific primers is directed to host sequence in order to reduce the presence of abundant host target nucleic acid amplicons in the resulting library in order to detect pathogen nucleic acid target sequence of interest.

In certain examples, the methods comprise use of primers containing a cleavable group wherein the cleavable group includes methylguanine, 8-oxo-guanine, xanthine, hypoxanthine, 5,6-dihydrouracil, uracil, 5-methylcytosine, thymine-dimer, 7-methylguanosine, 8-oxo-deoxyguanosine, xanthosine, inosine, dihydrouridine, bromodeoxyuridine, uridine or 5-methylcytidine. wherein each of the plurality of target-specific primers has any one or more of the following criteria: (1) includes two or more modified nucleotides within the primer sequence, at least one of which is included near or at the termini of the primer and at least one of which is included at, or about the center nucleotide position of the primer sequence; (2) length is about 15 to about 40 bases in length; (3) Tₘ is from about 60°C to about 70°C; (4) has low cross-reactivity with non-target sequences present in the sample; (5) at least the first four nucleotides (going from 3' to 5' direction) are non-complementary to any sequence within any other primer present in the reaction; and (6) are non-complementary to any consecutive stretch of at least 5 nucleotides within any other produced amplified target sequence.

In certain examples, provided methods comprising steps of amplifying, cleaving, repairing and preparing the library are carried out in a singles reaction vessel. In some examples provided methods further comprise combining two or more libraries and/or pools of libraries each separately prepared into a single reaction, thereby preparing a library of different adapter-ligated target sequences.

In some examples, an adapter includes a barcode, tag and/or universal priming sequence. In some examples a second amplification is carried out using universal amplification adapters comprising universal sequence complementary to the universal priming sequences. In some examples, universal adapters further comprise sequences for downstream analysis of target sequences of interest. In particular examples methods comprise use of no more than four different universal adapters, comprised of combinations of two different universal sequences and two different adapter sequences. In particular examples, methods comprise use of no more than two different universal adapters.

In provided second methods for preparing a library of one or more target sequences present in a sample at low frequency, the at least one of the plurality of target specific primers that does not include cleavable groups is a blocking primer wherein the at least one of the produced multiplex of different amplified target sequences includes at most a cleavable group at only one end of resulting amplicon is not processed through the remainder of the workflow and does not produce a productive adapter amplified target amplicon. In some examples methods comprise at least one blocking primer directed to a target sequence for suppression in library production, wherein the blocking primer comprises a DNA sequence complementary to the target sequence downstream (i.e., inside) of either a productive forward target specific primer or productive reverse target specific primer in the multiplex reaction. In some examples methods comprise at least one blocking primer directed to a target sequence that overlaps in part or in full with a productive forward target specific primer or productive reverse target specific primer in the multiplex for the target sequence. In some examples methods comprise at least one blocking primer directed to a target sequence that overlaps entirely with a productive forward target specific primer or a productive reverse target specific primer in the multiplex for the target sequence. In some examples methods comprise at least one blocking primer directed to a target sequence that does not overlap in part or in full with a productive forward target specific primer or a productive reverse target specific primer in the multiplex for the target sequence. In some examples the blocking primer is complementary to a wild type sequence that includes loci corresponding to at least one variant position of interest such that the blocking primer suppresses production of wild type sequence in the library.

In some aspects methods comprise at least one pair of forward and reverse blocking primers directed to a target sequence, wherein each of the blocking primers overlap in part or in full with a productive forward target specific primer and in part or in full with a productive reverse target specific primer for the target sequence. In certain aspects methods comprise at least one pair of forward and reverse blocking primers directed to a target sequence, wherein each of the forward and reverse blocking primers overlap substantially with a productive forward target specific primer and substantially with a productive reverse target specific primer for the target sequence. In certain aspects methods comprise at least one pair of forward and reverse blocking primers directed to a target sequence, wherein each of the forward and reverse blocking primers overlap entirely with a productive forward target specific primer and entirely with a productive reverse target specific primer for the target sequence. In certain aspects methods comprise at least one pair of forward and reverse blocking primers directed to a target sequence, wherein neither of the forward or reverse blocking primers overlap with a productive forward target specific primer or with a productive reverse target specific primer for the target sequence.

In some aspects, blocking primers comprise one or more of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence. In certain aspects, blocking primers comprise each of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence.

In some aspects, blocking primer(s) are included in methods of the invention in concentration similar to forward and reverse productive primers in the reaction. In some aspects, blocking primer(s) are included at concentration less than forward and reverse productive primers in the reaction. In some aspects, blocking primer(s) are included at least 0.5 fold of forward and reverse productive primers in the reaction. In some aspects, blocking primer(s) are included at concentration equal to forward and reverse productive primers in the reaction. In some aspects, blocking primer(s) are included in methods of the invention in concentration higher than forward and reverse productive primers in the reaction. In some aspects, blocking primer(s) are included at least 1.5 fold higher than forward and reverse productive primers in the reaction. In certain aspects, blocking primer(s) are included at least 2 fold higher than forward and reverse productive primers in the reaction. In certain aspects, blocking primer(s) are included at least 4 fold higher than forward and reverse productive primers in the reaction. In certain aspects, blocking primer(s) are included at least 8 fold or higher than forward and reverse productive primers in the reaction.

Methods of Detection of Low Frequency Target Polynucleotides in a Multiplex Reaction.

In another aspect, provided are methods of detecting a target polynucleotide of interest from a sample, wherein the target polynucleotide is present in the sample at a low frequency, the method comprising: preparing a library of one or more target sequences present in a sample according to the first methods for preparing a library of one or more target sequences present in a sample at low frequency provided herein; and analysis of the prepared library amplicons to determine the target sequences present in the sample, thereby detecting target polynucleotide of interest

In some aspects target nucleic acid sequences used in provided methods of detection are selected from one or more of RNA, DNA, cDNA, or TNA. In certain aspects target nucleic acid sequences are selected from cfNA, ctNA, and tissue nucleic acid from cells.

Disclosed but not claimed are one or more of the target nucleic acids of interest is a sequence variation. One or more sequence variations of interest are present in low concentration in a sample. In particular aspects a sequence variation is any one or more of a deletion, insertion, translocation, or inversion of one or more nucleotides. In some aspects, one or more blocking target specific primers is directed to wild type sequence. In certain aspects one or more blocking target specific primers is directed to wild type or an allele specific sequence in order to reduce the presence of wild type or allele specific amplicons in the resulting library in order to detect a variant allele target the sequence of interest.

In some aspects, one or more sequences of interest are present in low concentration in a sample. In certain aspects one or more of the target nucleic acids is a pathogenic nucleic acid to the host sample. In particular aspects one or more of the target nucleic acids is selected from viral, bacterial and microbe nucleic acid. In some aspects, one or more blocking target specific primers is directed to an allele specific sequence native to the host sample. In certain aspects one or more blocking target specific primers is directed to host sequence in order to reduce the presence of abundant host target nucleic acid amplicons in the resulting library in order to detect pathogen nucleic acid target sequence of interest.

In certain aspects, the methods comprise use of primers containing a cleavable group wherein the cleavable group includes methylguanine, 8-oxo-guanine, xanthine, hypoxanthine, 5,6-dihydrouracil, uracil, 5-methylcytosine, thymine-dimer, 7-methylguanosine, 8-oxo-deoxyguanosine, xanthosine, inosine, dihydrouridine, bromodeoxyuridine, uridine or 5-methylcytidine. wherein each of the plurality of target-specific primers has any one or more of the following criteria: (1) includes two or more modified nucleotides within the primer sequence, at least one of which is included near or at the termini of the primer and at least one of which is included at, or about the center nucleotide position of the primer sequence; (2) length is about 15 to about 40 bases in length; (3) Tₘ is from about 60°C to about 70°C; (4) has low cross-reactivity with non-target sequences present in the sample; (5) at least the first four nucleotides (going from 3' to 5' direction) are non-complementary to any sequence within any other primer present in the reaction; and (6) are non-complementary to any consecutive stretch of at least 5 nucleotides within any other produced amplified target sequence.

In certain aspects, provided methods comprise steps of amplifying, cleaving, ligating and preparing the library carried out in a singles reaction vessel. In some aspects provided methods further comprise combining two or more libraries and/or pools of libraries each separately prepared into a single reaction, thereby preparing a library of different adapter-ligated target sequences.

In some aspects, an adapter includes a barcode, tag and/or universal priming sequence. In particular embodiments, methods comprise use of no more than two different adapters.

In some aspects an adapter a double stranded adapter ligated to the end of at least one digested different target specific amplicon using a ligase capable of blunt-ended ligation. In certain aspects a ligase is an isothermal ligase.

In provided first methods for preparing a library of one or more target sequences present in a sample at low frequency, the at least one of the plurality of target specific primers that does not include cleavable groups is a blocking primer wherein the at least one of the produced multiplex of different amplified target sequences includes at most a cleavable group at only one end of resulting amplicon is not processed through the remainder of the workflow and does not produce a productive adapter ligated amplicon. In some examples methods comprise at least one blocking primer directed to a target sequence for suppression in library production, wherein the blocking primer comprises a DNA sequence complementary to the target sequence downstream (i.e., inside) of either a productive forward target specific primer or productive reverse target specific primer in the multiplex reaction. In some examples methods comprise at least one blocking primer directed to a target sequence that overlaps in part or in full with a productive forward target specific primer or productive reverse target specific primer in the multiplex for the target sequence. In some examples methods comprise at least one blocking primer directed to a target sequence that overlaps entirely with a productive forward target specific primer or a productive reverse target specific primer in the multiplex for the target sequence. In some examples methods comprise at least one blocking primer directed to a target sequence that does not overlap in part or in full with a productive forward target specific primer or a productive reverse target specific primer in the multiplex for the target sequence. In some examples the blocking primer is complementary to a wild type sequence that includes loci corresponding to at least one variant position of interest such that the blocking primer suppresses production of wild type sequence in the library.

In some examples methods comprise at least one pair of forward and reverse blocking primers directed to a target sequence, wherein each of the blocking primers overlap in part or in full with a productive forward target specific primer and in part or in full with a productive reverse target specific primer for the target sequence. In certain examples methods comprise at least one pair of forward and reverse blocking primers directed to a target sequence, wherein each of the forward and reverse blocking primers overlap substantially with a productive forward target specific primer and substantially with a productive reverse target specific primer for the target sequence. In certain examples methods comprise at least one pair of forward and reverse blocking primers directed to a target sequence, wherein each of the forward and reverse blocking primers overlap entirely with a productive forward target specific primer and entirely with a productive reverse target specific primer for the target sequence. In certain examples methods comprise at least one pair of forward and reverse blocking primers directed to a target sequence, wherein neither of the forward or reverse blocking primers overlap with a productive forward target specific primer or with a productive reverse target specific primer for the target sequence.

In some examples, blocking primers comprise one or more of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence. In certain examples, blocking primers comprise each of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence.

In some examples, blocking primer(s) are included in methods of the invention in concentration similar to forward and reverse productive primers in the reaction. In some examples, blocking primer(s) are included at concentration less than forward and reverse productive primers in the reaction. In some examples, blocking primer(s) are included at least 0.5 fold of forward and reverse productive primers in the reaction. In some examples, blocking primer(s) are included at concentration equal to forward and reverse productive primers in the reaction. In some examples, blocking primer(s) are included in methods of the invention in concentration higher than forward and reverse productive primers in the reaction. In some examples, blocking primer(s) are included at least 1.5 fold higher than forward and reverse productive primers in the reaction. In certain examples, blocking primer(s) are included at least 2 fold higher than forward and reverse productive primers in the reaction. In certain examples, blocking primer(s) are included at least 4 fold higher than forward and reverse productive primers in the reaction. In certain examples, blocking primer(s) are included at least 8 fold or higher than forward and reverse productive primers in the reaction.

Additionally provided but not claimed herein are methods that further comprises clonal amplification of a portion of the at least one adapter-ligated target-specific sequence. The clonal amplification of a portion of the at least one adapter-ligated target-specific amplicon includes emulsion PCR, bridge PCR or isothermal amplification.

In some examples, provided methods comprise analysis performed by sequencing the library. In certain examples sequencing includes sequencing carried out by sequencing by synthesis. In particular examples, provided methods comprise library preparation and/or sequencing are carried out on an automated system. Disclosed but not claimed methods includes clonal amplification of a portion of the at least one adapter-ligated target-specific sequence prior to sequencing. The clonal amplification of a portion of the at least one adapter-ligated target-specific amplicon includes emulsion PCR, bridge PCR or isothermal amplification. In certain examples provided methods further include normalization of prepared library prior to clonal amplification. In particular examples two or more prepared libraries are combined prior to clonal amplification.

In another aspect, provided are methods of detecting a target polynucleotide of interest from a sample, wherein the target polynucleotide is present in the sample at a low frequency, the method comprising: preparing a library of one or more target sequences present in a sample according to the second methods for preparing a library of one or more target sequences present in a sample at low frequency provided herein; and analysis of the prepared library amplicons to determine the target sequences present in the sample, thereby detecting target polynucleotide of interest.

In some examples target nucleic acid sequences used in provided methods are selected from one or more of RNA, DNA, cDNA, or TNA. In certain examples target nucleic acid sequences are selected from cfNA, ctNA, and tissue nucleic acid from cells.

Disclosed but not claimed are one or more of the target nucleic acids of interest is a sequence variation. One or more sequence variations of interest are present in low concentration in a sample. In particular examples a sequence variation is any one or more of a deletion, insertion, translocation, or inversion of one or more nucleotides. In some examples, one or more blocking target specific primers is directed to wild type sequence. In certain examples one or more blocking target specific primers is directed to wild type or an allele specific sequence in order to reduce the presence of wild type or allele specific amplicons in the resulting library in order to detect a variant allele target the sequence of interest.

In some examples, one or more sequences of interest are present in low concentration in a sample. In certain embodiments one or more of the target nucleic acids is a pathogenic nucleic acid to the host sample. In particular examples one or more of the target nucleic acids is selected from viral, bacterial and microbe nucleic acid. In some examples, one or more blocking target specific primers is directed to an allele specific sequence native to the host sample. In certain examples one or more blocking target specific primers is directed to host sequence in order to reduce the presence of abundant host target nucleic acid amplicons in the resulting library in order to detect pathogen nucleic acid target sequence of interest.

In certain examples, the methods comprise use of primers containing a cleavable group wherein the cleavable group includes methylguanine, 8-oxo-guanine, xanthine, hypoxanthine, 5,6-dihydrouracil, uracil, 5-methylcytosine, thymine-dimer, 7-methylguanosine, 8-oxo-deoxyguanosine, xanthosine, inosine, dihydrouridine, bromodeoxyuridine, uridine or 5-methylcytidine. wherein each of the plurality of target-specific primers has any one or more of the following criteria: (1) includes two or more modified nucleotides within the primer sequence, at least one of which is included near or at the termini of the primer and at least one of which is included at, or about the center nucleotide position of the primer sequence; (2) length is about 15 to about 40 bases in length; (3) Tₘ is from about 60°C to about 70°C; (4) has low cross-reactivity with non-target sequences present in the sample; (5) at least the first four nucleotides (going from 3' to 5' direction) are non-complementary to any sequence within any other primer present in the reaction; and (6) are non-complementary to any consecutive stretch of at least 5 nucleotides within any other produced amplified target sequence.

In certain examples, provided methods comprising steps of amplifying, cleaving, repairing and preparing the library are carried out in a single reaction vessel. In some aspects provided methods further comprise combining two or more libraries and/or pools of libraries each separately prepared into a single reaction, thereby preparing a library of different adapter-ligated target sequences.

In some aspects, an adapter includes a barcode, tag and/or universal priming sequence. In some aspects a second amplification is carried out using universal amplification adapters comprising universal sequence complementary to the universal priming sequences. In some aspects, universal adapters further comprise sequences for downstream analysis of target sequences of interest. In particular aspects methods comprise use of no more than four different universal adapters, comprised of combinations of two different universal sequences and two different adapter sequences. In particular aspects, methods comprise use of no more than two different universal adapters.

In provided second methods for preparing a library of one or more target sequences present in a sample at low frequency, the at least one of the plurality of target specific primers that does not include cleavable groups is a blocking primer wherein the at least one of the produced multiplex of different amplified target sequences includes at most a cleavable group at only one end of resulting amplicon is not processed through the remainder of the workflow and does not produce a productive adapter amplified target amplicon. In some aspects methods comprise at least one blocking primer directed to a target sequence for suppression in library production, wherein the blocking primer comprises a DNA sequence complementary to the target sequence downstream (i.e., inside) of either a productive forward target specific primer or productive reverse target specific primer in the multiplex reaction. In some aspects methods comprise at least one blocking primer directed to a target sequence that overlaps in part or in full with a productive forward target specific primer or productive reverse target specific primer in the multiplex for the target sequence. In some aspects methods comprise at least one blocking primer directed to a target sequence that overlaps entirely with a productive forward target specific primer or a productive reverse target specific primer in the multiplex for the target sequence. In some aspects methods comprise at least one blocking primer directed to a target sequence that does not overlap in part or in full with a productive forward target specific primer or a productive reverse target specific primer in the multiplex for the target sequence. In some aspects the blocking primer is complementary to a wild type sequence that includes loci corresponding to at least one variant position of interest such that the blocking primer suppresses production of wild type sequence in the library.

In some aspects methods comprise at least one pair of forward and reverse blocking primers directed to a target sequence, wherein each of the blocking primers overlap in part or in full with a productive forward target specific primer and in part or in full with a productive reverse target specific primer for the target sequence. In certain aspects methods comprise at least one pair of forward and reverse blocking primers directed to a target sequence, wherein each of the forward and reverse blocking primers overlap substantially with a productive forward target specific primer and substantially with a productive reverse target specific primer for the target sequence. In certain examples methods comprise at least one pair of forward and reverse blocking primers directed to a target sequence, wherein each of the forward and reverse blocking primers overlap entirely with a productive forward target specific primer and entirely with a productive reverse target specific primer for the target sequence. In certain aspects methods comprise at least one pair of forward and reverse blocking primers directed to a target sequence, wherein neither of the forward or reverse blocking primers overlap with a productive forward target specific primer or with a productive reverse target specific primer for the target sequence.

In some examples, blocking primers comprise one or more of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence. In certain examples, blocking primers comprise each of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence.

In some examples, blocking primer(s) are included in methods of the invention in concentration similar to forward and reverse productive primers in the reaction. In some examples, blocking primer(s) are included at concentration less than forward and reverse productive primers in the reaction. In some examples, blocking primer(s) are included at least 0.5 fold of forward and reverse productive primers in the reaction. In some examples, blocking primer(s) are included at concentration equal to forward and reverse productive primers in the reaction. In some examples, blocking primer(s) are included in methods of the invention in concentration higher than forward and reverse productive primers in the reaction. In some examples, blocking primer(s) are included at least 1.5 fold higher than forward and reverse productive primers in the reaction. In certain examples, blocking primer(s) are included at least 2 fold higher than forward and reverse productive primers in the reaction. In certain examples, blocking primer(s) are included at least 4 fold higher than forward and reverse productive primers in the reaction. In certain examples, blocking primer(s) are included at least 8 fold or higher than forward and reverse productive primers in the reaction.

Disclosed but not claimed methods further comprises clonal amplification of a portion of the at least one adapter-amplified target-specific sequence. Clonal amplification of a portion of the at least one adapter-ligated target-specific amplicon includes emulsion PCR, bridge PCR or isothermal amplification.

In some examples, provided methods comprise analysis performed by sequencing the library. In certain aspects sequencing includes sequencing carried out by sequencing by synthesis. In particular aspects, provided methods comprise library preparation and/or sequencing are carried out on an automated system. Additionally provided but not claimed methods includes clonal amplification of a portion of the at least one adapter-amplified target-specific sequence prior to sequencing. The clonal amplification of a portion of the at least one adapter-amplified target-specific amplicon includes emulsion PCR, bridge PCR or isothermal amplification. Provided but not claimed methods further include normalization of prepared library prior to clonal amplification. Two or more prepared libraries are combined prior to clonal amplification.

In some aspects, the library is prepared in less than 4 hours. In some aspects, the library is prepared, enriched and sequenced in less than 3 hours. In some aspects, the library is prepared, enriched and sequenced in 2 to 3 hours. In some aspects, the library is prepared in approximately 2.5 hours. In some aspects, the library is prepared in approximately 2.75 hours. In some aspects, the library is prepared in approximately 3 hours.

### Compositions

Additional aspects of the invention comprise compositions comprising a plurality of nucleic acid primers or adapters and blocking oligos, as well as library compositions prepared according to the methods of the invention. Disclosed but not claimed are library compositions prepared according to the first methods of preparing a library of the invention, as well as library compositions prepared according to the second methods of preparing a library of the invention are provided herein. Prepared library compositions provide optimized compositions for detection and/or analysis of low frequency sequences of interest. Provided compositions are useful in conjunction with the methods described herein as well as for additional analysis and applications known in the art.

Thus provided are composition comprising at least one blocking oligo for suppression of abundant target sequences in the library. Compositions comprise at least one target specific blocking oligo and up to one hundred thousand target specific blocking oligo pairs are included in provided compositions. Provided compositions allow for rapid simple production of highly multiplexed targeted libraries, while suppressing abundant target sequences to allow for effective detection and analysis of low frequency target sequences in a sample.

At least one of a plurality of target specific primers in a composition does not include cleavable groups is a blocking primer such that at least one of the produced multiplex of different amplified target sequences includes at most a cleavable group at only one end of resulting amplicon and therefore is not processed through the remainder of the library preparation workflow and therefore does not produce a productive adapter ligated or adapter amplified target amplicon. In some examples compositions comprise at least one blocking primer directed to a target sequence for suppression in library production, wherein the blocking primer comprises a DNA sequence complementary to the target sequence downstream (i.e., inside) of either a productive forward target specific primer or adapter or a productive reverse target specific primer or adapter in the multiplex reaction composition. In some examples compositions comprise at least one blocking primer directed to a target sequence that overlaps in part or in full with a productive forward target specific primer or adapter or a productive reverse target specific primer or adapter in the multiplex for the target sequence. In some examples compositions comprise at least one blocking primer directed to a target sequence that overlaps entirely with a productive forward target specific primer or adapter or a productive reverse target specific primer or adapter in the multiplex composition for the target sequence. In some examples compositions comprise at least one blocking primer directed to a target sequence that does not overlap in part or in full with a productive forward target specific primer or adapter or a productive reverse target specific primer or adapter in the multiplex composition for the target sequence. In some examples the blocking primer is complementary to a wild type sequence that includes loci corresponding to at least one variant position of interest such that the blocking primer suppresses production of wild type sequence in the library.

In some examples compositions comprise at least one pair of forward and reverse blocking primers directed to a target sequence, wherein each of the blocking primers overlap in part or in full with a productive forward target specific primer or adapter and in part or in full with a productive reverse target specific primer or adapter at the target sequence. In certain examples compositions comprise at least one pair of forward and reverse blocking primers directed to a target sequence, wherein each of the forward and reverse blocking primers overlap substantially with a productive forward target specific primer or adapter and substantially with a productive reverse target specific primer or adapter at the target sequence. In certain examples compositions comprise at least one pair of forward and reverse blocking primers directed to a target sequence, wherein each of the forward and reverse blocking primers overlap entirely with a productive forward target specific primer or adapter and entirely with a productive reverse target specific primer or adapter at the target sequence. In certain examples compositions comprise at least one pair of forward and reverse blocking primers directed to a target sequence, wherein neither of the forward or reverse blocking primers overlap with a productive forward target specific primer or adapter or with a productive reverse target specific primer or adapter at the target sequence.

In some examples, blocking primers comprise one or more of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence. In certain examples, blocking primers comprise each of the following criteria: (1) length is about 15 to about 40 bases in length; (2) Tₘ is from about 60°C to about 70°C; (3) has low cross-reactivity with non-target sequences present in the sample; (4) at least the first four nucleotides (going from 3' to 5' direction) are non-complementary to any sequence within any other primer present in the reaction; and (5) are non-complementary to any consecutive stretch of at least 5 nucleotides within any other produced amplified target sequence.

In some examples, blocking primer(s) are included in compositions of the invention in concentration similar to forward and reverse productive primers or adapters in the reaction. In some examples, blocking primer(s) are included at concentration less than forward and reverse productive primers or adapters in the reaction. In some examples, blocking primer(s) are included at least 0.5 fold of forward and reverse productive primers or adapters in the reaction. In some examples, blocking primer(s) are included at concentration equal to forward and reverse productive primers or adapters in the reaction. In some examples, blocking primer(s) are included in compositions of the invention in concentration higher than forward and reverse productive primers or adapters in the reaction. In some aspects, blocking primer(s) are included at least 1.5 fold higher than forward and reverse productive primers or adapters in the reaction. In certain examples, blocking primer(s) are included at least 2 fold higher than forward and reverse productive primers or adapters in the reaction. In certain examples, blocking primer(s) are included at least 4 fold higher than forward and reverse productive primers or adapters in the reaction. In certain examples, blocking primer(s) are included at least 8 fold or at least 10 fold or higher than forward and reverse productive primers or adapters in the reaction.

Blocking primers provided for use in the compositions and methods herein containing no cleavable group that is processed according to the library preparation protocol can be designed against any preferred sequence to reduce or eliminate the competing target sequence from being produced in a library preparation. In some aspects, a blocking primer is designed against wild type sequences downstream or inside standard cleavable primers of a target amplicon in order to reduce or eliminate a wild type sequence produced in a library and thereby increasing availability of detection and/or analysis of a target mutation present within the regions encompassed by the blocking primer. Blocking primer will amplify wild type sequence, however, because of lack of cleaving site or universal sequence, resulting amplicon will not be processed or incorporated into the resulting productive amplicon library for analysis. Since the blocking primer efficiently amplifies wild type sequence and not mismatch variants, while blocking availability for standard cleavable primers, low frequency variant sequences are available for efficient amplification and processing by standard cleavable primers and fully processed in the library preparation workflow.

Blocking primer pairs provided for use in the compositions and methods herein containing no cleavable group that is processed according to the library preparation protocol can be designed against any preferred target sequence to reduce or eliminate the target sequence from being produced in a library preparation. In some aspects, a blocking primer pair is designed against identical sequences to the target specific standard cleavable primers or adapters directed to a target amplicon in order to reduce or eliminate the sequence produced in a library and thereby increasing availability of detection and/or analysis of a other sequences present in the sample at lower frequency. Blocking primer pair will amplify the target sequence and compete with the standard library preparation target specific primers or adapters, however, because of lack of cleaving site or universal sequence, resulting amplicon will not be processed or incorporated into the resulting productive amplicon library for analysis. Since the blocking primer pair competes directly with the standard target specific primers or adapters in the reaction, decreased representation of the target amplicon will result in the final library preparation. In some aspects, compositions comprise blocking primer pair present at higher concentrations than the standard target specific primer or adapter, the resulting productive amplicon can be further reduced or eliminated. Because low frequency variant sequences are available for efficient amplification and processing by standard cleavable primers and fully processed in the library preparation workflow resulting analysis allows for additional sensitivity for detection of low frequency or rare sequences present in the sample.

Provided blocking primer and primer pair compositions are compatible with standard library preparation technology workflows and do not require modified protocols. Thus, blocking primer and primer pair compositions are compatible with drop in capability to existing library preparation panel workflows. In certain aspects a composition comprising one or more blocking primer is added to or spiked into an existing library preparation panel for methods of library preparation. In some aspects a composition comprising one or more blocking primer pairs is added to or spiked into an existing library preparation panel for methods of library preparation.

Provided blocking primer and primer pair compositions do not require complex design requirements or modifications and thus are simpler and less expensive than other methods. Oligos comprising native unmodified sequences directed to a target of interest for suppression can be easily prepared by methods well known in the art for addition to existing panels and workflows. Alternatively, where a custom library preparation panel is developed, blocking primers and primer pairs can optionally be designed concurrently and synthesized to be included in the prepared custom panel or to be used in conjunction with the custom synthesized panel. We have found that use of standard unmodified blocking primers and/or primer pairs in conjunction with standard target specific multiplex library preparation using cleavable sites results in significant reduction or elimination of target abundant sequences, thereby allowing for significant enrichment of analysis of target low frequency sequences of interest. The provided invention thus provides a surprisingly simple yet elegant method for suppression of specific amplicons to address a complex problem in the art.

Blocking primers or primer pairs may be designed to any competing target sequence of interest for reduction or elimination in a library preparation. There may be instances where a normal (or possibly elevated) representation of specific target amplicons is undesirable or deleterious to the purpose of the sequence analysis project. Designs and concentrations of blocking primers may be produced to achieve the desired reduction or elimination of a target amplicon. For example, a wild type sequence may compete with a low frequency mutant sequence for production in a library. Provided methods directed to reduce the presence of the wild type sequence amplicon in the library will increase availability and analysis of the mutant sequence. Similarly, an abundant human control sequence may overwhelm the presence of a rare or low frequency sequence of an infectious agent in the sample. Provided methods directed to reduce the presence of the human control sequence amplicon in the productive library will increase availability and analysis of the low frequency infectious agent sequence in the sample.

In some aspects, provided compositions comprise plurality of nucleic acid adapters, wherein each of the plurality of adapters comprise a 5' universal handle sequence, optionally one or more tag sequences, and a 3' target nucleic acid sequence wherein each adapter comprises a cleavable moiety; wherein the target nucleic acid sequence of the adapter includes at least one cleavable moiety, cleavable moieties are included flanking either end of the tag sequence and the universal handle sequence does not include the cleavable moiety. At least two and up to one hundred thousand target specific adapter pairs are included in provided compositions. Provided composition allow for rapid production of highly multiplexed, optionally tagged, targeted libraries.

Primer/adapter compositions may be single stranded or double stranded. In some aspects adapter compositions comprise are single stranded adapters. In some aspects adapter compositions comprise double stranded adapters. In some aspects adapter compositions comprise a mixture of single stranded and double stranded adapters.

In some aspects, compositions include a plurality of target specific primers or adapters capable of amplification of one or more target nucleic acid sequences comprising a multiplex of adapter pairs capable of amplification of at least two different target nucleic acid sequences wherein the target-specific primer sequence is substantially non-complementary to other target specific primer sequences in the composition. In some aspects, the composition comprises at least 25, 50, 75, 100, 150, 200, 250, 300, 350, 400, 450, 500, 750, 1000, 1250, 1500, 1750, 2000, 2250, 2500, 2750, 3000, 3250, 3500, 3750, 4000, 4500, 5000, 5500, 6000, 7000, 8000, 9000, 10000, 11000, or 12000, or more target-specific adapter pairs. In some aspects, target-specific adapter pairs comprise about 15 nucleotides to about 40 nucleotides in length, wherein at least one nucleotide is replaced with a cleavable group. In some aspects the cleavable group is a uridine nucleotide. In some aspects, the target-specific primer or adapter pairs are designed to amplify an exon, gene, exome or region of the genome associated with a clinical or pathological condition, e.g., amplification of one or more sites comprising one or more mutations (e.g., driver mutation) associated with a cancer, e.g., lung, colon, breast cancer, etc., mutations associated with an inherited disease, e.g., cystic fibrosis, muscular dystrophies, etc or sequences associated with an infectious agent (e.g., bacteria, virus, or fungus). In some aspects, the target-specific primer or adapter pairs when hybridized to a target sequence and amplified as provided herein generates a library of adapter-ligated amplified target sequences that are about 100 to about 600 base pairs in length. In some aspects, no one adapter-ligated amplified target sequence or adapter amplified target sequence is amplified in the library by more than 30% as compared to the remainder of other adapter-ligated amplified target sequences or adapter amplified target sequences in the library.

In some aspects, the target-specific primer sequences of primer pairs or adapter pairs in the compositions of the invention are target-specific sequences that can amplify specific regions of a nucleic acid molecule. In some aspects, the target-specific primers or adapters can amplify genomic DNA or cDNA. In some aspects, target-specific primers or adapters can amplify mammalian nucleic acid, such as, but not limited to human DNA or RNA, murine DNA or RNA, bovine DNA or RNA, canine DNA or RNA, equine DNA or RNA, or any other mammal of interest. In other aspects, target specific primers or adapters include sequences directed to amplify plant nucleic acids of interest. In other aspects, target specific adapters include sequences directed to amplify infectious agents, e.g., bacterial and/or viral nucleic acids. In some aspects, the amount of nucleic acid required for selective amplification is from about 1 ng to 1 microgram. In some aspects, the amount of nucleic acid required for selective amplification of one or more target sequences is about 1 ng, about 5 ng or about 10 ng. In some aspects, the amount of nucleic acid required for selective amplification of target sequence is about 10 ng to about 200 ng.

As described herein, each of the plurality of adapters comprises a 5' universal handle sequence. In some aspects a universal handle sequence comprises any one or any combination of an amplification primer binding sequence, a sequencing primer binding sequence and/or a capture primer binding sequence. In some aspects the comparable maximal minimum melting temperatures of each adapter universal handle sequence is higher than the comparable maximal minimum melting temperatures of each target nucleic acid sequence and each tag sequence present in the same adapter. Preferably, the universal handle sequences of provided adapters do not exhibit significant complementarity and/or hybridization to any portion of a unique tag sequence and/or target nucleic acid sequence of interest. In some aspects a first universal handle sequence comprises any one or any combination of an amplification primer binding sequence, a sequencing primer binding sequence and/or a capture primer binding sequence. In some aspects a second universal handle sequence comprises any one or any combination of an amplification primer binding sequence, a sequencing primer binding sequence and/or a capture primer binding sequence. In certain aspects first and second universal handle sequences correspond to forward and reverse universal handle sequences and in certain aspects the same first and second universal handle sequences are included for each of the plurality of target specific adapter pairs. Such forward and reverse universal handle sequences are targeted in conjunction with universal primers to carry out a second amplification of repaired amplicons in production of libraries according to methods of the invention. In certain aspects a first 5' universal handle sequence comprises two universal handle sequences(e.g., a combination of an amplification primer binding sequence, a sequencing primer binding sequence and/or a capture primer binding sequence); and a second 5' universal sequence comprises two universal handle sequences (e.g., a combination of an amplification primer binding sequence, a sequencing primer binding sequence and/or a capture primer binding sequence), wherein the 5' first and second universal handle sequences do not exhibit significant hybridization to any portion of a target nucleic acid sequence of interest.

The structure and properties of universal amplification primers or universal primers are well known to those skilled in the art and can be implemented for utilization in conjunction with provided methods and compositions to adapt to specific analysis platforms. Universal handle sequences of the adapters provided herein are adapted accordingly to accommodate a preferred universal primer sequences. For example, e.g., as described herein universal P1 and A primers with optional barcode sequences have been described in the art and utilized for sequencing on Ion Torrent sequencing platforms (Ion Xpress^{™} Adapters, Thermo Fisher Scientific). Similarly, additional and other universal adapter/primer sequences described and known in the art (e.g., Illumina universal adapter/primer sequences can be found,e.g., at https://support.illumina.com/content/dam/illumina-
support/documents/documentation/chemistry_documentation/experiment-design/illumina-adapter-sequences_1000000002694-01.pdf; PacBio universal adapter/primer sequences, can be found, e.g., at https://s3.amazonaws.com/files.pacb.com/pdf/Guide_Pacific_Biosciences_Template_Preparation_and_Se quencing.pdf; etc.) can be used in conjunction with the methods and compositions provided herein. Suitable universal primers of appropriate nucleotide sequence for use with adapters of the invention are readily prepared using standard automated nucleic acid synthesis equipment and reagents in routine use in the art. One single type of universal primer or separate types (or even a mixture) of two different universal primers, for example a pair of universal amplification primers suitable for amplification of repaired amplicons in a second amplification are included for use in the methods of the invention. Universal primers optionally include a different tag (barcode) sequence, where the tag (barcode) sequence does not hybridize to the adapter. Barcode sequences incorporated into amplicons in a second universal amplification can be utilized e.g., for effective identification of sample source.

In some aspects adapters further comprise a unique tag sequence located between the 5' first universal handle sequence and the 3' target-specific sequence, and wherein the unique tag sequence does not exhibit significant complementarity and/or hybridization to any portion of a unique tag sequence and/or target nucleic acid sequence of interest. In some examples the plurality of primer adapter pairs has 10⁴-10⁹ different tag sequence combinations. Thus in certain embodiments each generated target specific adapter pair comprises 10⁴-10⁹ different tag sequences. In some examples the plurality of primer adapters comprise each target specific adapter comprising at least 1 different unique tag sequence and up to 10 ⁵ different unique tag sequences. In some examples the plurality of primer adapters comprise each target specific adapter comprising at least 1 different unique tag sequence and up to 10⁵ different unique tag sequences. In certain examples each generated target specific amplicon generated comprises at least two and up to 10⁹ different adapter combinations comprising different tag sequences, each having two different unique tag sequences. In some embodiments the plurality of primer adapters comprise each target specific adapter comprising 4096 different tag sequences. In certain aspects each generated target specific amplicon generated comprises up to 16,777,216 different adapter combinations comprising different tag sequences, each having two different unique tag sequences.

In some aspects individual primer adapters in the plurality of adapters include a unique tag sequence (e.g., contained in a tag adapter) comprising different random tag sequences alternating with fixed tag sequences. In some aspects, the at least one unique tag sequence comprises a at least one random sequence and at least one fixed sequence, or comprises a random sequence flanked on both sides by a fixed sequence, or comprises a fixed sequence flanked on both sides by a random sequence. In some aspects a unique tag sequence includes a fixed sequence that is 2-2000 nucleotides or base-pairs in length. In some aspects a unique tag sequence includes a random sequence that is 2-2000 nucleotides or base-pairs in length.

Target specific primers and adapters provided herein comprise at least one cleavable moiety. In some aspects a cleavable moiety is within the 3' target-specific sequence. In some aspects a cleavable moiety is at or near the junction between the 5' first universal handle sequence and the 3' target-specific sequence. In some aspects a cleavable moiety is at or near the junction between the 5' first universal handle sequence and the unique tag sequence, and at or near the junction between the unique tag sequence and the 3' target-specific sequence. The cleavable moiety can be present in a modified nucleotide, nucleoside or nucleobase. In some embodiments, the cleavable moiety can include a nucleobase not naturally occurring in the target sequence of interest.

In some aspects the at least one cleavable moiety in the plurality of adapters is a uracil base, uridine or a deoxyuridine nucleotide. In some aspects a cleavable moiety is within the 3' target-specific sequence and the junctions between the 5' universal handle sequence and the unique tag sequence and/or the 3'target specific sequence wherein the at least one cleavable moiety in the plurality of adapters is cleavable with uracil DNA glycosylase (UDG). In some aspects, a cleavable moiety is cleaved, resulting in a susceptible abasic site, wherein at least one enzyme capable of reacting on the abasic site generates a gap comprising an extendible 3' end. In certain aspects the resulting gap comprises a 5'-deoxyribose phosphate group. In certain aspects the resulting gap comprises an extendible 3' end and a 5' ligatable phosphate group.

In another example, inosine can be incorporated into a DNA-based nucleic acid as a cleavable group. In one exemplary embodiment, EndoV can be used to cleave near the inosine residue. In another exemplary embodiment, the enzyme hAAG can be used to cleave inosine residues from a nucleic acid creating abasic sites.

Where a cleavable moiety is present, the location of the at least one cleavable moiety in the adapters does not significantly change the melting temperature (Tm) of any given double-stranded adapter in the plurality of double-stranded adapters. The melting temperatures (Tm) of any two given double-stranded adapters from the plurality of double-stranded adapters are substantially the same, wherein the melting temperatures (Tm) of any two given double-stranded adapters does not differ by more than 10 °C of each other. However, within each of the plurality of adapters, the melting temperatures of sequence regions differs, such that the comparable maximal minimum melting temperature of, for example, the universal handle sequence, is higher than the comparable maximal minimum melting temperatures of either the unique tag sequence and/or the target specific sequence of any adapter. This localized differential in comparable maximal minimum melting temperatures can be adjusted to optimize digestion and repair of amplicons and ultimately improved effectiveness of the methods provided herein.

Further provided but not claimed are compositions comprising a nucleic acid library generated by methods of the invention. At least two and up to one hundred thousand target specific amplicons are included in provided compositions. Provided compositions include highly multiplexed targeted libraries. At least two and up to one hundred thousand target specific tagged amplicons are included in provided compositions. Library compositions include a plurality of target specific amplicons comprising a multiplex of at least two different target nucleic acid sequences. The composition comprises at least 25, 50, 75, 100, 150, 200, 250, 300, 350, 400, 450, 500, 750, 1000, 1250, 1500, 1750, 2000, 2250, 2500, 2750, 3000, 3250, 3500, 3750, 4000, 4500, 5000, 5500, 6000, 7000, 8000, 9000, 10000, 11000, or 12000, or more target-specific amplicons. In some embodiments, the target-specific amplicons comprise one or more exon, gene, exome or region of the genome associated with a clinical or pathological condition, e.g., amplicons comprising one or more sites comprising one or more mutations (e.g., driver mutation) associated with a cancer, e.g., lung, colon, breast cancer, etc., or amplicons comprising mutations associated with an inherited disease, e.g., cystic fibrosis, muscular dystrophies, etc. In some examples, the target-specific amplicons comprise a library of adapter-ligated amplicon target sequences or adapter amplified amplicon target sequences that are about 100 to about 750 base pairs in length.

The structure and properties of universal amplification primers or universal primers are well known to those skilled in the art and can be implemented for utilization in conjunction with provided methods and compositions to adapt to specific analysis platforms. Universal handle sequences of the adapters and amplicons provided herein are adapted accordingly to accommodate a preferred universal primer sequences. For example, e.g., as described herein universal P1 and A primers with optional barcode sequences have been described in the art and utilized for sequencing on Ion Torrent sequencing platforms (Ion Xpress^{™} Adapters, Thermo Fisher Scientific). Similarly, additional and other universal adapter/primer sequences described and known in the art (e.g., Illumina universal adapter/primer sequences can be found,e.g., at https://support.illumina.com/content/dam/illumina-support/documents/documentation/chemistry_documentation/experiment-design/illumina-adapter-sequences_1000000002694-01.pdf; PacBio universal adapter/primer sequences, can be found, e.g., at https://s3.amazonaws.com/files.pacb.com/pdf/Guide_Pacific_Biosciences_Template_Preparation_and_Se quencing.pdf; etc.) can be used in conjunction with the methods and compositions provided herein. Suitable universal primers of appropriate nucleotide sequence for use with libraries of the invention are readily prepared using standard automated nucleic acid synthesis equipment and reagents in routine use in the art. One single type or separate types (or even a mixture) of two different universal primers, for example a pair of universal amplification primers suitable for amplification of a preliminary library may be used in production of the libraries of the invention. Universal primers optionally include a tag (barcode) sequence, where the tag (barcode) sequence does not hybridize to adapter sequence or to target nucleic acid sequences. Barcode sequences incorporated into amplicons in a second universal amplification can be utilized e.g., for effective identification of sample source to thereby generate a barcoded library. Thus provided compositions include highly multiplexed barcoded targeted libraries. Provided compositions also include highly multiplexed barcoded tagged targeted libraries.

In some aspects amplicon libraries comprise a unique tag sequence located between the 5' first universal handle sequence and the 3' target-specific sequence, and wherein the unique tag sequence does not exhibit significant complementarity and/or hybridization to any portion of a unique tag sequence and/or target nucleic acid sequence. In some aspects the plurality of amplicons has 10⁴-10⁹ different tag sequence combinations. Thus in certain aspects each of the plurality of amplicons in a library comprises 10⁴-10⁹ different tag sequences. In some aspects each of the plurality of amplicons in a library comprises at least 1 different unique tag sequence and up to 10 ⁵ different unique tag sequences. In certain aspects each target specific amplicon in a library comprises at least two and up to 10⁹ different combinations comprising different tag sequences, each having two different unique tag sequences. In some aspects each of the plurality of amplicons in a library comprise a tag sequence comprising 4096 different tag sequences. In certain aspects each target specific amplicon of a library comprises up to 16,777,216 different combinations comprising different tag sequences, each having two different unique tag sequences.

### Kits, Systems

Further provided herein are kits for use in preparing libraries of target nucleic acids using methods of the first or second aspects of the invention. Embodiments of a kit comprise a supply of at least a pair of target specific primers and/or adapters as defined herein which are capable of producing a first amplification product; one or more blocking target specific primers as defined herein which are capable of producing a first amplification product, however, are not processed through the workflow to produce adapter ligated amplicon or adapter amplified target amplicon; as well as a supply of at least one universal pair of amplification adapters capable of i) ligating to the digested amplicons of a first aspect of the invention or ii) annealing to the universal handle(s) of the adapter and priming synthesis of an amplification product of a second aspect of the invention, which amplification product would include a target sequence of interest ligated to a universal sequence. Adapters and/or primers may be supplied in kits ready for use, or more preferably as concentrates requiring dilution before use, or even in a lyophilized or dried form requiring reconstitution prior to use. In certain aspects kits further include a supply of a suitable diluent for dilution or reconstitution of the components. Optionally, kits further comprise supplies of reagents, buffers, enzymes, dNTPs, etc., for use in carrying out amplification, digestion, repair, and/or purification in the generation of library as provided herein. Non-limiting examples of such reagents are as described in the Materials and Methods sections of the accompanying Exemplification. Further components which optionally are supplied in the kit include components suitable for purification of libraries prepared using the provided methods. In some aspects, provided is a kit for generating a target-specific library comprising a plurality of target-specific adapters having a 5' universal handle sequence, a 3' target specific sequence and a cleavable group, a DNA polymerase, an adapter, dATP, dCTP, dGTP, dTTP, and a digestion reagent. In some aspects, the kit further comprises one or more antibodies, a repair reagent, universal primers optionally comprising nucleic acid barcodes, purification solutions or columns.

Particular features of adapters for inclusion in kits have been described elsewhere. US Patent No., US9,957, 558B2 and US Patent Application Publication No. US2019/0002872A1. The structure and properties of universal amplification primers are well known to those skilled in the art and can be implemented for utilization in conjunction with provided methods and compositions to adapt to specific analysis platforms (e.g., as described herein universal P1 and A primers have been described in the art and utilized for sequencing on Ion Torrent sequencing platforms). Similarly, additional and other universal adapter/primer sequences described and known in the art (e.g., Illumina universal adapter/primer sequences, PacBio universal adapter/primer sequences, etc.) can be used in conjunction with the methods and compositions provided herein. Suitable primers of appropriate nucleotide sequence for use with adapters included in the kit is readily prepared using standard automated nucleic acid synthesis equipment and reagents in routine use in the art. A kit may include a supply of one single type of universal primer or separate types (or even a mixture) of two different universal primers, for example a pair of amplification primers suitable for amplification of templates modified with adapters in a first amplification. A kit may comprise at least a pair of adapters for first amplification of a sample of interest according to the methods of the invention, plus at least two different amplification primers that optionally carry a different tag (barcode) sequence, where the tag (barcode) sequence does not hybridize to the adapter. A kit can be used to amplify at least two different samples where each sample is amplified according to methods of the invention separately and a second amplification comprises using a single universal primer having a barcode, and then pooling prepared sample libraries after library preparations. In some aspects a kit includes different universal primer-pairs for use in second amplification step described herein. In this context the 'universal' primer-pairs may be of substantially identical nucleotide sequence but differ with respect to some other feature or modification.

Further provided but not claimed are systems, e.g., systems used to practice methods provided herein, and/or comprising compositions provided herein. In some aspects, systems facilitate methods carried out in automated mode. In certain aspects, systems facilitate high throughput mode. In certain aspects, systems include, e.g., a fluid handling element, a fluid containing element, a heat source and/or heat sink for achieving and maintaining a desired reaction temperature, and/or a robotic element capable of moving components of the system from place to place as needed (e.g., a multiwell plate handling element).

### Samples

As defined herein, "sample" and its derivatives, is used in its broadest sense and includes any specimen, culture and/or the like that is suspected of including a target nucleic acid. In some aspects, a sample comprises DNA, RNA, chimeric nucleic acid, hybrid nucleic acid, multiplex-forms of nucleic acids or any combination of two or more of the foregoing. In some aspects a sample useful in conjunction with methods of the invention includes any biological, clinical, surgical, agricultural, atmospheric or aquatic-based specimen containing one or more target nucleic acid of interest. In some aspects, a sample includes nucleic acid molecules obtained from an animal such as a human or mammalian source. In another aspects, a sample includes nucleic acid molecules obtained from a non-mammalian source such as a plant, bacteria, virus or fungus. In some aspects, the source of the nucleic acid molecules may be an archived or extinct sample or species. In some aspects a sample includes isolated nucleic acid sample prepared, for example, from a source such as genomic DNA, RNA or a prepared sample such as, e.g., fresh-frozen or formalin-fixed paraffin-embedded (FFPE) nucleic acid specimen. It is also envisioned that a sample is from a single individual, a collection of nucleic acid samples from genetically related members, multiple nucleic acid samples from genetically unrelated members, multiple nucleic acid samples (matched) from a single individual such as a tumor sample and normal tissue sample, or genetic material from a single source that contains two distinct forms of genetic material such as maternal and fetal DNA obtained from a maternal subject, or the presence of contaminating bacteria or virus nucleic acid in a sample that contains plant or animal nucleic acid. In some aspects, a source of nucleic acid material includes nucleic acids obtained from a newborn (e.g., a blood sample for newborn screening). In some aspects, provided methods comprise amplification of multiple target-specific sequences from a single nucleic acid sample. In some embodiments, provided methods comprise target-specific amplification of two or more target sequences from two or more nucleic acid samples or species. In certain aspects, provided methods comprise amplification of highly multiplexed target nucleic acid sequences from a single sample. In particular examples, provided methods comprise amplification of highly multiplexed target nucleic acid sequences from more than one sample, each from the same source organism.

In some examples a sample comprises a mixture of target nucleic acids and non-target nucleic acids. In certain examples a sample comprises a plurality of initial polynucleotides which comprises a mixture of one or more target nucleic acids and may include one or more non-target nucleic acids. In some examples a sample comprising a plurality of polynucleotides comprises a portion or aliquot of an originating sample; in some embodiments, a sample comprises a plurality of polynucleotides which is the entire originating sample. In some examples a sample comprises a plurality of initial polynucleotides is isolated from the same source or from the same subject at different time points.

In some examples, a nucleic acid sample includes cell-free nucleic acids from a biological fluid, nucleic acids from a tissue, nucleic acids from a biopsied tissue, nucleic acids from a needle biopsy, nucleic acids from a single cell or nucleic acids from two or more cells. In certain examples, a single reaction mixture contains 1-100 ng of the plurality of initial polynucleotides. In some examples a plurality of initial polynucleotides comprises a formalin fixed paraffin-embedded (FFPE) sample; genomic DNA; RNA; cell free DNA or RNA; circulating tumor DNA or RNA; fresh frozen sample, or a mixture of two or more of the foregoing; and in some examples a the plurality of initial polynucleotides comprises a nucleic acid reference standard. In some examples, a sample includes nucleic acid molecules obtained from biopsies, tumors, scrapings, swabs, blood, mucus, urine, plasma, semen, hair, laser capture micro-dissections, surgical resections, and other clinical or laboratory obtained sample. In some examples, a sample is an epidemiological, agricultural, forensic or pathogenic sample. In certain examples, a sample includes a reference. In some examples a sample is a normal tissue or well documented tumor sample. In certain examples a reference is a standard nucleic acid sequence (e.g., Hg19).

### Target Nucleic Acid Sequence Analysis

Provided methods and compositions of the invention are particularly suitable for amplifying, optionally tagging, and preparing target sequences for subsequent analysis. Thus, in some aspects, methods provided herein include analyzing resulting library preparations. For example, methods comprise analysis of a polynucleotide sequence of a target nucleic acid, and, where applicable, analysis of any tag sequence(s) added to a target nucleic acid. In some aspects wherein multiple target nucleic acid regions are amplified, provided methods include determining polynucleotide sequences of multiple target nucleic acids. Provided methods further optionally include using a second tag sequence(s), e.g., barcode sequence, to identify the source of the target sequence (or to provide other information about the sample source). In certain aspects, use of prepared library composition is provided for analysis of the sequences of the nucleic acid library.

In particular aspects, use of prepared tagged library compositions is provided for further analyzing the sequences of the target nucleic acid library. In some aspects determination of sequences comprises determining the abundance of at least one of the target sequences in the sample. In some aspects determination of a low frequency allele in a sample is comprised in determination of sequences of a nucleic acid library. In certain aspects, determination of the presence of a mutant target nucleic acid in the plurality of polynucleotides is comprised in determination of sequences of a nucleic acid library. In some aspects, determination of the presence of a mutant target nucleic acid comprises detecting the abundance level of at least one mutant target nucleic acid in the plurality of polynucleotides. For example, such determination comprises detecting at least one mutant target nucleic acid is present at 0.05% to 1% of the original plurality of polynucleotides in the sample, detecting at least one mutant target nucleic acid is present at about 1% to about 5% of the polynucleotides in the sample, and/or detecting at least 85%-100% of target nucleic acids in sample. In some aspects, determination of the presence of a mutant target nucleic acid comprises detecting and identification of copy number variation and/or genetic fusion sequences in a sample.

In some aspects, nucleic acid sequencing of the amplified target sequences produced by the teachings of this disclosure include *de novo* sequencing or targeted re-sequencing. In some aspects, nucleic acid sequencing further includes comparing the nucleic acid sequencing results of the amplified target sequences against a reference nucleic acid sequence. In some aspects, nucleic acid sequencing of the target library sequences further includes determining the presence or absence of a mutation within a nucleic acid sequence. In some aspects, nucleic acid sequencing includes the identification of genetic markers associated with disease (e.g., cancer and/or inherited disease).

In some aspects, prepared library of target sequences of the disclosed methods is used in various downstream analysis or assays with, or without, further purification or manipulation. In some aspects analysis comprises sequencing by traditional sequencing reactions, high throughput next generation sequencing, targeted multiplex array sequence detection, or any combination of two or more of the foregoing. In certain aspects analysis is carried out by high throughput next generation sequencing. In particular aspects sequencing is carried out in a bidirectional manner, thereby generating sequence reads in both forward and reverse strands for any given amplicon.

In some aspects, library prepared according to the methods provided herein is then further manipulated for additional analysis. For example, prepared library sequences are used in downstream enrichment techniques known in the art, such a bridge amplification or emPCR to generate a template library that is then used in next generation sequencing. In some aspects, the target nucleic acid library is used in an enrichment application and a sequencing application. For example, sequence determination of a provided target nucleic acid library is accomplished using any suitable DNA sequencing platform. In some aspects, the library sequences of the disclosed methods or subsequently prepared template libraries is used for single nucleotide polymorphism (SNP) analysis, genotyping or epigenetic analysis, copy number variation analysis, gene expression analysis, analysis of gene mutations including but not limited to detection, prognosis and/or diagnosis, detection and analysis of rare or low frequency allele mutations, nucleic acid sequencing including but not limited to de novo sequencing, targeted resequencing and synthetic assembly analysis. In one aspect, prepared library sequences are used to detect mutations at less than 5% allele frequency. In some aspects, the methods disclosed herein is used to detect mutations in a population of nucleic acids at less than 4%, 3%, 2% or at about 1% allele frequency. In another aspects, libraries prepared as described herein are sequenced to detect and/or identify germline or somatic mutations from a population of nucleic acid molecules. In certain aspects, sequencing adapters are ligated to the ends of the prepared libraries generate a plurality of libraries suitable for nucleic acid sequencing.

In some aspects, methods for preparing a target-specific amplicon library are provided for use in a variety of downstream processes or assays such as nucleic acid sequencing or clonal amplification. Additionally disclosed but not claimed herein, the library is amplified using bridge amplification or emPCR to generate a plurality of clonal templates suitable for nucleic acid sequencing. For example, optionally following target-specific amplification a secondary and/or tertiary amplification process including, but not limited to, a library amplification step and/or a clonal amplification step is performed. "Clonal amplification" refers to the generation of many copies of an individual molecule. Various methods known in the art is used for clonal amplification. For example, emulsion PCR is one method, and involves isolating individual DNA molecules along with primer-coated beads in aqueous bubbles within an oil phase. A polymerase chain reaction (PCR) then coats each bead with clonal copies of the isolated library molecule and these beads are subsequently immobilized for later sequencing. Emulsion PCR is used in the methods published by Marguilies *et al.* and Shendure and Porreca *et al.* (also known as "polony sequencing", commercialized by Agencourt and recently acquired by Applied Biosystems). Margulies, et al. (2005) Nature 437: 376-380; Shendure et al., Science 309 (5741): 1728-1732. Another method for clonal amplification is "bridge PCR," where fragments are amplified upon primers attached to a solid surface. Another method for clonal amplification uses isothermal amplification in lieu of PCR, along with separation of nucleic acid molecules on a support, e.g., primer coated beads; and isothermal amplification coats each support with clonal copies of the isolated nucleic acid molecule. These methods, as well as other methods of clonal amplification, produce many physically isolated locations that each contain many copies derived from a single molecule polynucleotide fragment. Thus, in some embodiments not covered by the claimed invention, the one or more target specific amplicons are amplified using for example, bridge amplification or emPCR or isothermal amplification to generate a plurality of clonal templates suitable for nucleic acid sequencing.

In some aspects, at least one of the library sequences to be clonally amplified are attached to a support or particle. A support can be comprised of any suitable material and have any suitable shape, including, for example, planar, spheroid or particulate. In some aspects, the support is a scaffolded polymer particle as described in U.S. Published App. No. 20100304982. In certain aspects methods comprise depositing at least a portion of an enriched population of library sequences onto a support (e.g., a sequencing support), wherein the support comprises an array of sequencing reaction sites. In some aspects, an enriched population of library sequences are attached to the sequencing reaction sites on the support. wherein the support comprises an array of 10² - 10¹⁰ sequencing reaction sites.

Sequence determination means determination of information relating to the sequence of a nucleic acid and may include identification or determination of partial as well as full sequence information of the nucleic acid. Sequence information may be determined with varying degrees of statistical reliability or confidence. In some examples sequence analysis includes high throughput, low depth detection such as by qPCR, rtPCR, and/or array hybridization detection methodologies known in the art. In some examples, sequencing analysis includes the determination of the in depth sequence assessment, such as by Sanger sequencing or other high throughput next generation sequencing methods. Next-generation sequencing means sequence determination using methods that determine many (typically thousands to billions) nucleic acid sequences in an intrinsically massively parallel manner, e.g. where many sequences are read out, e.g., in parallel, or alternatively using an ultra-high throughput serial process that itself may be parallelized. Thus, in certain examples, methods of the invention include sequencing analysis comprising massively parallel sequencing. Such methods include but are not limited to pyrosequencing (for example, as commercialized by 454 Life Sciences, Inc., Branford, Conn.); sequencing by ligation (for example, as commercialized in the SOLiD^{™} technology, Life Technologies, Inc., Carlsbad, Calif.); sequencing by synthesis using modified nucleotides (such as commercialized in TruSeq^{™} and HiSeg^{™}. technology by Illumina, Inc., San Diego, Calif.; HeliScope ^{™} by Helicos Biosciences Corporation, Cambridge, Mass.; and PacBio Sequel^{®} or RS systems by Pacific Biosciences of California, Inc., Menlo Park, Calif.), sequencing by ion detection technologies (e.g., Ion Torrent^{™} semiconductor sequencing technology, Life Technologies, Carlsbad, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods.

For example, in certain aspects, libraries produced by the teachings of the present disclosure are sufficient in yield to be used in a variety of integrated and/or downstream sequencing applications including the Ion Torrent^{™} Genexus^{™} Sequencing System (Life Technologies), the Ion GeneStudio S5 Sequencing System (Life Technologies) with either manual template preparation or automated preparation using the Ion Chef System; and/or the Ion Xpress^{™} Template Kit using an Ion Torrent^{™} PGM system (e.g., PCR-mediated addition of the nucleic acid fragment library onto Ion Sphere^{™} Particles)(Life Technologies, Part No. 4467389) or Ion Torrent Proton^{™} system). For example, instructions to prepare a template library from the amplicon library can be found in the Ion Xpress Template Kit User Guide (Life Technologies, Part No. 4465884). Instructions for loading the subsequent template library onto the Ion Torrent^{™} Chip for nucleic acid sequencing are described in the Ion Sequencing User Guide (Part No. 4467391). Similarly, sequencing using other platforms (e.g., PacBio, Illumina, Helicos, Complete Genomics, Oxford Nanopore) may be carried out using adapted methodologies to incorporate the relevant template preparation according to the instructions and guidance provided with each of the respective platforms.

The initiation point for the sequencing reaction may be provided by annealing a sequencing primer to a product of a solid-phase amplification reaction. In this regard, one or both of the adapters added during formation of template library may include a nucleotide sequence which permits annealing of a sequencing primer to amplified products derived by whole genome or solid-phase amplification of the template library. Depending on implementation of an embodiment of the invention, a tag sequence and/or target nucleic acid sequence may be determined in a single read from a single sequencing primer, or in multiple reads from two different sequencing primers. In the case of two reads from two sequencing primers, a 'tag read' and a 'target sequence read' are performed in either order, with a suitable denaturing step to remove an annealed primer after the first sequencing read is completed.

In some aspects, a sequencer is coupled to server that applies parameters or software to determine the sequence of the amplified target nucleic acid molecules. In certain aspects, the sequencer is coupled to a server that applies parameters or software to determine the presence of a low frequency mutation allele present in a sample.

Certain embodiments are further described in the following examples. The following is provided as exemplification only and are not intended to limit the scope of the embodiments nor the claimed invention in any way.

### EXEMPLIFICATION

### Example 1.

Blocking primer methods using DNA, RNA and LNA blockers previously described were compared to methods of the present invention. Briefly, libraries were constructed using Ion Ampliseq Cancer Hotspot Panel v2 using standard Ion AmpliSeq protocols according to manufacturer instructions, including thermal cycling conditions (99°C, 15 sec denature, 60°C, 4min anneal/extend). In some cases, slightly modified cycling conditions (i.e., the ramp rate during annealing was decreased to 10% of maximum (0.33°C/sec) from 72°C to 60°C). Experiments were carried out using genomic DNA HD701 (Horizon Discovery, Waterbeach, UK). DNA blocking primers were added at 15, 45, 135, or 405nM final concentration and cycled using standard or modified cycling protocols. Subsequent library preparation steps followed standard Ion AmpliSeq protocol, followed by sequencing using Ion Torrent Sequencing. See Ion AmpliSeq Library Kit 2.0 User Guide MAN0006735 Thermo Fisher Scientific. Results are depicted in Figure 3A. Modified conditions appear to be required for effective blocking, and longer blockers are more effective, though not substantially. Our experiments indicate lack of high specificity and strong suppression using previously described DNA blocker oligos in conjunction with Ion AmpliSeq technology. Further, requirements for modified cycling conditions are not amenable to simple addition to existing panels for flexible selection of targets of interest.

**TABLE 1: DNA and RNA Blocker Oligos**

| **Oligo** | **DNA Blocker Sequence *** | **SEQ ID NO** |
|---|---|---|
| EGFR_BL01 | TTTGGGCTGGCCAAACTGCTGGGTGC-X | 1 |
| EGFR_BL02 | TTTGGGCTGGCCAAACTGCTGGGTG-X | 2 |
| EGFR_BL03 | TTTGGGCTGGCCAAACTGCTGGGT-X | 3 |
| EGFR_BL04 | TTTGGGCTGGCCAAACTGCTGGG-X | 4 |
| EGFR_BL05 | TTTGGGCTGGCCAAACTGCTGG-X | 5 |
| EGFR_BL06 | TTTGGGCTGGCCAAACTGCTG-X | 6 |
| EGFR_BL07 | TTTGGGCTGGCCAAACTGCT-X | 7 |
| EGFR_BL08 | TTTGGGCTGGCCAAACTGC-X | 8 |
| EGFR_BL09 | TTTGGGCTGGCCAAACTG-X | 9 |
| EGFR_BL10 | TTGGGCTGGCCAAACTGCTGGGTGC-X | 10 |
| EGFR_BL11 | TGGGCTGGCCAAACTGCTGGGTGC-X | 11 |
| EGFR_BL12 | GGGCTGGCCAAACTGCTGGGTGC-X | 12 |
| EGFR_BL13 | GGCTGGCCAAACTGCTGGGTGC-X | 13 |
| EGFR_BL14 | GCTGGCCAAACTGCTGGGTGC-X | 14 |
| EGFR_BL15 | CTGGCCAAACTGCTGGGTGC-X | 15 |

| **Oligo** | **RNA Blocker Sequence*** | |
|---|---|---|
| EGFR_BL01 | UUUGGGCUGGCCAAACUGCUGGGUGC-X | 16 |
| EGFR_BL02 | UUUGGGCUGGCCAAACUGCUGGGUG-X | 17 |
| EGFR_BL03 | UUUGGGCUGGCCAAACUGCUGGGU-X | 18 |
| EGFR_BL04 | UUUGGGCUGGCCAAACUGCUGGG-X | 19 |
| EGFR_BL05 | UUUGGGCUGGCCAAACUGCUGG-X | 20 |
| EGFR_BL06 | UUUGGGCUGGCCAAACUGCUG-X | 21 |
| EGFR_BL07 | UUUGGGCUGGCCAAACUGCU-X | 22 |
| EGFR_BL08 | UUUGGGCUGGCCAAACUGC-X | 23 |
| EGFR_BL09 | UUUGGGCUUGGCCAAACUG-X | 24 |
| EGFR_BL10 | UUGGGCUGGCCAAACUGCUGGGUGC-X | 25 |
| EGFR_BL11 | UGGGCUGGCCAAACUGCUGGGUGC-X | 26 |
| EGFR_BL12 | GGGCUUGGCCAAACUGCUGGGUGC-X | 27 |
| EGFR_BL13 | GGCUGGCCAAACUGCUGGGUGC-X | 28 |
| EGFR_BL14 | GCUGGCCAAACUGCUGGGUGC-X | 29 |
| EGFR_BL15 | CUGGCCAAACUGCUGGGUGC-X | 30 |
| **Corresponding EGFR L858R SNP location is underlined; X denotes non-extendable 3-carbon spacer* | | |

For RNA blocker experiments, libraries were prepared using blocking oligos as described for DNA blocking, except blocking primers were made with RNA rather than DNA. Results are depicted in Figure 3B. Although identical designs, use of RNA blocker oligos does not require modified cycling conditions for blocking, and results demonstrate more effective blocking than DNA oligo blocker experiments. Like DNA blockers, RNA blocking does not appear to generate specificity. Thus, RNA blocking may not be amenable to multiplex targeting or drop in to standard reactions without significant modification and optimization requirements.

For LNA blocker experiments, libraries were prepared as described above except blocking primers included 3-8 LNA bases to increase the Tm of the primer relative to a DNA blocker. Blockers to 10 alleles (1-24.5% allele frequency) were multiplexed and added to the standard cycling reaction for library preparation. Templating and sequencing of libraries was carried out as described for DNA blocking experiment above. Results are depicted in Figure 3C. Unlike DNA and RNA oligos discussed above, LNA blockers provided effective and specific suppression of targets, including in multiplex format compatible with standard cycling conditions. However, LNA blockers did seem to require significant amounts of oligo for suppression effect (at least 135nM, data not shown). Additionally, LNA synthesis is complex and relatively expensive and in view of high levels of oligo required for suppression, would be an expensive endeavor for multiplex selection of suppression.

**TABLE 2: LNA Blocker Oligos**

| **Name** | **LNA Blocker Sequence** | **SEQ ID NO** |
|---|---|---|
| NRAS_Q61K | A+C+TCT+TCT+T+G+TCC+AGC+TG-X | 31 |
| PIK3CA_E545K | A+AAT+CAC+T+G+AGC+AGG+AG-X | 32 |
| PIK3CA_H1047R | T+GAT+GCA+C+A+TCA+TGG+TG-X | 33 |
| KIT_D816V | A+GCC+AGA+G+A+CAT+CAA+GA-X | 34 |
| EGFR_G719S | A+AGT+GCT+G+G+GCT+CCG+GT-X | 35 |
| EGFR_T790M | G+CTC+ATC+A+C+GCA+GCT+CA-X | 36 |
| EGFR_L858R | T+TT+GGG+C+T+GGC+CAA+AC-X | 37 |
| BRAF_V600E | C+G+AGA+TTT+C+A+CTG+TAG+CT-X | 38 |
| KRAS_G12D/G13D | GGAGCT+GGT+GGCGTAG+GC-X | 39 |
| *+ denotes LNA backbone positions; X denotes a non-extendable 3-carbon spacer blocking group; corresponding SNP location is underlined* | | |

In accordance with the instant invention, standard DNA oligos as blocking primers were prepared for use in conjunction with the Ion AmpliSeq Cancer Hotspot Panel v2. Blocking primers were designed against NRAS Q61K with SNP placed at 0,1,2,3,or 4 bases from the 3' end of the blocking primer, and primers were included at 25, 50, 100, or 200nM final concentration in a standard Ion AmpliSeq Cancer Hotspot Panel v2 reaction. Blocking primers were effective across the range of input concentrations as well as locations across the location across the WT sequence corresponding to the SNP location. Results are depicted in FIG 3D. The blocking oligos demonstrated strong suppression, similar to LNA, however, with better specificity and at lower concentrations. No strong tendency regarding site of mismatch was seen. Thus, in view of the results, standard DNA oligos used in conjunction with Ion AmpliSeq technology represents the most efficient and effective suppression for use in conjunction with detection of low frequency target sequences. Standard suppression oligo sequences resulted in strong, specific suppression of target wild type alleles, generating effective detection of low frequency alleles down to 0.2%. Oligos can be simply incorporated into standard Ion AmpliSeq multiplex technology protocols with simplified optimization.

**TABLE 3: Ion AmpliSeq Blocker Oligos**

| **Name** | **Ion AmpliSeq Blocker Sequence** | **SEQ ID NO** |
|---|---|---|
| NRAS_Q61K-0 | TCTCATGGCACTGTACTCTTCTTG | 40 |
| NRAS_Q61K-1 | TCTCATGGCACTGTACTCTTCTTGT | 41 |
| NRAS_Q61K-2 | CTCATGGCACTGTACTCTTCTTGTC | 42 |
| NRAS_Q61K-3 | CATGGCACTGTACTCTTCTTGTCC | 43 |
| NRAS_Q61K-4 | TGGCACTGTACTCTTCTTGTCCA | 44 |

Example 2: A custom Ion AmpliSeq 122-plex panel comprising two pools of Ion AmpliSeq primers directed to target sequences comprising oncology hotspot SNP sites and including blocking oligos directed to wild type sequences comprising the targeted variant SNP sites was designed. Blocking oligos were designed to target wild type sequences covering position of SNPs at various locations. Each targeted site represented locations of SNPs at various distances (e.g., 17-35bp) from 3' end of the Ion AmpliSeq primer in the panel. Blocker oligos were designed to the wild type sequence with analogous SNP located at 0 to 7bp from 3' end and a single blocker may be designed against multiple adjacent SNPs. While each of the blockers performed effectively, the highest performing candidate was selected for multiplex format.

Libraries were constructed with and without blocking using HD701 DNA supplemented with synthetic templates representing rare alleles under the standard Ion AmpliSeq Library Preparation protocol for the 122-plex custom Ion Ampliseq panel, and sequenced. Blocker oligos were added to the Library Amplification reaction at 100 nM each. Allele frequency was compared and showed 10-1000 fold enrichment of rare alleles with blocker (full data set average enrichment was 40X). Representative Data from 10 amplicons using the custom blocking panel are depicted in FIG 4A. Amplicons are indicated by design coordinates (ie 1.10023 or 2.25456) and alleles are indicated by SNP. Median WT suppression for entire panel was 91% with 0% mutant suppression. Comparison of rare allele enrichment in blocked or unblocked samples is depicted in FIG 4B. Each point represents a single SNP.

**TABLE4: CUSTOM ION AMPLISEQ 122-PLEX PANEL WITH BLOCKERS**

| **4A** | **Forward Primer** | **SEQ ID NO** |
|---|---|---|
| 01.10023 | CAGGCACUTGATGATACUCACT | 45 |
| 03.29476 | GGAAGAAAAACAAUCCCACTUGCG | 46 |
| 04.7199 | CCATTAACAUGGCCTACCAGAGTUG | 47 |
| 06.3669 | AGCCACACAUGCCATCATTCUAG | 48 |
| 08.21927 | TCTGGTTTCUGGTGGGACCATUA | 49 |
| 10.40643 | TGGATCACATCUCTACCAGAGTTAAUCA | 50 |
| 11.23267 | GCTATTAUTGATGGCAAAUACACAGAGG | 51 |
| 12.11818 | CATCTACAAAGTGGUTCTGGATTAGCUG | 52 |
| 13.3146 | AGGAGGTCAUGGCATCGAGUT | 53 |
| 14.2747 | ATTAAAGCTGGCUATGGCACCUG | 54 |
| 15.10751 | GUGCCAAGCCUCACACCA | 55 |
| 17.5475 | ATTTTTCCUCACAGCTCGTTCAUCG | 56 |
| 18.14007 | CTTTACCCCTCCUTCCTAGAGAGTUAG | 57 |
| 19.25590 | TGCCCACUGTGTTACUGCCAT | 58 |
| 21.9067 | CATTCGGCACAGGAUGACTGTUA | 59 |
| 22.2410 | ACATTCCGAATAUAGAGAACCTCAATCUC | 60 |
| 23.3877 | AGGCATTUGCCGGCAGUC | 61 |
| 24.15926 | CGCCAGGCUCACCTCTATAGUG | 62 |
| 25.29220 | TGCAGAAAACAGAUCTGTATTTATTTCAGUGT | 63 |
| 26.7457 | CAGTCCTCAUGTACTGGUCCC | 64 |
| 27.8414 | CAAGATTTACCTCUATTGTTGGATCATATUCG | 65 |
| 28.5397 | CAGACACTGUACAAGCTCUACGA | 66 |
| 29.26684 | TCACAGTGGAUTCGAGAAGUGAC | 67 |
| 31.17423 | CATTGGTAGCUGGTGATGTTCCUC | 68 |
| 32.2472 | ACACUCTUGAGGGCCACAAAG | 69 |
| 33.12700 | GCTCCTTGUAGCCAATGAAGGUG | 70 |
| 34.13971 | GGACCAACTUGGAGGCCTUG | 71 |
| 36.25412 | TCTATTTTCTCTUCCCTGCAGATGUCA | 72 |
| 37.17650 | CUAGGCGAGGAGCUCCAGT | 73 |
| 39.22318 | CATTCTGCUGGTCGTGGUCT | 74 |
| 39.69182 | TCTTCTCACTCAUATCCTCCTCTTTCUG | 75 |
| 40.10525 | AGCGUACCCTTGUCCCCA | 76 |
| 40.65199 | GCCCUCCCAGAAGGTCTACAUG | 77 |
| 41.2301 | CAGCTACCUGACCGACGTUG | 78 |
| 43.15757 | TCCTTTCGAAGUCATCGTCTTUGA | 79 |
| 44.6908 | CCAGGGUCACTCAGCTUGAT | 80 |
| 45.72120 | TCTGACTCAGCUGGTATTGGGAUT | 81 |
| 47.759 | AATGTGAGCCCUTGAGATCUGC | 82 |
| 48.24545 | GAGGGCGGGUCTCUCGGA | 83 |
| 49.3900 | ACGATTTCCCTUGGAGATATCGATCUG | 84 |
| 50.8773 | ATACAAGTUGGAAATTTCUGGGCCAT | 85 |
| 52.679 | AATGTCAATTAGCUGGAACATCUGAAACT | 86 |
| 54.18517 | GACAGAAAAGCGGCUGTTAGUCA | 87 |
| 55.43552 | GAATCCTAGTAGAAUGTTTACTACCAAAUGGA | 88 |
| 55.767 | AAACAAGACGACUTTGTGACCTUCG | 89 |
| 56.25166 | TGAATGGAGAAACAUCTACAAAAUCCCT | 90 |
| 57.24859 | TGTGCATATGTGTAUGTTGAGTGTATACATUAG | 91 |
| 58.8828 | GGGAAGAAAAGUGTTTTGAAATGTGTUT | 92 |
| 59.18973 | TTTATTTTACAGAGUAACAGACTAGCUAGAGAC | 93 |
| 60.28665 | GCCTGCTTTUGGAGTCCTATUGT | 94 |
| 61.4724 | AGTTGGCCUGAATCACTATATTUCCAT | 95 |
| 62.14162 | ATAAATCTTTTCTCAAUGATGCTTGGCUCT | 96 |
| 63.4977 | GGTGGCCCCUGAGCGUC | 97 |
| 65.24277 | GCAGACUTCGGGCUGGC | 98 |
| 66.11768 | CCAGTTACCTGUCCTGGTCATTUA | 99 |
| 68.19575 | GCACAAGGAAAAAUTGTGAAGATCUGT | 100 |
| 69.20842 | GACATATGGCCAUTTCTGTTTTCCTGUA | 101 |
| 71.122176 | GTGCTTTTTGCUAAAATGCATGTTUCC | 102 |
| 71.96540 | GATCTATUTTTCCCTTTCUCCCCACAG | 103 |
| 72.2307 | AATGAATTTAAATGGUTTTCTTTTCTCCUCCAA | 104 |
| 73.6720 | AGGGCATTTGCAUTATGAAACCAAUAT | 105 |
| 75.5088 | ATGGAGCAUCTGTACAGCAUGA | 106 |
| 76.27276 | GGTTATGTCCUCATTGCCCUCAAC | 107 |
| 78.27403 | TCAGGAAACAAAAAUTTGTGCTAUGCAA | 108 |
| 80.2024 | ACACCCAGUGGAGAAGCUC | 109 |
| 81.9975 | CAGTTAACGTCTUCCTTCTCTCTCUGT | 110 |
| 82.11913 | CCTCCAGGAAGCCUACGUGAT | 111 |
| 83.29222 | TGAAAACACCGCAGCAUGUCA | 112 |
| 84.23210 | GCTTGTAAGUGCCCGAAGTGUAA | 113 |
| 85.11502 | GCAGTGCUAACCAAGTTCTTTCTUT | 114 |
| 86.2729 | AATTCACAGUCAAGGTTGCTGATTTUG | 115 |
| 88.12504 | GGCTACAAGAACUACCGAUACCGT | 116 |
| 90.5484 | CATCCCTGACUGTGAGATCAAGAAUC | 117 |
| 91.4096 | ACAAAATGGAUCCAGACAACTGTUC | 118 |
| 92.17956 | GTTGTTAAACATAUCCTATTATGACTTGUCACA | 119 |
| 94.1226 | ACATGATAGAGGUGACATTTUCAAAGC | 120 |
| 96.21382 | GGATGUACAGCCAGTGTGUC | 121 |
| 02.25456 | TGTTGCCATTUCAGGGTTTCUGAAT | 122 |
| 03.22932 | CTGTGCUCACACATGTTCTUCAGAAT | 123 |
| 05.8811 | CTTCTTCTCAUCGCGGGCUT | 124 |
| 07.15610 | TTGCCUAGACAGCACCGTAAUG | 125 |
| 09.10389 | GGGAAGAGCCUCCACCATCUC | 126 |
| 10.14770 | CAGTTCGUGGGCTTGTTTTGTAUC | 127 |
| 11.23267 | GCTATTAUTGATGGCAAAUACACAGAGG | 128 |
| 12.11818 | CATCTACAAAGTGGUTCTGGATTAGCUG | 129 |
| 14.2747 | ATTAAAGCTGGCUATGGCACCUG | 130 |
| 15.10751 | GUGCCAAGCCUCACACCA | 131 |
| 16.21992 | TCCATCCUGACCTGGTATGGUC | 132 |
| 17.5475 | ATTTTTCCUCACAGCTCGTTCAUCG | 133 |
| 20.15351 | CGAATGCAGTUTTTCCTCCTACUCA | 134 |
| 21.9067 | CATTCGGCACAGGAUGACTGTUA | 135 |
| 23.3877 | AGGCATTUGCCGGCAGUC | 136 |
| 24.15926 | CGCCAGGCUCACCTCTATAGUG | 137 |
| 24.16763 | CGGTGCGCAUGTACTGGUC | 138 |
| 25.1913 | AATAAAAGGAATTCCAUAACTTCTTGCTAAGUC | 139 |
| 26.7457 | CAGTCCTCAUGTACTGGUCCC | 140 |
| 27.8414 | CAAGATTTACCTCUATTGTTGGATCATATUCG | 141 |
| 29.26684 | TCACAGTGGAUTCGAGAAGUGAC | 142 |
| 30.27449 | TTCCATAGUGACCAAGACCATCUGT | 143 |
| 31.13656 | AUGGTAAGAAGTTGUAAAGAGACAGCCT | 144 |
| 32.2472 | ACACUCTUGAGGGCCACAAAG | 145 |
| 34.13971 | GGACCAACTUGGAGGCCTUG | 146 |
| 35.7365 | CAGCTAATUCATCTGGAGAUCAAACCC | 147 |
| 37.28991 | GGGATGTTTUTGCAGATGAUGGG | 148 |
| 38.15804 | GTATGTGGCUACATGTTCCTGAUCT | 149 |
| 39.36690 | CTGAGGAAGGUGAAGGTGCUT | 150 |
| 39.75561 | TGTUGAGGGAAAACACAUCCC | 151 |
| 40.10525 | AGCGUACCCTTGUCCCCA | 152 |
| 40.2682 | ACAACACACAGUTGGAGGACTUC | 153 |
| 42.11359 | GGGATTGCAGAUTGGGCCTUG | 154 |
| 43.15757 | TCCTTTCGAAGUCATCGTCTTUGA | 155 |
| 45.14205 | ATGACCTUCAGCAGCACCUC | 156 |
| 46.11706 | GCAGCCACACCCCAUTCTUG | 157 |
| 48.20498 | CUCACCCCAAUGCAGCGA | 158 |
| 49.13870 | CACTTCTTACCUTCACAGCCACTUG | 159 |
| 49.8398 | AGGGCTUCCATGAGGAAAUCCA | 160 |
| 51.18783 | TTATAAACCGTUCCAAAAGCACCUGA | 161 |
| 53.9888 | CAAGGTGCCCUATTACCTCAATCAUC | 162 |
| 54.18517 | GACAGAAAAGCGGCUGTTAGUCA | 163 |
| 55.5844 | AAGATGAATCTTCTUACATTTTCGTAAGTGTUA | 164 |
| 56.25166 | TGAATGGAGAAACAUCTACAAAAUCCCT | 165 |
| 57.13656 | GAACAGGUATCTACCAUGGAGGAGA | 166 |
| 58.279 | AAACTTGTTTGATUACACAGACACTCUA | 167 |
| 59.18973 | TTTATTTTACAGAGUAACAGACTAGCUAGAGAC | 168 |
| 60.28665 | GCCTGCTTTUGGAGTCCTATUGT | 169 |
| 62.26615 | CCTTAGATAAAACUGAGCAAGAGGCUT | 170 |
| 63.4977 | GGTGGCCCCUGAGCGUC | 171 |
| 64.5856 | GAGCTGGUGGAGGCUGAC | 172 |
| 65.8114 | CAGGACAGCCUGACCUCAC | 173 |
| 67.1666 | AAGCTCTCATGUCTGAACTGAAGAUAAT | 174 |
| 68.19575 | GCACAAGGAAAAAUTGTGAAGATCUGT | 175 |
| 70.2019 | AATGGCACGGUTGAATGUAAGG | 176 |
| 71.8138 | AAGCCCTCAUGTCTGAACUCAAAG | 177 |
| 71.9091 | AAGGTTGTUGAGGAGATAAAUGGAAACAA | 178 |
| 72.26276 | TCCTTACTCAUGGTCGGAUCACAAA | 179 |
| 74.10774 | CTCCTAAGATGUCCACTGCTGTUC | 180 |
| 75.5088 | ATGGAGCAUCTGTACAGCAUGA | 181 |
| 77.4991 | CCCACCACGUACCAGAUGGA | 182 |
| 79.11036 | CCAGTGUGCCCACTACATUGA | 183 |
| 80.2024 | ACACCCAGUGGAGAAGCUC | 184 |
| 82.11913 | CCTCCAGGAAGCCUACGUGAT | 185 |
| 83.29222 | TGAAAACACCGCAGCAUGUCA | 186 |
| 84.23210 | GCTTGTAAGUGCCCGAAGTGUAA | 187 |
| 86.4292 | AGAAUACTATAGTGUACACAACAAAACAGGT | 188 |
| 87.13668 | CTTCTTTGUGGGCAGCATUGG | 189 |
| 89.6924 | CCTGTCCUCCACAGGCATTTUT | 190 |
| 91.4096 | ACAAAATGGAUCCAGACAACTGTUC | 191 |
| 92.17956 | GTTGTTAAACATAUCCTATTATGACTTGUCACA | 192 |
| 93.19150 | TCCUTAGTCTTTCTTUGAAGCAGCAA | 193 |
| 95.2994 | ACCTGAAAAUGACACTTTGTAAGUCAAA | 194 |
| 97.6706 | CAACCCTGTTTUTCTCCCTCTTATUGT | 195 |
| | | |

| **4B** | **Reverse Primer** | **SEQ ID NO** |
|---|---|---|
| 01.10023 | AACUACAGAATCACAUGCCACACAG | 196 |
| 03.29476 | CCAACAAGAT TCUGAAGAACATGT GUG | 197 |
| 04.7199 | CCAAAACCCUCCTGATGUACACG | 198 |
| 06.3669 | CCCUTGTGGCCTAUGACAAGAAAAT | 199 |
| 08.21927 | GGAUCTCTTCAUGCACCGGAA | 200 |
| 10.40643 | CTGGCCAAGAGUTACGGGATUC | 201 |
| 11.23267 | AACAAGTGGTTAUAGATGGTGAAACCUG | 202 |
| 12.11818 | ATTAACCCUGATTACTGGTTUCCAACA | 203 |
| 13.3146 | TATGGTCTTGGACAUCCAGGATCUG | 204 |
| 14.2747 | GATGTGCTGTUGAGACCTCTGUG | 205 |
| 15.10751 | TGAGGAGAUGGGTGGCTUGT | 206 |
| 17.5475 | CACCTGGCUCCTCTUCACG | 207 |
| 18.14007 | ACTTACACAUCACTTTGCGUGGT | 208 |
| 19.25590 | TAGCAGCCAGAAAUGTTTTGGUAACAG | 209 |
| 21.9067 | GCAATCUAGCGCCUGGAAGAG | 210 |
| 22.2410 | GTGCGACGCCUTGUGGT | 211 |
| 23.3877 | GTCACUGACAGCCCTCUGGA | 212 |
| 24.15926 | TGAGGAGCGAUGACGGAATAUAAG | 213 |
| 25.29220 | TTGCCUTCTAGAACAGUAGACACAAAAC | 214 |
| 26.7457 | CTGTGTTUCTCCCTTCUCAGGA | 215 |
| 27.8414 | ATTATAAGGCCUGCTGAAAATGACUGA | 216 |
| 28.5397 | GAAUAAAGAGGAGCAGGTUGAGGAA | 217 |
| 29.26684 | TAGTTCAACAUGACGAAGAUGGCAA | 218 |
| 31.17423 | AGCCCATTTTTAUCTACTTCCATCTUGT | 219 |
| 32.2472 | ATTGTAGGGTCUCCCTTGATCUGAG | 220 |
| 33.12700 | GAGGGTCUGACGGGTAGAGUG | 221 |
| 34.13971 | CTGATCTTCTCAAAGUCGTCATCCTUC | 222 |
| 36.25412 | GUAGGACCTTGUGCCCACGA | 223 |
| 37.17650 | AACAUCCCACGCCTAGUCC | 224 |
| 39.22318 | CGGGCUGGGAGGACTUCAC | 225 |
| 39.69182 | GGGCTTACGUCTAAGATTTCTTUGT | 226 |
| 40.10525 | CAUGGTCUAAGAGGCAGCCA | 227 |
| 40.65199 | TAATTTTGACATGGUTGGGACTCTUGA | 228 |
| 41.2301 | GATGATGTUCTCCAGGUCGAAAGG | 229 |
| 43.15757 | TCUCUGCAGGGCAAACCT | 230 |
| 44.6908 | TCCTGTGTCUGGCCCCCTUA | 231 |
| 45.72120 | GACTTTTCACCUCTGATTTCTGGTTTUT | 232 |
| 47.759 | CGTGGCUCGGGACATUGC | 233 |
| 48.24545 | CTCTCTGCUCTGCAGCAAATUCA | 234 |
| 49.3900 | AACAGGACGAACUGGATTUCCT | 235 |
| 50.8773 | AATAUCCCCCGGCTTGUGAGT | 236 |
| 52.679 | ATGTGAACATUCTGCTTTTCAUGGG | 237 |
| 54.18517 | TATCCACATCCUCTTCCTCAGGAUT | 238 |
| 55.43552 | TTCATCTUGAAGAAGTTGAUGGAGG | 239 |
| 55.767 | TGCATTTTAGAAUAGGATATTGTATCAUACCAA | 240 |
| 56.25166 | AAGCATCAGCATUTGACTTTACCTTAUC | 241 |
| 57.24859 | GGATTGGAACAAGGUACTCTTUGAG | 242 |
| 58.8828 | TTTCCAGATACUAGAGTGTCTGTGTAAUCA | 243 |
| 59.18973 | AAGAAAAAGAAACAGAGAAUCTCCATTTUAGC | 244 |
| 60.28665 | TGCAGUGAAAAGAGTCUCAAACACAA | 245 |
| 61.4724 | TGACGATCUCCAATTCCCAAAAUGAAG | 246 |
| 62.14162 | TCATGAAATACUCCAAAGCCTCTUGC | 247 |
| 63.4977 | GGGCTGUGCGTCACTGUA | 248 |
| 65.24277 | GGAGGGUGUGGGAAGGCG | 249 |
| 66.11768 | GGAAACTCCCAUCTTGAGTCAUAAGG | 250 |
| 68.19575 | TTAGAGATTAAAGUGAAGGAGGAUGAGC | 251 |
| 69.20842 | CTCAGTTCCUGGACAAAAATACCAAUCT | 252 |
| 71.122176 | GCAGGCTCCAAGUAGATTCACAAUA | 253 |
| 71.96540 | GGAAACTCCCAUTTGTGATCAUAAGGAA | 254 |
| 72.2307 | ATCATTCTTGAUGTCTCTGGCUAGACC | 255 |
| 73.6720 | TTGTCTGCTGAAUGAACCCCAAUAC | 256 |
| 75.5088 | AAGTGGCTTUGGTCCGTCUC | 257 |
| 76.27276 | ATTTGCATCATAGUTAGATAAGACTGCUAAGG | 258 |
| 78.27403 | CAGAAAGCGGUGACTTACUGCA | 259 |
| 80.2024 | TGUGCCAGGGACCTUACCT | 260 |
| 81.9975 | CTCACATCGAGGAUTTCCTTGTUGG | 261 |
| 82.11913 | GTGTUCCCGGACATAGUCCAG | 262 |
| 83.29222 | CAGGAAAATGCUGGCTGACCUAAA | 263 |
| 84.23210 | CACAACCCACUGAGGTATATGTAUAGG | 264 |
| 85.11502 | AAAATGCCACTUACTGTTCAAGGATUT | 265 |
| 86.2729 | AGUTTGCAGACTTUCCAAAGCCAT | 266 |
| 88.12504 | ATTTTGTUGCCCAACTGGUCCT | 267 |
| 90.5484 | ACCAGGUACGCCTCCAGAUG | 268 |
| 91.4096 | GTTTTCCTTTACTTACUACACCTCAGATATAUT | 269 |
| 92.17956 | GGGAGATTCCUGATGGGCAGATUA | 270 |
| 94.1226 | AAGTTTGTAAGTAGUGCTATATTTATGTUGACT | 271 |
| 96.21382 | GGGCATTCCCUGCACTTCUA | 272 |
| 02.25456 | TTCCTTCUAAAAGGCCATGAAGATCUG | 273 |
| 03.22932 | AACCCACATTTCCUTTATAGATGTTTCTUC | 274 |
| 05.8811 | CTGTTGGUCACAGCTCCAGUG | 275 |
| 07.15610 | AGGATAAAGACCUGGTCCAUGGAAAT | 276 |
| 09.10389 | AGACAGGUCUCCCACAAACAC | 277 |
| 10.14770 | ATATATTCCCGTTUTTAGGGAGCAGAUT | 278 |
| 11.23267 | AACAAGTGGTTAUAGATGGTGAAACCUG | 279 |
| 12.11818 | ATTAACCCUGATTACTGGTTUCCAACA | 280 |
| 14.2747 | GATGTGCTGTUGAGACCTCTGUG | 281 |
| 15.10751 | TGAGGAGAUGGGTGGCTUGT | 282 |
| 16.21992 | CTGCTUCAGGACGTTGAACUCT | 283 |
| 17.5475 | CACCTGGCUCCTCTUCACG | 284 |
| 20.15351 | AGAAAGACCTTUCTGATCTGGTGUCA | 285 |
| 21.9067 | GCAATCUAGCGCCUGGAAGAG | 286 |
| 23.3877 | GTCACUGACAGCCCTCUGGA | 287 |
| 24.15926 | TGAGGAGCGAUGACGGAATAUAAG | 288 |
| 24.16763 | TGTCCTCCUGCAGGATTCCUAC | 289 |
| 25.1913 | CCAGAGAACAAATUAAAAGAGTTAAGGACUC | 290 |
| 26.7457 | CTGTGTTUCTCCCTTCUCAGGA | 291 |
| 27.8414 | ATTATAAGGCCUGCTGAAAATGACUGA | 292 |
| 29.26684 | TAGTTCAACAUGACGAAGAUGGCAA | 293 |
| 30.27449 | AAAGGGTACAUACAAAGCAGTCTGUG | 294 |
| 31.13656 | CATCAATGTUGCTCGAGUCCAC | 295 |
| 32.2472 | ATTGTAGGGTCUCCCTTGATCUGAG | 296 |
| 34.13971 | CTGATCTTCTCAAAGUCGTCATCCTUC | 297 |
| 35.7365 | AGATACTGAUCTCGCCAUCGCT | 298 |
| 37.28991 | GCTGCAGUGGGACCACTATTAUC | 299 |
| 38.15804 | CACTTCUCACACCGCTGTGUT | 300 |
| 39.36690 | CTTGCCCCUCCCAUCAGAAC | 301 |
| 39.75561 | CUCCCCAAACCCCAAUGAAGA | 302 |
| 40.10525 | CAUGGTCUAAGAGGCAGCCA | 303 |
| 40.2682 | CATCACACACCAUAACUCCACACAT | 304 |
| 42.11359 | AACATGATGGAUGTCACGTTCUCAAA | 305 |
| 43.15757 | TCUCUGCAGGGCAAACCT | 306 |
| 45.14205 | CTCACUGGCCTCTTCUGCT | 307 |
| 46.11706 | CCCUGGAAGAGUGGCCAAGAT | 308 |
| 48.20498 | GCTGCTGAAAAUGTAACTTTGTAUCCT | 309 |
| 49.13870 | TTGACTCTGTCUCCTCTTGTCTTCUC | 310 |
| 49.8398 | CGGACTCTGUAGGCTGCAGUT | 311 |
| 51.18783 | TGCAGTTGGUGGAACCATTAACUC | 312 |
| 53.9888 | CTTTAACACCUCCAGTCCCTCAUC | 313 |
| 54.18517 | TATCCACATCCUCTTCCTCAGGAUT | 314 |
| 55.5844 | CGGTTGCCTACUGGTTCAATTACTTUT | 315 |
| 56.25166 | AAGCATCAGCATUTGACTTTACCTTAUC | 316 |
| 57.13656 | ACTGCTAAACACTAAUATAACCTTTGGAAAUAT | 317 |
| 58.279 | ACTTTATTTGGAUTTGATCCAGUAACACC | 318 |
| 59.18973 | AAGAAAAAGAAACAGAGAAUCTCCATTTUAGC | 319 |
| 60.28665 | TGCAGUGAAAAGAGTCUCAAACACAA | 320 |
| 62.26615 | AGTTCAATGCAUGCTGTTTAATTGTGUG | 321 |
| 63.4977 | GGGCTGUGCGTCACTGUA | 322 |
| 64.5856 | GAGCCCAGGCCUTTCTUGG | 323 |
| 65.8114 | CATGCGGUGGCCCUCCT | 324 |
| 67.1666 | ACAACCACAUGTGTCCAGUGAAAAT | 325 |
| 68.19575 | TTAGAGATTAAAGUGAAGGAGGAUGAGC | 326 |
| 70.2019 | GATATGGUAGACAGAGCCTAAACAUCC | 327 |
| 71.8138 | TGTTTTGATAACCUGACAGACAAUAAAAGG | 328 |
| 71.9091 | ACATGGAAAGCCCCUGTTTCAUAC | 329 |
| 72.26276 | CTTUGCAGGACTGUCAAGCAG | 330 |
| 74.10774 | AUTTTACAGTACTCTUCCAACCCAAGA | 331 |
| 75.5088 | AAGTGGCTTUGGTCCGTCUC | 332 |
| 77.4991 | GCGGGACAAGGAUGCCUGAC | 333 |
| 79.11036 | CGTAGGUGCAGTTTGGAUGGC | 334 |
| 80.2024 | TGUGCCAGGGACCTUACCT | 335 |
| 82.11913 | GTGTUCCCGGACATAGUCCAG | 336 |
| 83.29222 | CAGGAAAATGCUGGCTGACCUAAA | 337 |
| 84.23210 | CACAACCCACUGAGGTATATGTAUAGG | 338 |
| 86.4292 | TAAAAGAGGAGAAACUCAGAGATAACCAAUAC | 339 |
| 87.13668 | TCCCTGTACCUTCACCTCUGG | 340 |
| 89.6924 | TCACAGCACAUAGUCCCGGAA | 341 |
| 91.4096 | GTTTTCCTTTACTTACUACACCTCAGATATAUT | 342 |
| 92.17956 | GGGAGATTCCUGATGGGCAGATUA | 343 |
| 93.19150 | CAGATGCTCUGAGAAAGGCATUAGAAA | 344 |
| 95.2994 | CTCCTCTAGCUATCTTAATGACTUGGAC | 345 |
| 97.6706 | ATCTCUGCCATCATTUCCGGAAAG | 346 |

| **4C** | **Blocking Primer** | **SEQ ID NO** |
|---|---|---|
| 01.10023 | CAGCCTCCAGTTCAGC | 347 |
| 03.29476 | CACAGCAACACCCTGGT | 348 |
| 04.7199 | CGTCCAGGGCTTCTTCC | 349 |
| 06.3669 | GCCTCGAGGCAGCG | 350 |
| 08.21927 | TCTCACCAAGTAGCTCAGGG | 351 |
| 10.40643 | CCATTCATTGAAACCTCAGCC | 352 |
| 11.23267 | CATACTGGATACAGCTGGACAAGA | 353 |
| 12.11818 | TGCGCTTTTCCCAACACCA | 354 |
| 13.3146 | CCATGTACAAGATCAATTACAGTCGGG | 355 |
| 14.2747 | GGAGGAGAAGTGCTTCTGC | 356 |
| 15.10751 | CCACTGTGCGACGAGCT | 357 |
| 17.5475 | CCAGAAACATCCTGGTAGCT | 358 |
| 18.14007 | GGGATTCAATTGCCATCCAT | 359 |
| 19.25590 | CCATCGACTTACATTGGTGGTCTT | 360 |
| 21.9067 | GCGATTAAGAAGACCCCTATGCAGTA | 361 |
| 22.2410 | ACCGGCGGCCTAGA | 362 |
| 23.3877 | GGCCGGTGAGGCGATC | 363 |
| 24.15926 | CGCCGGCGGTGTG | 364 |
| 25.29220 | CCTGTCTTGTCTTTGCTGATG | 365 |
| 26.7457 | CACTGTACTCCTCTTGACCT | 366 |
| 27.8414 | TAGTTGGAGCTGGTGGCG | 367 |
| 28.5397 | TGAGGTGGTGATGGGGAA | 368 |
| 29.26684 | TCCTCGTGGCCATGAATGA | 369 |
| 31.17423 | TGACACAGGATGTTTGATCCACC | 370 |
| 33.12700 | CGCACGTCTGTAGGGGAGT | 371 |
| 34.13971 | CTTCTGGGTAAGAAAGGCCT | 372 |
| 36.25412 | TCAACTCCCGTGGGGAGAT | 373 |
| 37.17650 | CCCATCACCATTGGCAGGC | 374 |
| 39.22318 | CTCATCAAGCGACGGC | 375 |
| 39.69182 | GGGATGTGTTTTCCCTCAACA | 376 |
| 40.65199 | GCCAAGTCCCTGTGTACGA | 377 |
| 41.2301 | GGTGGGCACGCGGA | 378 |
| 43.15757 | CCTTGGCTTTCTGGGTGAG | 379 |
| 44.6908 | GCCCCCTCCCGAGC | 380 |
| 45.72120 | GATGATGTGGGTGGGCTGT | 381 |
| 47.759 | AAACCACTTCATCCACCGG | 382 |
| 48.24545 | CCGCCATGAGCTCCAGC | 383 |
| 49.3900 | TGGAAGCCCTGATCATCA | 384 |
| 50.8773 | CTATCATCATAGGTCGTCATGCT | 385 |
| 52.679 | CTGGCAATTGTGACCCAGT | 386 |
| 54.18517 | AGCAACAGTCTTACCTGGAC | 387 |
| 55.43552 | GTGACTTTAGAATGCCTCCG | 388 |
| 57.24859 | CATAAGGGTTCTCCTCCATG | 389 |
| 58.8828 | CCATGCCAATGGACAGTGT | 390 |
| 60.28665 | GGATGTATTTGAAGCACCTGA | 391 |
| 61.4724 | CTACACAGTATCCAGCACATGA | 392 |
| 62.14162 | CAGTTTTATCTAAGGCTAGGGTCT | 393 |
| 63.4977 | CCCCACAGAGCGCTCCC | 394 |
| 66.11768 | TGAAATTCGCTGGAGGGTCA | 395 |
| 68.19575 | GGCCTGGCCAGAGACA | 396 |
| 69.20842 | CCAGAAATCCTGACTTACGACA | 397 |
| 71.122176 | CAAGGTAACTCAGGACTTTGAG | 398 |
| 71.96540 | CCCATGTATGAAGTACAGTGGA | 399 |
| 72.2307 | AGACTTGGCAGCCAGAAA | 400 |
| 73.6720 | TGGAACTGATGGAGGGAGGA | 401 |
| 75.5088 | CGTGGTGCCCCTCTAT | 402 |
| 76.27276 | GAATTTTCGTAGTACATATTTCCTCTGA | 403 |
| 78.27403 | AGCTGTTTTCACCTCTGTTGCTT | 404 |
| 80.2024 | TCTTGAGGATCTTGAAGGAAACTG | 405 |
| 81.9975 | GAGATGTTGCTTCTCTTAATTCCTTG | 406 |
| 82.11913 | TGATGGCCAGCGTGG | 407 |
| 83.29222 | ACAGATTTTGGGCTGGCCAA | 408 |
| 85.11502 | GGCATTTTGGTTGTGTATATCATG | 409 |
| 86.2729 | TCTTGCCAGAGACATGTATGA | 410 |
| 88.12504 | CCCAATCGGCCTGGTGC | 411 |
| 90.5484 | GCCTTGGTCCAGACCCAG | 412 |
| 91.4096 | CCACTCCATCGAGATTTCACTG | 413 |
| 92.17956 | GGACAAAGAATTGGATCTGGATCATTTGGA | 414 |
| 94.1226 | ATTTTCTTCTCTCTGACCTTTGG | 415 |
| 96.21382 | CTAGGCACTTACTAATGCTGAAGA | 416 |
| 02.25456 | TGAGGTTTTTCCGAAGAGATGT | 417 |
| 03.22932 | CCCTCATCTACCCACTGACA | 418 |
| 05.8811 | TGTAGTGTAGCACAGCTTCGAAGT | 419 |
| 07.15610 | CTCCTCCTGGCCCGTG | 420 |
| 09.10389 | GTACCTCTATGGCGTCTGTGTC | 421 |
| 10.14770 | CCTTGTTGGCAAATCACACTTGTT | 422 |
| 11.23267 | TGTTGGACATACTGGATACAGC | 423 |
| 12.11818 | CTTTTCCCAACACCACCTGC | 424 |
| 14.2747 | CCTGAGGAGGAGAAGTGC | 425 |
| 15.10751 | CGAGCTGTGCCGCA | 426 |
| 17.5475 | CCTCTTCACGTAGGAATCCTCTTCATAA | 427 |
| 20.15351 | TGCAGGCTCCAAGAAGATTT | 428 |
| 21.9067 | AGATTACAGCTTCCCCAGACTAC | 429 |
| 23.3877 | GGGCCGGTGAGGCG | 430 |
| 24.15926 | GTGGGCGCCGGC | 431 |
| 24.16763 | TGGCGCTGTACTCCTCCTG | 432 |
| 25.1913 | ATGTACCTATGGTCCTAGTAGGAAATAAATG | 433 |
| 26.7457 | CTGTACTCCTCTTGACCTGCTGT | 434 |
| 27.8414 | TGGTAGTTGGAGCTGGTG | 435 |
| 29.26684 | CCCTCGCACCACGC | 436 |
| 30.27449 | GCAGCCCCCGGCAC | 437 |
| 31.13656 | AGCCTGTTCCCTCACAGG | 438 |
| 34.13971 | CAGTTCTCCCACCTTCTGCTT | 439 |
| 35.7365 | GCCCACGATGTACGGAGAG | 440 |
| 37.28991 | CCCCAGGATGTTCCGGATA | 441 |
| 38.15804 | GGACGTGGGATCCTGC | 442 |
| 39.36690 | GGCGCTTTTGGCACAGT | 443 |
| 39.75561 | CCCAAAGCCAACAAAGAAATCTTAGA | 444 |
| 40.10525 | GGTGTGGGCTCCCCATATGT | 445 |
| 40.2682 | CCGCCGGCGGAGAA | 446 |
| 42.11359 | GCTCCGACCGCTGG | 447 |
| 43.15757 | CTGGGTGAGAAAGGCTTC | 448 |
| 45.14205 | GCATGAGAACCTGGGCC | 449 |
| 46.11706 | ACTTCGGGATGGCCCGAG | 450 |
| 48.20498 | CGAACAATGTTCTGGTGGTTGAATT | 451 |
| 49.13870 | GCGCCTTTGGGGAGG | 452 |
| 49.8398 | CACTTCAGGCAGCGTCT | 453 |
| 51.18783 | CACCCAATCAAGCTCAACTTCGTA | 454 |
| 53.9888 | CCAGAGGCAGAGGTCGA | 455 |
| 54.18517 | TGCCTTTACCACTCAGAGAAG | 456 |
| 55.5844 | TTTAAAAAGGGTTGAAAAAGCCGAA | 457 |
| 56.25166 | TACCTTATCAATGTCTCGAATATTTACATT | 458 |
| 57.13656 | CCTGGGATTGGAACAAGGTA | 459 |
| 58.279 | CCAATAGGGTTCAGCAAATCTTCT | 460 |
| 59.18973 | TCCATAGAAAATCTTTCTCCTGC | 461 |
| 60.28665 | GAGTCACACACCTTTTGTGTTTC | 462 |
| 62.26615 | ATTTCATGAAACAAATGAATGATGCACATC | 463 |
| 63.4977 | CCCCCACAGAGCGCTCCC | 464 |
| 64.5856 | GGCGGGCAGTGTGTATG | 465 |
| 65.8114 | CCACAGGGATGCCGGGG | 466 |
| 67.1666 | GGGCCACATTTGAACATTGTAAAC | 467 |
| 68.19575 | GCCTGGCCAGAGACATCAT | 468 |
| 70.2019 | TGGGCAAGACTTCTGCCTATTT | 469 |
| 71.8138 | CCTTGGTAATCACATGAATATTGTGAAT | 470 |
| 71.9091 | AGACCCAACACAACTTCCT | 471 |
| 72.26276 | ATTTTGGTCTAGCCAGAGACA | 472 |
| 74.10774 | GGCTCCACTTCCCAGCAA | 473 |
| 75.5088 | TGCCCCTCTATGACCTGC | 474 |
| 77.4991 | CAGCTTTGGTGCCACCT | 475 |
| 79.11036 | TGCCCGGCAGGAGTC | 476 |
| 80.2024 | CGCACCGGAGCCCAG | 477 |
| 82.11913 | GCATGAGCTGCGTGAT | 478 |
| 83.29222 | CTGGCCAAACTGCTGGG | 479 |
| 84.23210 | TCTCAGAACAATAAACTGAAATATACCTTC | 480 |
| 86.4292 | CAGTGAAGTGGATGGCT | 481 |
| 87.13668 | ATGGTGCCCGCCGA | 482 |
| 89.6924 | TCAGCCTGGTTGAAGAAGTCGT | 483 |
| 91.4096 | CCTCACAGTAAAAATAGGTGATTTT | 484 |
| 92.17956 | GGGACAAAGAATTGGATCTGGAT | 485 |
| 93.19150 | TTACTCTCGTCTCCACAGACACA | 486 |
| 95.2994 | TGGTGGGGACTCGAACT | 487 |
| 97.6706 | GAGAGCTGCATCAGTTCACT | 488 |

Example 3: We tested the impact of blocking primers on fusion detection in FFPE samples using Ion AmpliSeq library preparation. As above, blocking primers are not processed during the Ion AmpliSeq library preparation workflow, and do not produce amplicons capable of sequencing. We hypothesized that addition of blocking primers to suppress wild-type transcripts would result in better reproducibility and increased sensitivity of fusion detection. To test this, a modified version of the Oncomine Focus RNA Assay was prepared by supplementing with 7 control detection primer pairs.

**Table 5: 7 GEX Ion AmpliSeq Fusion Primer Sets**

| **Target** | **Forward Primer (U)** | **SEQ ID NO** |
|---|---|---|
| CHMP2A.E2E3.110 | AGACAUTAAGAAGAUGGCCAAGCAA | 489 |
| FBXW2.E3E4.110 | ATTAATUGGACACAGUGCCAGAGT | 490 |
| GUSB.E10E11.110 | GCCCATTATUCAGAGCGAGTAUGG | 491 |
| JUN.E1.110 | GAGCUGGAGCGCCTGAUAAT | 492 |
| PSMB2.E1E2.110 | CTATGTTCUTGTCGCCUCCGA | 493 |
| SNRPD3.E2E3.110 | GGAAGCUCATUGAAGCAGAGGA | 494 |
| VCP.E13E14.110 | GCTCACCAUGTGGTTUGGG | 495 |

| **Target** | **Reverse Primer (U)** | |
|---|---|---|
| CHMP2A.E2E3.110 | TGGCCCGCAUCAAUACAAACT | 503 |
| FBXW2.E3E4.110 | ACTGCCCUGTGCTCACAUC | 504 |
| GUSB.E10E11.110 | GATGGTACTGCUCTAGCAGACTTUT | 505 |
| JUN.E1.110 | CGAAGCCCUCCTGCTCAUC | 506 |
| PSMB2.E1E2.110 | CCTCUCCAACACACAGGAGUAAT | 507 |
| SNRPD3.E2E3.110 | GCUGCCACGGATGTAUACCT | 508 |
| VCP.E13E14.110 | TGGCAAUCGAATCCAGCUCAT | 509 |

**Table 6: 7 GEX Ion AmpliSeq blocker primer pairs**

| **Target** | **Forward Primer** | **SEQ ID NO** |
|---|---|---|
| CHMP2A.E2E3.110BL | AGACATTAAGAAGATGGCCAAGCAA | 496 |
| FBXW2.E3E4.110BL | ATTAATTGGACACAGTGCCAGAGT | 497 |
| GUSB.E10E11.110BL | GCCCATTATTCAGAGCGAGTATGG | 498 |
| JUN.E1.110BL | GAGCTGGAGCGCCTGATAAT | 499 |
| PSMB2.E1E2.110BL | CTATGTTCTTGTCGCCTCCGA | 500 |
| SNRPD3.E2E3.110BL | GGAAGCTCATTGAAGCAGAGGA | 501 |
| VCP.E13E14.110BL | GCTCACCATGTGGTTTGGG | 502 |

| **Target** | **Reverse Primer** | **SEQ ID NO** |
|---|---|---|
| CHMP2A.E2E3.110BL | TGGCCCGCATCAATACAAACT | 510 |
| FBXW2.E3E4.110BL | ACTGCCCTGTGCTCACATC | 511 |
| GUSB.E10E11.110BL | GATGGTACTGCTCTAGCAGACTTTT | 512 |
| JUN.E1.110BL | CGAAGCCCTCCTGCTCATC | 513 |
| PSMB2.E1E2.110BL | CCTCTCCAACACACAGGAGTAAT | 514 |
| SNRPD3.E2E3.110BL | GCTGCCACGGATGTATACCT | 515 |
| VCP.E13E14.110BL | TGGCAATCGAATCCAGCTCAT | 516 |

### Library Prep

Libraries were manually prepared, n=4 according to the Ion AmpliSeq Library Prep User Manual using FFPE RNA samples (BON1027, Fusion Negative and BON1020, CD74-ROS1; BioChain Institute).

Panels: Libraries were constructed using standard Oncomine Focus Assay v2 library reaction supplemented with GEX primer pairs or Oncomine Focus Assay v2 reaction supplemented with GEX primer pairs as well as blocking primers added at 0%, 50%, 90% or 100% relative to standard primer amount per reaction in the presence of 10ng cDNA with 50nM each standard primer and amplified with 31 cycles according to the Ion AmpliSeq user guide. Templating was carried out according to Chef 540 Templating User Guide, and sequencing of 40 samples per chip (2 Chips) was carried out according to Ion Torrent S5 Sequencer User Guide. Results are depicted in FIG 5A-5D.

Competitor primers effectively blocked targeted wild type alleles. For standard Ion AmpliSeq ONCOMINE FOCUS ASSAY procedure, the seven target standard primer sets were responsible for 38.5% of the Total Mapped Reads. With 90% blocking primers for the seven GEX primer sets, the seven target primer sets are responsible for 0.7% of the Total Mapped Reads. These results indicate 37% additional bandwidth for the total mapped reads for other, less abundant targets.

Signals for the majority of mutant amplicons increased as the ratio of unmodified competitor primers increased. For example, detection of CD74-ROS1.C6R35 increased approximately two-fold with 90% competitor primers over standard primer set. Average yield for all libraries containing competitor primers was similar to libraries run with no competitor primers, and % Reads on Target comparable with typical FFPE RNA runs.

**TABLE 7: Fusion Detection in CD74-ROS1.C6R35 positive FFPE Sample:**

| | **No GEX** | **STD GEX** | **50% block GEX** | **90% block GEX** | **100% block GEX** |
|---|---|---|---|---|---|
| | 12339 | 7653 | 10319 | 7911 | 12225 |
| | 3771 | 8820 | 2763 | 11618 | 5895 |
| | 1827 | 7464 | 9575 | 18443 | 7425 |
| | 2396 | 4363 | 8770 | 15934 | 8016 |
| Avg= | 5083 | 7075 | 7857 | 13477 | 8390 |
| StDev= | 4906 | 1905 | 3454 | 4660 | 2708 |
| %CV= | 97 | 27 | 44 | 35 | 32 |

### Example 4. Blocking using Ion AmpliSeq HD Technology

To further explore the utility of blocking primers for Ion AmpliSeq HD technology, we redesigned the panel described in Example 2 to be compatible with Ion AmpliSeq HD technology, and suppressed wildtype amplicons using the same set of blocking primers. In this scenario, blocking primers similarly prevent downstream processing in library preparation, resulting in elimination of blocked wild type targets from sequencing. Blocked libraries were prepared by adding blocker to initial target amplification step at 2X concentration relative to standard Ion AmpliSeq HD panel primers and processed according to Ion AmpliSeq HD user guide (MAN0017392) and sequenced using and Ion 530 Chip (MAN0010846) according to manufacturer instructions. Thermo Fisher Scientific, Inc. Unblocked libraries resulted in even family representation across all amplicons; while the presence of blocker significantly suppressed wild-type amplicons though mutant alleles are detected were detected at high abundance. See FIGS. 6A-6B. Both family numbers and sizes of family are increased when targets are blocked. See FIGS 6C-6D. Thus, suppressing wild type amplicons using the provided blocking methods enables more even coverage and therefore improved detection of rare alleles.

### Example 5: Titration of Competitor Primers into SARS-CoV-2 Panel

### Materials and Methods

Competitor blocking primer spike-in primer sets specific for human control sequence amplicons were prepared and spiked into a standard Ion AmpliSeq SARS-CoV-2 panel preparation. Similar to methods described above, competitor primer sequences are not processed and cannot proceed through Ion AmpliSeq workflow to product amplicons for sequencing. Rather than a single competitor blocking primer, a primer pair was included for reduction of high levels of control sequences in a human sample, wherein viral sequences of primary interest may be present in significantly lower titer levels.

**TABLE 8: Competitor blocking primer sets for human control sequences**

| **Target** | **Forward Primer** | **SEQ ID NO** |
|---|---|---|
| HMBS | CCAGCTCCCTGCGAAGAG | 517 |
| ITGB7 | CCTATCTTGGATGATGGCTGGTGCAAAG | 518 |
| LRP1 | GACGGGTCCAACTACACGTT | 519 |
| MYC | CCTGGTGCTCCATGAGGAGACA | 520 |
| TBP | GACTCCCATGACCCCCATC | 521 |

| **Target** | **Reverse Primer** | |
|---|---|---|
| HMBS | AGGATGATGGCACTGAACTCC | 522 |
| ITGB7 | CGTGTGGTCTGCTCCCTTTTCTTG | 523 |
| LRP1 | GCACATTGCTCCCGTTAAGG | 524 |
| MYC | GTGATCCAGACTCTGACCTTTTGCCA | 525 |
| TBP | ACGAAGTGCAATGGTCTTTAGGT | 526 |

Titration of competition primers spiked-in at 0, 50, 100, 200, 400, 600, 800, and 1000 nM at final concentrations of 0X 1X, 2X, 4X, 6X, 8X, 10X, respectively. Library construction: 16 libraries, 8 different conditions were prepared according to the Ion AmpliSeq User Guide (Thermo Fisher Scientific).

Briefly, samples comprised Twist SARS-CoV-2 RNA control-2 and Human Lung Total RNA (Thermo Fisher Scientific). 20 copies Viral RNA and 5ng total lung RNA as background were used for sample input. As a no viral control: 5 ng human lung total RNA only with 10X spike-in was used. Reactions were carried out in duplicate. Reverse Transcription using SSIII and target amplification were carried out according to manufacturer instruction in the Ion Ampliseq Library Kit Plus User Guide using 25 cycles of target amplification. Following library preparation, libraries were quantified by qPCR, templating carried out on Ion Chef according to manufacturer instructions and libraries were sequenced using the Ion Torrent S5 on 530-chips.

Results are depicted in Figures 7A-7D. Competitor primer spike-in primers significantly reduced library yield, resulting in suppression of reads of highly abundant human control sequences. Overall uniformity of the sequencing output was improved without changing bed file results. For example, at 4X competitor spike-in, the uniformity reached about 85%.

With the same amount of input for templating and sequencing, competitor primer spike-in increased viral reads and mean depth detected. About 4X competitor spike achieved optimal results. No significant negative impact was observed on viral sequencing data for this low viral input (e.g., 20 copies).

### Example 6. Titration of Competitor Primers into SARS-CoV-2 Panel

### Materials and Methods

Competitor blocking control primer sets specific for human control sequence amplicons were included in two panel Ion AmpliSeq SARS-CoV-2 library preparations. Like methods described above, competitor primer sequences are not processed and cannot proceed through Ion AmpliSeq workflow to product amplicons for sequencing. As in the prior example, primer pairs were included for reduction of high levels of control sequences in a human sample, wherein viral sequences of primary interest may be present in significantly lower titer levels. Various primer pairs were generated to control targets, including small, medium, and long lengths:

**TABLE 9: Control primer sets for human control sequences, including blocker and detection pairs**

| **Target** | **Forward Primer** | **SEQ ID NO** | **length** |
|---|---|---|---|
| Ame | CAACTTCAGCTATGAGGTAATTTTTCTC | 527 | NA |
| Ame | CAACTTCAGCTAUGAGGTAATTTTTCUC | 528 | NA |
| HMBS | AGACCAGGAGTCAGACTGTAGG | 529 | long |
| HMBS | AGACCAGGAGUCAGACTGUAGG | 530 | long |
| HMBS | TCATCCTCAGGGCCATCTTCAT | 531 | medium |
| HMBS | TCATCCUCAGGGCCATCTUCAT | 532 | medium |
| HMBS | CCAGCTCCCTGCGAAGAG | 533 | short |
| HMBS | CCAGCUCCCUGCGAAGAG | 534 | short |
| ITGB7 | GTCCTTGTTTTGCTGCTGGT | 535 | long |
| ITGB7 | GTCCTTGUTTTGCTGCUGGT | 536 | long |
| ITGB7 | CACTTCAGACGACACATTCCATACA | 537 | medium |
| ITGB7 | CACTUCAGACGACACATTCCAUACA | 538 | medium |
| ITGB7 | CGTGTGGTCTGCTCCCTTTTCTTG | 539 | short |
| ITGB7 | CGTGTGGTCUGCTCCCTTTTCTUG | 540 | short |
| LRP1 | GCACAGCGGAACTCACTGG | 541 | long |
| LRP1 | GCACAGCGGAACUCACUGG | 542 | long |
| LRP1 | GAGGCAGCCTCCATGAGTG | 543 | medium |
| LRP1 | GAGGCAGCCUCCATGAGUG | 544 | medium |
| LRP1 | ATGGTGGATGTCGTGTAGCTT | 545 | short |
| LRP1 | ATGGTGGATGUCGTGTAGCUT | 546 | short |
| MYC | GTGATCCAGACTCTGACCTTTTGCCA | 547 | NA |
| MYC | GTGATCCAGACUCTGACCTTTUGCCA | 548 | NA |
| TBP | AAGGGTACATGAGAGCCATTACG | 549 | long |
| TBP | AAGGGTACAUGAGAGCCATUACG | 550 | long |
| TBP | GCGGAAGTGACATTATCAACGC | 551 | medium |
| TBP | GCGGAAGUGACATTAUCAACGC | 552 | medium |
| TBP | GACTCCCATGACCCCCATC | 553 | short |
| TBP | GACTCCCAUGACCCCCAUC | 554 | short |

| **Target** | **Reverse Primer** | | **length** |
|---|---|---|---|
| Ame | ATCAGAGCTTAAACTGGGAAGCTG | 555 | NA |
| Ame | ATCAGAGCTUAAACTGGGAAGCUG | 556 | NA |
| HMBS | AAGAATCTTGTCCCCTGTGGTG | 557 | long |
| HMBS | AAGAATCTTGUCCCCTGTGGUG | 558 | long |
| HMBS | ACGATCCCGAGACTCTGCTT | 559 | medium |
| HMBS | ACGATCCCGAGACUCTGCUT | 560 | medium |
| HMBS | AGGATGATGGCACTGAACTCC | 561 | short |
| HMBS | AGGATGAUGGCACTGAACUCC | 562 | short |
| ITGB7 | CTGGGTGGCACCCTCTC | 563 | long |
| ITGB7 | CTGGGUGGCACCCTCUC | 564 | long |
| ITGB7 | ACTGCAGACTTAGGAATCAGTTTACTC | 565 | medium |
| ITGB7 | ACTGCAGACTUAGGAATCAGTTTACUC | 566 | medium |
| ITGB7 | CCTATCTTGGATGATGGCTGGTGCAAAG | 567 | short |
| ITGB7 | CCTATCTTGGAUGATGGCTGGUGCAAAG | 568 | short |
| LRP1 | CTGTGTGACGGCAGCGAT | 569 | long |
| LRP1 | CUGTGUGACGGCAGCGAT | 570 | long |
| LRP1 | CCAGTCAGTCCAGTAGACCTC | 571 | medium |
| LRP1 | CCAGTCAGUCCAGTAGACCUC | 572 | medium |
| LRP1 | AATCGCATCTTCTTCAGCGACA | 573 | short |
| LRP1 | AATCGCAUCTTCTUCAGCGACA | 574 | short |
| MYC | CCTGGTGCTCCATGAGGAGACA | 575 | NA |
| MYC | CCTGGUGCTCCAUGAGGAGACA | 576 | NA |
| TBP | AAAATATGCTAGAGTTGTACAGAAGTTGG | 577 | long |
| TBP | AAAATATGCTAGAGUTGTACAGAAGTUGG | 578 | long |
| TBP | CGCTGGAACTCGTCTCACT | 579 | medium |
| TBP | CGCTGGAACUCGTCUCACT | 580 | medium |
| TBP | ACGAAGTGCAATGGTCTTTAGGT | 581 | short |
| TBP | ACGAAGUGCAATGGTCTTUAGGT | 582 | short |

Libraries were prepared as in Example 5. Samples comprised Twist SARS-CoV-2 RNA control-2 and Human Lung Total RNA (Thermo Fisher Scientific). Viral RNA was titrated in at various amounts 0, 50, 100, 200 copies, and 5ng total lung RNA as background were used for sample input. Various combinations of control primers and competitor primers with each panel were also assessed. Results are depicted in Figures 8A-8D. In all cases, competitor primers significantly reduced library yield, resulting in suppression of reads of highly abundant human control sequences over a range of viral inputs.

### Example 7. Titration of Competitor Primers into SARS-CoV-2 Panel

### Materials and Methods

Competitor blocking control primer sets specific for human control sequence amplicons were included in two panel Ion AmpliSeq SARS-CoV-2 library preparations. Like methods described above, competitor primer sequences are not processed and cannot proceed through Ion AmpliSeq workflow to product amplicons for sequencing. As in the prior examples, primer pairs were included for reduction of high levels of control sequences in a human sample, wherein viral sequences of primary interest may be present in significantly lower titer levels. Various primer pairs were generated to control targets, Ame, as well as short and medium length amplicons for HMBS and ITGB7:

**TABLE 10: Control primer sets for human control sequences, including blocker and detection pairs**

| **Target** | **Forward Primer** | **SEQ ID NO** | **length** |
|---|---|---|---|
| Ame | CAACTTCAGCTATGAGGTAATTTTTCTC | 583 | NA |
| Ame | CAACTTCAGCTAUGAGGTAATTTTTCUC | 584 | NA |
| HMBS | TCATCCTCAGGGCCATCTTCAT | 585 | medium |
| HMBS | TCATCCUCAGGGCCATCTUCAT | 586 | medium |
| HMBS | CCAGCTCCCTGCGAAGAG | 587 | short |
| HMBS | CCAGCUCCCUGCGAAGAG | 588 | short |
| ITGB7 | CACTTCAGACGACACATTCCATACA | 589 | medium |
| ITGB7 | CACTUCAGACGACACATTCCAUACA | 590 | medium |
| ITGB7 | CGTGTGGTCTGCTCCCTTTTCTTG | 591 | short |
| ITGB7 | CGTGTGGTCUGCTCCCTTTTCTUG | 592 | short |

| **Target** | **Reverse Primer** | **SEQ ID NO** | **length** |
|---|---|---|---|
| Ame | ATCAGAGCTTAAACTGGGAAGCTG | 593 | NA |
| Ame | ATCAGAGCTUAAACTGGGAAGCUG | 594 | NA |
| HMBS | ACGATCCCGAGACTCTGCTT | 595 | medium |
| HMBS | ACGATCCCGAGACUCTGCUT | 596 | medium |
| HMBS | AGGATGATGGCACTGAACTCC | 597 | short |
| HMBS | AGGATGAUGGCACTGAACUCC | 598 | short |
| ITGB7 | ACTGCAGACTTAGGAATCAGTTTACTC | 599 | medium |
| ITGB7 | ACTGCAGACTUAGGAATCAGTTTACUC | 600 | medium |
| ITGB7 | CCTATCTTGGATGATGGCTGGTGCAAAG | 601 | short |
| ITGB7 | CCTATCTTGGAUGATGGCTGGUGCAAAG | 602 | short |

The competitor primer pair was included at 2x the Ion AmpliSeq primer detection set for Ame; and 1x the Ion AmpliSeq primer detection set for expression controls HMBS and ITGB7.

Libraries were prepared as in Example 6. Reactions were carried out in duplicate. Reverse Transcription using the Ion Torrent NGS Reverse Transcription Kit according to manufacturer instructions, and target amplification were carried out according to manufacturer instruction in the Ion Ampliseq Library Kit Plus User Guide using cycles of target amplification according to the following Table 11:

**TABLE 11: Target Amplification Cycle Optimization**

| est copies | bin | #Cycles | |
|---|---|---|---|
| | | Manual prep | DL8 prep |
| 50-1500 | Low input | 26 | 27 |
| 1500-50000 | Med input | 20 | 21 |
| 50000-1500000 | High input | 15 | 16 |

Samples comprised Acrometrix 122010 or ATCC extracted MAR2021 titrated in at various amounts 0, 50, 100 copies, or Twist SARS-CoV-2 RNA 1, 9, 12 and 14 at 1000 copies; and Human Lung Total RNA (Thermo Fisher Scientific). 5ng total lung RNA as background were used for sample input. In all cases, competitor primers significantly reduced library yield, resulting in suppression of reads of highly abundant human control sequences over a range of viral inputs. Following library preparation, libraries were quantified by qPCR, templating carried out on Ion Chef according to manufacturer instructions and libraries were sequenced using the Ion Torrent S5 on 530-chips.

Results yielded consistent uniformity, high base coverage, mapped reads (all mapping to reference genome) and depth of coverage across samples (e.g., above 1000 copies); and the majority of amplicons generated correspond to viral amplicons, whereas a comparator panel (SARS-CoV2 described in Example 5 above) lacking competitor primers and prepared in parallel, resulted in dominant control amplicons in the parallel library preparation. Significantly higher read depth and least 99% of viral base coverage was achieved from the panel having the three control and competitor primers present, while the comparator panel lacking competitor primers yielded libraries dominated by control amplicons and yielded less efficient sequencing results for detection of low frequency virus in samples.

Libraries were prepared as described above using the same panel with controls in conjunction with clinical samples containing various levels of virus as previously determined by concordant methods. Results yielded consistent uniformity, high base coverage, mapped reads and depth of coverage across medium and high input samples (e.g., above 1000 copies); whereas samples with low virus (<1000 copies), including very low virus level yielded efficient detection of virus and mapped reads, though often generated inconsistent base coverage, read depth and/or uniformity, particularly for samples with little to no virus, or very poor quality samples. Consistent with these results, in samples with little to no virus or very poor quality samples, controls sequences were prevalent in sequencing data from libraries (data not shown).

While preferred embodiments of the present invention have been shown and described herein, it will be obvious to those skilled in the art that such embodiments are provided by way of example only.

## Claims

1. A method for preparing a library of one or more target nucleic acid sequences present in a sample at low frequency comprising:
amplifying within a single amplification reaction mixture a multiplex of different target sequences from a sample including a plurality of different target sequences,
wherein the amplifying includes contacting at least a portion of the sample with a plurality of target-specific primers, and a polymerase under amplification conditions, thereby producing a multiplex of different amplified target sequences,
wherein at least two of the plurality of target-specific primers include cleavable groups and at least one of the produced multiplex of different amplified target sequences include cleavable groups at both ends of resulting amplicon, and wherein at least one of the plurality of target specific primers is a blocking primer that does not include the cleavable groups and at least one of the produced multiplex of different amplified target sequences includes a cleavable group at only one end of resulting amplicon;
cleaving the cleavable groups of the multiplex of different amplified target sequences and forming cleaved ends; and
ligating at least two adapters to cleaved ends of at least one of the multiplex of different amplified target sequences, thereby producing one or more adapter-ligated amplified target sequences;
thereby preparing a library of different adapter-ligated target sequences,
wherein amplicon ends resulting from blocking primers are not capable of ligating adapter, and
wherein none of the adapters in the ligation reaction hybridizes under high stringency conditions to any one of the multiplex of different amplified target sequences.

2. The method of claim 1 wherein the nucleic acid target sequences are selected from one or more of RNA, DNA, cDNA, TNA, cfNA, ctNA, and tissue nucleic acid from cells.

3. The method of claim 1 wherein one or more of the nucleic acids target sequences is one or more pathogenic nucleic acid to the host sample, selected from viral, bacterial and microbe nucleic acid.

4. The method of claim 1 wherein one or more of the blocking primers is directed to wild type sequence or allele specific sequences native to the host sample.

5. The method of claim 1, wherein the cleavable group includes methylguanine, 8-oxo-guanine, xanthine, hypoxanthine, 5,6-dihydrouracil, uracil, 5-methylcytosine, thymine-dimer, 7-methylguanosine, 8-oxo-deoxyguanosine, xanthosine, inosine, dihydrouridine, bromodeoxyuridine, uridine or 5-methylcytidine.

6. The method of claim 1, wherein each of the plurality of target-specific primers has any one or more of the following criteria: (1) includes two or more modified nucleotides within the primer sequence, at least one of which is included near or at the termini of the primer and at least one of which is included at, or about the center nucleotide position of the primer sequence; (2) length is about 15 to about 40 bases in length; (3) Tₘ is from about 60°C to about 70°C; (4) has low cross-reactivity with non-target sequences present in the sample; (5) at least the first four nucleotides (going from 3' to 5' direction) are non-complementary to any sequence within any other primer present in the reaction; and (6) are non-complementary to any consecutive stretch of at least 5 nucleotides within any other produced amplified target sequence.

7. The method of claim 1, wherein the steps of amplifying, cleaving, ligating and preparing the library are carried out in a single reaction vessel, optionally further comprising combining two or more libraries each separately prepared in a single reaction vessel, thereby preparing a library of different adapter-ligated target sequences.

8. The method of claim 1, wherein the adapters further include one or more of a barcode, tag and/or universal priming sequence.

9. The method of claim 1, wherein the at least one of the plurality of target specific primers is a blocking primer and wherein the at least one of the produced multiplex of different amplified target sequences that includes at most a cleavable group at only one end of resulting amplicon is not processed through the remainder of the workflow and does not produce a productive adapter ligated amplicon; and
wherein the at least one blocking primer comprises a DNA sequence complementary to the target sequence downstream of either a productive forward target specific primer or a productive reverse target specific primer in the multiplex reaction, or
wherein the at least one blocking primer comprises a DNA sequence complementary to the target sequence that overlaps in part or in full with a productive forward target specific primer or a productive reverse target specific primer in the multiplex reaction.

10. A method for preparing a library of one or more nucleic acid target sequences present in a sample at low frequency comprising:
amplifying within a single amplification reaction mixture a multiplex of different target sequences from a sample including a plurality of different target sequences,
wherein the amplifying includes contacting at least a portion of the sample with a plurality of adapters capable of amplification of one or more nucleic acid target sequences in the sample under conditions wherein the nucleic acid target sequences undergo a first amplification, wherein at least two of the plurality of adapters comprise a universal handle sequence and a target nucleic acid sequence and a cleavable moiety and optionally one or more tag sequences wherein the target nucleic acid sequence of the adapter includes at least one cleavable moiety and the universal handle sequence does not include the cleavable moiety and at least one of the produced multiplex of different amplified target sequences include cleavable groups at both ends of resulting amplicon, and
wherein at least one of the plurality of adapters is a blocking primer that does not include the cleavable moiety or universal handle sequence and at least one of the produced multiplex of different amplified target sequences includes a cleavable group and universal handle sequence at no more than one end of resulting amplicon;
digesting resulting first amplification products to reduce or eliminate resulting primer dimers and prepare partially digested target amplicons, producing gapped, double stranded amplicons, then repairing the partially digested target amplicons; and
amplifying the repaired target amplicons in a second amplification using universal primers, thereby producing one or more adapter- amplified target sequences;
thereby preparing a library of different adapter-amplified target sequences, wherein amplicons resulting from blocking primer are not compatible with second universal primer amplification and
wherein none of the adapters in the amplification reaction hybridizes under high stringency conditions to any one of the multiplex of different amplified target sequences.

11. The method of claim 10 wherein the nucleic acid target sequences are selected from one or more of RNA, DNA, cDNA, TNA, cfNA, ctNA, and tissue nucleic acid from cells.

12. The method of claim 10 wherein one or more of the target nucleic acids is a pathogenic nucleic acid to the host sample selected from viral, bacterial and microbe nucleic acid.

13. The method of claim 10 wherein one or more blocking primers is directed to wild type sequence or allele specific sequence native to the host sample.

14. The method of claim 10, wherein the cleavable group includes methylguanine, 8-oxo-guanine, xanthine, hypoxanthine, 5,6-dihydrouracil, uracil, 5-methylcytosine, thymine-dimer, 7-methylguanosine, 8-oxo-deoxyguanosine, xanthosine, inosine, dihydrouridine, bromodeoxyuridine, uridine or 5-methylcytidine.

15. The method of claim 10, wherein each of the plurality of adapters has any one or more of the following criteria: (1) includes two or more modified nucleotides within the primer sequence, at least one of which is included near or at the termini of the primer and at least one of which is included at, or about the center nucleotide position of the primer sequence; (2) length is about 15 to about 40 bases in length; (3) Tₘ is from about 60°C to about 70°C; (4) has low cross-reactivity with non-target sequences present in the sample; (5) at least the first four nucleotides (going from 3' to 5' direction) are non-complementary to any sequence within any other primer present in the reaction; and (6) are non-complementary to any consecutive stretch of at least 5 nucleotides within any other produced amplified target sequence.

16. The method of claim 10, wherein the steps of amplifying, digesting, repairing, producing and preparing the library are carried out in a single reaction vessel, optionally further comprising combining two or more libraries each separately prepared in a single reaction vessel, thereby preparing a library of different adapter-amplified target sequences.

17. The method of claim 10, wherein the adapters and/or the universal primers further include one or more of a barcode, tag or universal priming sequence.

18. The method of claim 10, wherein the at least one of the plurality of adapters is a blocking primer and wherein the at least one of the produced multiplex of different amplified target sequences includes at most a cleavable group at only one end of resulting amplicon is not processed through the remainder of the workflow and does not produce a productive adapter ligated amplicon; and
wherein the at least one blocking primer comprises a DNA sequence complementary to the target sequence downstream of either a productive forward target specific primer or a productive reverse target specific primer in the multiplex reaction or
wherein the at least one blocking primer comprises a DNA sequence complementary to the target sequence that overlaps in part or in full with a productive forward target specific primer or a productive reverse target specific primer in the multiplex reaction.

## Patentansprüche

1. Ein Verfahren zur Herstellung einer Sammlung von einer oder mehreren Zielnukleinsäuresequenzen, welche in einer Probe mit geringer Häufigkeit vorhanden sind, umfassend:
Amplifikation innerhalb einer einzigen Amplifikationsreaktionsmischung eines Multiplex verschiedener Zielsequenzen aus einer Probe, die mehrere unterschiedliche Zielsequenzen umfasst,
wobei die Amplifikation den Kontakt von mindestens eines Teils der Probe mit mehreren zielspezifischen Primern und einer Polymerase unter Amplifikationsbedingungen umfasst, wodurch ein Multiplex verschiedener amplifizierter Zielsequenzen erzeugt wird,
wobei mindestens zwei der mehreren zielspezifischen Primer spaltungsfähige Gruppen umfassen und mindestens eines der erzeugten Multiplexe verschiedener amplifizierter Zielsequenzen spaltungsfähige Gruppen an beiden Enden des resultierenden Amplikons umfassen, und wobei mindestens einer der mehreren zielspezifischen Primer ein blockierender Primer ist, welcher die spaltungsfähigen Gruppen nicht einschließt, und mindestens eines der erzeugten Multiplexe verschiedener amplifizierter Zielsequenzen eine spaltungsfähige Gruppe an nur einem Ende des resultierenden Aplikons umfasst;
Spaltung der spaltbaren Gruppen des Multiplexes verschiedener amplifizierter Zielsequenzen und Bildung gespaltener Enden; und
Ligation von mindestens zwei Adaptern mit den gespaltenen Enden von mindestens einem des Multiplexes verschiedener amplifizierter Zielsequenzen, um dadurch eine oder mehrere adapterligierte amplifizierte Zielsequenzen zu erzeugen;
wodurch eine Sammlung verschiedener adapterligierter Zielsequenzen hergestellt wird.
wobei die aus blockierenden Primern resultierenden Amplikon-Enden nicht in der Lage sind, Adapter zu ligieren, und wobei keiner der Adapter in der Ligationsreaktion unter hochstringenten Bedingungen mit einem beliebigen Multiplex verschiedener amplifizierter Zielsequenzen hybridisiert.

2. Verfahren nach Anspruch 1, wobei die Zielnukleinsäuresequenzen aus einer oder mehreren der folgenden Sequenzen ausgewählt sind: RNA, DNA, cDNA, TNA, cfNA, ctNA und Gewebenukleinsäure aus Zellen.

3. Verfahren nach Anspruch 1, wobei eine oder mehrere der Zielnukleinsäuresequenzen eine oder mehrere pathogene Nukleinsäuren für die Wirtsprobe sind, ausgewählt aus viralen, bakteriellen und mikrobiellen Nukleinsäuren.

4. Verfahren nach Anspruch 1, wobei eine oder mehrere der blockierenden Primärsequenzen auf die Wildtyp-Sequenz oder allelspezifische Sequenzen gerichtet sind, die aus der Wirtsprobe stammen.

5. Verfahren nach Anspruch 1, wobei die spaltbare Gruppe Methylguainin, 8-Oxo-Guanin, Xanthin, Hypoxanthin, 5,6-Dihydrouracil, Uracil, 5-Methylcytosin, Thymin-Dimer, 7-Methylguanosin, 8-Oxo-Desoxyguanosin, Xanthosin, Inosin, Dihydrouridin, Brodesoxyuridin, Uridin oder 5-Methylcytidin umfasst.

6. Verfahren nach Anspruch 1, wobei jeder der mehreren zielspezifischen Primer eines oder mehrere der folgenden Kriterien aufweist: (1) beinhaltet zwei oder mehr modifizierte Nukleotide innerhalb der Primersequenz, von denen wenigstens eines nahe oder an dem Endpunkt des Primers enthalten ist und von denen wenigstens eines an oder um die zentrale Nukleotidposition der Primersequenz enthalten ist; (2) Länge beträgt etwa 15 bis etwa 40 Basen; (3) Tₘ liegt zwischen etwa 60 °C bis etwa 70 °C; (4) weist eine geringe Kreuzreaktivität mit in der Probe vorhandenen Nicht-Zielsequenzen auf; (5) mindestens die ersten vier Nukleotide (in 3'-5'-Richtung) sind nicht komplementär zu einer beliebigen Sequenz innerhalb eines beliebigen anderen Primers, der in der Reaktion vorhanden ist; und (6) sind nicht komplementär zu einem beliebigen aufeinanderfolgenden Abschnitt von mindestens 5 Nukleotiden innerhalb einer beliebigen anderen erzeugten amplifizierten Zielsequenz.

7. Verfahren nach Anspruch 1, wobei die Schritte der Amplifikation, Spaltung, Ligation und Vorbereitung der Sammlung in einem einzigen Reaktionsgefäß durchgeführt werden, gegebenenfalls darüber hinaus die Kombination von zwei oder mehr Sammlungen umfassend, die jeweils separat in einem einzigen Reaktionsgefäß hergestellt wurden, wodurch eine Sammlung verschiedener adapterligierter Zielsequenzen hergestellt wird.

8. Verfahren nach Anspruch 1, wobei die Adapter weiterhin einen oder mehrere Barcode-, Tag- und/oder universelle Primingsequenzen umfassen.

9. Verfahren nach Anspruch 1, wobei der mindestens eine der mehreren zielspezifischen Primer ein blockierender Primer ist und wobei das mindestens eine der erzeugten Multiplexe unterschiedlicher amplifizierter Zielsequenzen, das höchstens eine spaltbare Gruppe an nur einem Ende des resultierenden Amplikons enthält, nicht durch den Rest des Arbeitsablaufs verarbeitet wird und kein produktives adapterligiertes Amplikon erzeugt; und
wobei der mindestens eine blockierende Primer eine DNA-Sequenz umfasst, die komplementär zur Zielsequenz ist, stromabwärts von entweder einem produktiven zielspezifischen Vorwärtsprimer oder einem produktiven zielspezifischen Rückwärtsprimer in der Multiplexreaktion, oder
wobei der mindestens eine blockierende Primer eine DNA-Sequenz umfasst, die komplementär zur Zielsequenz ist, welche teilweise oder vollständig mit einem produktiven zielspezifischen Vorwärtsprimer oder einem produktiven zielspezifischen Rückwärtsprimer in der Multiplexreaktion zusammenfällt.

10. Ein Verfahren zur Herstellung einer Sammlung von einer oder mehreren Zielnukleinsäuresequenzen, welche in einer Probe mit geringer Häufigkeit vorhanden sind, umfassend:
Amplifikation innerhalb einer einzigen Amplifikationsreaktionsmischung eines Multiplex verschiedener Zielsequenzen aus einer Probe, die mehrere unterschiedliche Zielsequenzen umfasst,
wobei die Amplifikation den Kontakt mit mindestens einem Teil der Probe mit mehreren Adaptern umfasst, die zur Amplifikation einer oder mehrerer Nukleinsäurezielsequenzen in der Probe unter Bedingungen fähig sind, unter denen die Nukleinsäure-Zielsequenzen eine erste Amplifikation durchlaufen, wobei mindestens zwei der mehreren Adapter eine universelle Handhabungssequenz und eine Zielnukleinsäuresequenz sowie eine spaltbare Einheit und optional eine oder mehrere Tag-Sequenzen umfassen, wobei die Zielnukleinsäuresequenz des Adapters mindestens eine spaltbare Einheit enthält und die universelle Handhabungssequenz die spaltbare Einheit nicht enthält und
mindestens eines der erzeugten Multiplexe verschiedener amplifizierter Zielsequenzen spaltbare Gruppen an beiden Enden des resultierenden Amplikons umfassen, und wobei mindestens einer der mehreren Adapter ein blockierender Primer ist, der die spaltbare Einheit oder universelle Handhabungssequenz nicht enthält und mindestens eines der erzeugten Multiplexe verschiedener amplifizierter Zielsequenzen eine spaltbare Gruppe und eine universelle Handhabungssequenz an nicht mehr als einem Ende des resultierenden Aplikons umfasst;
Verdau der resultierenden ersten Amplifikationsprodukte, um resultierende Primerdimere zu reduzieren oder zu eliminieren und teilweise verdaute Zielamplikone herzustellen, die beabstandete,
doppelsträngige Amplikone erzeugen, anschließende Reparatur der teilweise verdauten Zielamplikone; und
Amplifikation der reparierten Zielamplikons in einer zweiten Amplifikation unter Verwendung von Universalprimern, wodurch eine oder mehrere von Adaptern amplifizierte Zielsequenzen erzeugt werden;
wodurch eine Sammlung verschiedener adapteramplifizierter Zielsequenzen hergestellt wird, wobei die aus blockierenden Primern resultierenden Amplikons nicht mit der Amplifikation des zweiten Universalprimers kompatibel sind und wobei keiner der Adapter in der Amplifikationsreaktion unter hochstringenten Bedingungen mit einem beliebigen Multiplex verschiedener amplifizierter Zielsequenzen hybridisiert.

11. Verfahren nach Anspruch 10, wobei die Zielnukleinsäuresequenzen aus einer oder mehreren der folgenden Sequenzen ausgewählt sind: RNA, DNA, cDNA, TNA, cfNA, ctNA und Gewebenukleinsäure aus Zellen.

12. Verfahren nach Anspruch 10, wobei eine oder mehrere der Zielnukleinsäuren eine oder mehrere pathogene Nukleinsäuren für die Wirtsprobe sind, welche aus viralen, bakteriellen und mikrobiellen Nukleinsäuren ausgewählt sind.

13. Verfahren nach Anspruch 10, wobei einer oder mehrere der blockierenden Primer auf die Wildtyp-Sequenz oder allelspezifische Sequenz gerichtet sind, die aus der Wirtsprobe stammen.

14. Verfahren nach Anspruch 10, wobei die spaltbare Gruppe Methylguainin, 8-Oxo-Guanin, Xanthin, Hypoxanthin, 5,6-Dihydrouracil, Uracil, 5-Methylcytosin, Thymin-Dimer, 7-Methylguanosin, 8-Oxo-Desoxyguanosin, Xanthosin, Inosin, Dihydrouridin, Brodesoxyuridin, Uridin oder 5-Methylcytidin umfasst.

15. Verfahren nach Anspruch 10, wobei jeder der mehreren Adapter eines oder mehrere der folgenden Kriterien aufweist: (1) beinhaltet zwei oder mehr modifizierte Nukleotide innerhalb der Primersequenz, von denen wenigstens eines nahe oder an dem Endpunkt des Primers enthalten ist und von denen wenigstens eines an oder um die zentrale Nukleotidposition der Primersequenz enthalten ist; (2) Länge beträgt etwa 15 bis etwa 40 Basen; (3) Tₘ liegt zwischen etwa 60 °C bis etwa 70 °C; (4) weist eine geringe Kreuzreaktivität mit in der Probe vorhandenen Nicht-Zielsequenzen auf; (5) mindestens die ersten vier Nukleotide (in 3'-5'-Richtung) sind nicht komplementär zu einer beliebigen Sequenz innerhalb eines beliebigen anderen Primers, der in der Reaktion vorhanden ist; und (6) sind nicht komplementär zu einem beliebigen aufeinanderfolgenden Abschnitt von mindestens 5 Nukleotiden innerhalb einer beliebigen anderen erzeugten amplifizierten Zielsequenz.

16. Verfahren nach Anspruch 10, wobei die Schritte der Amplifikation, des Aufschlusses, der Korrektur, der Herstellung und der Vorbereitung der Sammlung in einem einzigen Reaktionsgefäß durchgeführt werden, gegebenenfalls darüber hinaus die Kombination von zwei oder mehr Sammlungen, die jeweils separat in einem einzigen Reaktionsgefäß hergestellt wurden, umfasst, wodurch eine Sammlung von verschiedenen adapteramplifizierten Zielsequenzen hergestellt wird.

17. Verfahren nach Anspruch 10, wobei die Adapter und/oder die universellen Primer weiterhin einen oder mehrere Barcode-, Tag- und/oder universelle Primingsequenzen umfassen.

18. Verfahren nach Anspruch 10, wobei der mindestens eine der mehreren Adaptern ein blockierender Primer ist und wobei das mindestens eine der erzeugten Multiplexe unterschiedlicher amplifizierter Zielsequenzen höchstens eine spaltbare Gruppe an nur einem Ende des resultierenden Amplikons enthält, das nicht durch den Rest des Arbeitsablaufs verarbeitet wird und kein produktives adapterligiertes Amplikons erzeugt; und
wobei der mindestens eine blockierende Primer eine DNA-Sequenz umfasst, die komplementär zur Zielsequenz ist, stromabwärts von entweder einem produktiven zielspezifischen Vorwärtsprimer oder einem produktiven zielspezifischen Rückwärtsprimer in der Multiplexreaktion oder
wobei der mindestens eine blockierende Primer eine DNA-Sequenz umfasst, die komplementär zur Zielsequenz ist, welche teilweise oder vollständig mit einem produktiven zielspezifischen Vorwärtsprimer oder einem produktiven zielspezifischen Rückwärtsprimer in der Multiplexreaktion zusammenfällt.

## Revendications

1. Méthode de préparation d'une bibliothèque d'une ou plusieurs séquences d'acides nucléiques cibles présentes dans un échantillon à faible fréquence, comprenant :
l'amplification dans un seul mélange de réaction d'amplification d'un multiplex de séquences cibles différentes à partir d'un échantillon comprenant plusieurs séquences cibles différentes ;
dans laquelle l'amplification consiste à mettre en contact au minimum une partie de l'échantillon avec plusieurs amorces spécifiques et une polymérase dans des conditions d'amplification, produisant ainsi un multiplex de différentes séquences cibles amplifiées ;
dans laquelle au minimum deux des différentes amorces spécifiques de la cible comprennent des groupes clivables et au minimum un des multiplex produits de différentes séquences cibles amplifiées comprend des groupes clivables aux deux extrémités de l'amplicon résultant, et dans lequel au minimum une des différentes amorces spécifiques de la cible est une amorce de blocage qui ne comprend pas les groupes clivables et au minimum un des multiplex produits de différentes séquences cibles amplifiées comprend un groupe clivable à une seule extrémité de l'amplicon résultant ;
le clivage des groupes clivables du multiplex de différentes séquences cibles amplifiées et la formation d'extrémités clivées ; et
la ligature d'au moins deux adaptateurs aux extrémités clivées d'au moins une des multiples séquences cibles amplifiées différentes, produisant ainsi une ou plusieurs séquences cibles amplifiées ligaturées par l'adaptateur ;
permettant ainsi la préparation d'une bibliothèque de différentes séquences cibles ligaturées par l'adaptateur ;
où les extrémités des amplicons résultant des amorces de blocage ne sont pas capables de ligaturer l'adaptateur, et où aucun des adaptateurs de la réaction de ligature ne s'hybride, dans des conditions très strictes, à l'une quelconque des multiples séquences cibles amplifiées différentes.

2. Méthode selon la revendication 1, dans laquelle les séquences cibles d'acide nucléique sont choisies parmi un ou plusieurs ARN, ADN, ADNc, ADNt, ADNc, ADNcf, ADNct et acide nucléique tissulaire provenant de cellules.

3. Méthode selon la revendication 1, dans laquelle une ou plusieurs des séquences cibles d'acides nucléiques est un ou plusieurs acides nucléiques pathogènes pour l'échantillon hôte, choisis parmi les acides nucléiques viraux, bactériens et microbiens.

4. Méthode selon la revendication 1, dans laquelle une ou plusieurs des amorces de blocage sont dirigées vers des séquences de type sauvage ou des séquences spécifiques d'allèles natives de l'échantillon hôte.

5. Méthode selon la revendication 1, dans laquelle le groupe clivable comprend la méthylguanine, la 8-oxo-guanine, la xanthine, l'hypoxanthine, le 5,6-dihydrouracile, l'uracile, la 5-méthylcytosine, le dimère de thymine, la 7-méthylguanosine, la 8-oxo-désoxyguanosine, la xanthosine, l'inosine, la dihydrouridine, la bromodésoxyuridine, l'uridine ou la 5-méthylcytidine.

6. Méthode selon la revendication 1, dans laquelle chacune des différentes amorces spécifiques à la cible présente un ou plusieurs des critères suivants : (1) comprend deux nucléotides modifiés ou plus dans la séquence d'amorce, dont au minimum un est inclus à proximité ou aux extrémités de l'amorce et dont au minimum un est inclus à la position du nucléotide central de la séquence d'amorce ou autour de cette position ; (2) la longueur est d'environ 15 à environ 40 bases en longueur ; (3) Tₘ est d'environ 60 °C à environ 70 °C ; (4) a une faible réactivité croisée avec les séquences non cibles présentes dans l'échantillon ; (5) au minimum, les quatre premiers nucléotides (dans le sens 3' vers 5') ne sont pas complémentaires d'une séquence d'une autre amorce présente dans la réaction ; et (6) ne sont pas complémentaires d'une séquence consécutive d'au moins 5 nucléotides d'une autre séquence cible amplifiée produite.

7. Méthode selon la revendication 1, dans laquelle les étapes d'amplification, de clivage, de ligature et de préparation de la bibliothèque sont effectuées dans une seule cuve de réaction, comprenant éventuellement en outre la combinaison de deux bibliothèques ou plus, chacune préparée séparément dans une seule cuve de réaction, préparant ainsi une bibliothèque de différentes séquences cibles ligaturées par l'adaptateur.

8. Méthode selon la revendication 1, dans laquelle les adaptateurs comprennent en outre un ou plusieurs codes-barres, étiquettes et/ou séquences d'amorces universelles.

9. Méthode selon la revendication 1, dans laquelle au minimum l'une des différentes amorces spécifiques à la cible est une amorce de blocage et dans laquelle au minimum l'un des multiplex produits de différentes séquences cibles amplifiées qui comprend au plus un groupe clivable à une seule extrémité de l'amplicon résultant n'est pas traité par le reste du flux de travail et ne produit pas d'amplicon ligaturé par adaptateur productif ; et
dans laquelle au minimum l'amorce de blocage comprend une séquence d'ADN complémentaire à la séquence cible en aval d'une amorce productive spécifique à la cible en avant ou d'une amorce productive spécifique à la cible en arrière dans la réaction multiplex, ou
dans laquelle au minimum l'amorce de blocage comprend une séquence d'ADN complémentaire à la séquence cible qui se superpose en partie ou en totalité à une amorce productive spécifique à la cible en avant ou à une amorce productive spécifique à la cible en arrière dans la réaction multiplex.

10. Méthode de préparation d'une bibliothèque d'une ou plusieurs séquences cibles d'acide nucléique présentes dans un échantillon à faible fréquence, comprenant :
l'amplification dans un seul mélange de réaction d'amplification d'un multiplex de séquences cibles différentes à partir d'un échantillon comprenant plusieurs séquences cibles différentes ;
dans laquelle l'amplification consiste à mettre en contact au minimum une partie de l'échantillon avec différents adaptateurs capables d'amplifier une ou plusieurs séquences cibles d'acides nucléiques dans l'échantillon dans des conditions où les séquences cibles d'acides nucléiques subissent une première amplification, dans laquelle au minimum deux des différents adaptateurs comprennent une séquence de poignée universelle et une séquence d'acide nucléique cible et une fraction clivable et éventuellement une ou plusieurs séquences d'étiquetage dans lequel la séquence d'acide nucléique cible de l'adaptateur comprend au minimum une fraction clivable et la séquence de poignée universelle ne comprend pas la fraction clivable et au minimum un des multiplex produits de différentes séquences cibles amplifiées comprend des groupes clivables aux deux extrémités de l'amplicon résultant, et dans laquelle au minimum un des adaptateurs est une amorce de blocage qui n'inclut pas la fraction clivable ou la séquence de poignée universelle et au minimum un des multiplex produits de différentes séquences cibles amplifiées inclut un groupe clivable et une séquence de poignée universelle à pas plus d'une extrémité de l'amplicon résultant ;
la digestion des premiers produits d'amplification obtenus pour réduire ou éliminer les dimères d'amorces obtenus et la préparation d'amplicons cibles partiellement digérés, la production d'amplicons double brin séparés, puis la réparation des amplicons cibles partiellement digérés ; et
l'amplification des amplicons cibles réparés dans une seconde amplification à l'aide d'amorces universelles, produisant ainsi une ou plusieurs séquences cibles amplifiées par l'adaptateur;
la préparation ultérieure d'une bibliothèque de différentes séquences cibles amplifiées par l'adaptateur, dans laquelle les amplicons résultant de l'amorce de blocage ne sont pas compatibles avec l'amplification de la seconde amorce universelle et dans laquelle aucun des adaptateurs de la réaction d'amplification ne s'hybride, dans des conditions de stringence élevée, à l'une quelconque des séquences cibles amplifiées du multiplex de différentes séquences cibles.

11. Méthode selon la revendication 10, dans laquelle les séquences cibles d'acide nucléique sont choisies parmi un ou plusieurs ARN, ADN, ADNc, ADNt, ADNc, ADNcf, ADNct et acide nucléique tissulaire provenant de cellules.

12. Méthode selon la revendication 10, dans laquelle une ou plusieurs des séquences cibles d'acides nucléiques est un ou plusieurs acides nucléiques pathogènes pour l'échantillon hôte, choisis parmi les acides nucléiques viraux, bactériens et microbiens.

13. Méthode selon la revendication 10, dans laquelle une ou plusieurs des amorces de blocage sont dirigées vers des séquences de type sauvage ou des séquences spécifiques d'allèles natives de l'échantillon hôte.

14. Méthode selon la revendication 10, dans laquelle le groupe clivable comprend la méthylguanine, la 8-oxo-guanine, la xanthine, l'hypoxanthine, le 5,6-dihydrouracile, l'uracile, la 5-méthylcytosine, le dimère de thymine, la 7-méthylguanosine, la 8-oxo-désoxyguanosine, la xanthosine, l'inosine, la dihydrouridine, la bromodésoxyuridine, l'uridine ou la 5-méthylcytidine.

15. Méthode selon la revendication 10, dans laquelle chacun des différents adaptateurs présente un ou plusieurs des critères suivants : (1) comprend deux nucléotides modifiés ou plus dans la séquence d'amorce, dont au minimum un est inclus à proximité ou aux extrémités de l'amorce et dont au minimum un est inclus à la position du nucléotide central de la séquence d'amorce ou autour de cette position ; (2) la longueur est d'environ 15 à environ 40 bases en longueur ; (3) Tₘ est d'environ 60 °C à environ 70 °C ; (4) a une faible réactivité croisée avec les séquences non cibles présentes dans l'échantillon ; (5) au minimum, les quatre premiers nucléotides (dans le sens 3' vers 5') ne sont pas complémentaires d'une séquence d'une autre amorce présente dans la réaction ; et (6) ne sont pas complémentaires d'une séquence consécutive d'au moins 5 nucléotides d'une autre séquence cible amplifiée produite.

16. Méthode selon la revendication 10, dans laquelle les étapes d'amplification, de digestion, de réparation, de production et de préparation de la bibliothèque sont effectuées dans une seule cuve de réaction, comprenant éventuellement en outre la combinaison de deux bibliothèques ou plus, chacune préparée séparément dans une seule cuve de réaction, préparant ainsi une bibliothèque de différentes séquences cibles amplifiées par l'adaptateur.

17. Méthode selon la revendication 10, dans laquelle les adaptateurs et/ou les amorces universelles comprennent en outre un ou plusieurs codes-barres, étiquettes et/ou séquences d'amorces universelles.

18. Méthode selon la revendication 10, dans laquelle au minimum l'un des différents adaptateurs est une amorce de blocage et dans laquelle au minimum l'un des multiplex produits de différentes séquences cibles amplifiées qui comprend au plus un groupe clivable à une seule extrémité de l'amplicon résultant n'est pas traité par le reste du flux de travail et ne produit pas d'amplicon ligaturé par adaptateur productif ; et
dans laquelle au minimum l'amorce de blocage comprend une séquence d'ADN complémentaire à la séquence cible en aval d'une amorce productive spécifique à la cible en avant ou d'une amorce productive spécifique à la cible en arrière dans la réaction multiplex, ou
dans laquelle au minimum l'amorce de blocage comprend une séquence d'ADN complémentaire à la séquence cible qui se superpose en partie ou en totalité à une amorce productive spécifique à la cible en avant ou à une amorce productive spécifique à la cible en arrière dans la réaction multiplex.
